(19) Europäisches Patentamt
European Patent Office
Office européen des brevets

(11) Veröffentlichungsnummer: **0 129 906**
**B1**

# (12) EUROPÄISCHE PATENTSCHRIFT

(45) Veröffentlichungstag der Patentschrift: **08.06.88**

(51) Int. Cl.⁴: **C 07 D 311/58**, C 07 D 311/64, C 07 D 311/66, A 61 K 31/35

(21) Anmeldenummer: **84107289.5**

(22) Anmeldetag: **25.06.84**

(54) **Phenoxyalkoxy-3,4-dihydro-2H-1-benzopyranderivate.**

(30) Priorität: **24.06.83 US 507383**

(43) Veröffentlichungstag der Anmeldung: **02.01.85 Patentblatt 85/1**

(45) Bekanntmachung des Hinweises auf die Patenterteilung: **08.06.88 Patentblatt 88/23**

(84) Benannte Vertragsstaaten: **AT BE CH DE FR GB IT LI LU NL SE**

(56) Entgegenhaltungen:
**EP-A-0 003 221**
**AT-B-333 755**
**DE-A-2 301 541**
**US-A-4 133 889**

**Burger's Medicinal Chemistry, Fourth Edition, Part III, Wiley, New York, 1981, 598-609**

(73) Patentinhaber: **F. HOFFMANN- LA ROCHE & CO. Aktiengesellschaft, CH- 4002 Basel (CH)**

(72) Erfinder: **Cohen, Noal, 19 Euclid Place, Montclair, N.J. 07042 (US)**
Erfinder: **Weber, Giuseppe F., 192 Anderson Parkway, Cedar Grove, N.J. 07009 (US)**

(74) Vertreter: **Lederer, Franz, Dr., Van der Werth, Lederer & Riederer Patentanwälte Lucile- Grahn-Strasse 22, D-8000 München 80 (DE)**

**EP 0 129 906 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die vorliegende Erfindung betrifft neue Verbindungen der allgemeinen Formel

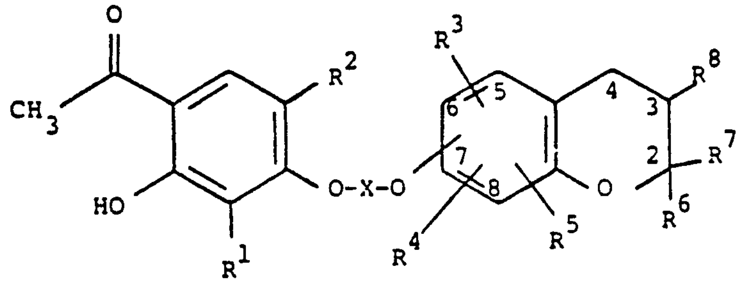

I

worin $R^1$ Wasserstoff oder niederes Alkyl, $R^2$ Wasserstoff oder Halogen und $R^3$, $R^4$ und $R^5$ unabhängig voneinander Wasserstoff, Acyl oder niederes Alkyl bedeuten, wobei höchstens einer von $R^3$, $R^4$ und $R^5$ Acyl bedeuten kann, und entweder $R^6$ und $R^7$ je Wasserstoff und $R^8$ die Gruppe $-COOR^9$ bedeuten, oder $R^6$ Wasserstoff oder niederes Alkyl, $R^7$ die Gruppe $-CH_2)_n-COOR^9$, $R^8$ Wasserstoff, $R^9$ Wasserstoff oder niederes Alkyl. X $(C_{3-7})$-Alkylen und n eine Zahl von 0-4 bedeuten,
und, wenn $R^9$ Wasserstoff bedeutet, pharmazeutisch annehmbare Salze davon mit Basen. Diese Verbindungen sind nützlich als Wirkstoffe für die Behandlung von allergischen Zuständen.

Gegenstand der vorliegenden Erfindung sind: Die vorerwähnten Verbindungen und Salze als solche und als therapeutische Wirkstoffe; ein Verfahren zur Herstellung besagter Produkte und Zwischenprodukte für deren Herstellung; Arzneimittel, enthaltend besagte Produkte, und ein Verfahren zur Herstellung solcher Arzneimittel; sowie die Verwendung besagter Produkte bei der Behandlung oder Verhütung von Krankheiten.

Aus der U.S. Patenschrift 4 133 889 und Burger's Medicinal Chemistry, Vierte Ausgabe, Teil III, Wiley, New York, 1981, Seiten 598-609 sind Benzopyran-4-(4H)-on-Derivate sowie deren SRS-A antagonisierenden und damit antiallergischen Eigenschaften bekannt.

Der in der vorliegenden Beschreibung verwendete Ausdruck "niederes Alkyl" bezeichnet geradkettige oder verzweigte, gesättigte Kohlenwasserstoffreste mit bis zu 7, vorzugsweise bis zu 4 Kohlenstoffatomen, wie Methyl. Äthyl, Propyl, Isopropyl, Butyl, t-Butyl, Neopentyl, Pentyl, Heptyl und dergleichen. Der Ausdruck "Halogen" bezeichnet die Halogene Brom, Chlor, Fluor und Jod. Der Ausdruck "Acyl" bezeichnet einerseits von aliphatischen Carbonsäuren abgeleitete niedere Alkanoylgruppen mit bis zu 7, vorzugsweise bis zu 4 Kohlenstoffatomen, wie Formyl, Acetyl, Propionyl und dergleichen, und andererseits von aromatischen Carbonsäuren abgeleitete Arylcarbonylgruppen, wie Benzoyl und dergleichen. Der Ausdruck "Acyl" bezeichnet vorzugsweise niedere Alkanoylgruppen. Der Ausdruck "$(C_{3-7})$-Alkylen" bezeichnet geradkettige oder verzweigte Reste mit 3-7 Kohlenstoffatomen, wie Propylen, 2-Methylpropylen, Butylen, Pentamethylen, Hexamethylen, Heptamethylen und dergleichen. Der Ausdruck "Aryl" bezeichnet gegebenenfalls durch Halogen, Trifluormethyl, niederes Alkyl, niederes Alkoxy, Nitro, Amino, niederes Alkylamino und di-nieders Alkylamino einfach oder mehrfach substituiertes Phenyl. Der Ausdruck "Arylalkyl" bezeichnet geradkettige oder verzweigte niedere Alkylgruppen, worin ein oder mehrere Wasserstoffatome durch Arylgruppen ersetzt sind, wie Benzyl und dergleichen. Die als "nieder" bezeichneten Gruppen und Verbindungen enthalten bis zu 7, vorzugsweise bis zu 4 Kohlenstoffatome.

Bevorzugte Verbindungen der Formel I sind diejenigen der allgemeinen Formel

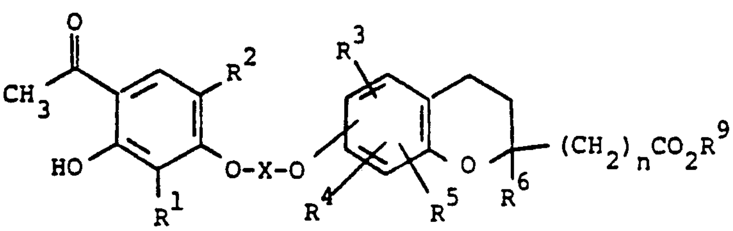

Ia

worin $R^1$, $R^2$, $R^3$, $R^4$, $R^5$, $R^6$, $R^9$, X und n obige Bedeutung besitzen.

Eine weitere bevorzugte Gruppe von erfindungsgemässen Verbindungen entsprechen der allgemeinen Formel

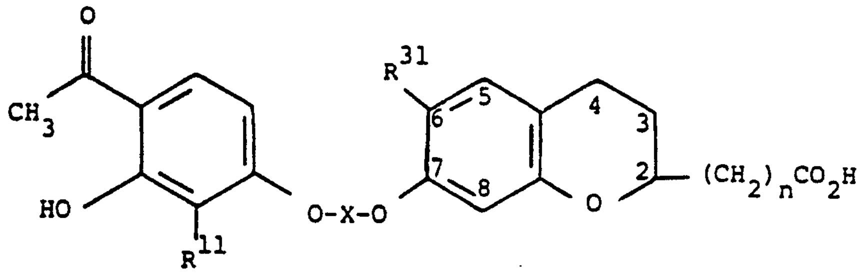

Ib

worin $R^{11}$ niederes Alkyl, $R^{31}$ Acyl, X $(C_{3-7})$-Alkylen und n eine Zahl von 0-4 bedeuten.

Besonders bevorzugte erfindungsgemässe Verbindungen sind diejenigen der Formel Ia, worin $R^1$ niederes Alkyl, $R^2$ Wasserstoff, $R^3$ Acyl, $R^4$, $R^5$, $R^6$ und $R^9$ je Wasserstoff und X $(C_{3-5})$-Alkylen bedeuten.

Ganz besonders bevorzugt werden die Verbindungen der Formel Ia worin $R^1$ Propyl, $R^2$ Wasserstoff, $R^3$ Acetyl, $R^4$, $R^5$, $R^6$ und $R^9$ je Wasserstoff und X $(C_{3-5})$-Alkylen bedeuten.

Die am stärksten bevorzugten Verbindungen der Formel I sind:

rac-6-Acetyl-7-[5-(4-acetyl-3-hydroxy-2-propyl-phenoxy)pentyloxy]-3,4-dihydro-2H-1-benzopyran-2-carbon säure,

(S)-(+)-6-Acetyl-7-[5-(4-acetyl-3-hydroxy-2-propylphenoxy)pentyloxy]-3,4-dihydro-2H-1-benzopyran-2-carbonsäure.

rac-7-[3-(4-Acetyl-3-hydroxy-2-propylphenoxy)propoxy]-3,4-dihydro-2H-1-benzopyran-2-carbonsäure,

rac-6-Acetyl-7-[3-(4-acetyl-3-hydroxy-2-propylphenoxy)propoxy]-2-methyl-8-propyl-2H-1-benzopyran-2-propansäure,

rac-6-[3-(4-Acetyl-3-hydroxy-2-propylphenoxy)propoxy]-3,4-dihydro-2-methyl-2H-1-benzopyran-2-carbonsäure,

rac-7-[3-(4-Acetyl-3-hydroxy-2-propylphenoxy)propoxy]-3,4-dihydro-8-propyl-2H-1-benzopyran-2-carbonsäure und

rac-6-Acetyl-7-[3-(4-acetyl-3-hydroxy-2-propylphenoxy)propoxy-3,4-dihydro-2H-benzopyran-2-carbonsäure.

Beispiele von anderen Verbindungen der Formel I sind:

(R)-(-)-6-Acetyl-7-[5-(4-acetyl-3-hydroxy-2-propylphenoxy)pentyloxy]-3,4-dihydro-2H-1-benzopyran-2-carbonsäure. säure.

rac-7-[3-(4-Acetyl-3-hydroxy-2-propylphenoxy)propoxy]-3,4-dihydro-2H-1-benzopyran-2-buttersäure,

rac-8-[3-(4-Acetyl-3-hydroxy-2-propylphenoxy)propoxy]-3,4-dihydro-7-acetyl-2H-1-benzopyran-2-carbonsäure,

rac-5-Acetyl-6-[5-(4-acetyl-3-hydroxy-2-propylphenoxy)pentyloxy]-3,4-dihydro-2H-1-benzopyran-2-propansäure,

rac-6-Acetyl-7-[5-(4-acetyl-3-hydroxy-2-propylphenoxy)pentyloxy]-3,4-dihydro-2H-1-benzopyran-2-buttersäure und

rac-5-Propanoyl-7-[7-(4-acetyl-3-hydroxy-2-propylphenoxy)heptyloxy]-3,4-dihydro-8-propyl-2H-1-benzopyran-2-buttersäure.

Die Verbindungen der Formel I und ihre pharmazeutisch annehmbaren Salze können erfindungsgemäss dadurch hergestellt werden, dass man

a) eine Verbindung der allgemeinen Formel

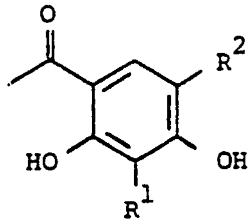

II

oder

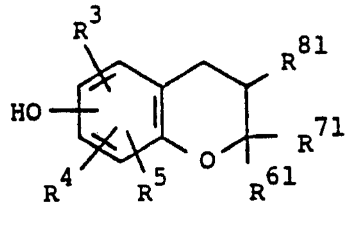

III

worin entweder $R^{61}$ und $R^{71}$ je Wasserstoff und $R^{81}$ die Gruppe -COOR$^{91}$ oder $R^{61}$ Wasserstoff oder niederes Alkyl, $R^{71}$ die Gruppe $-(CH_2)_n$-COOR$^{91}$, $R^{81}$ Wasserstoff und $R^{91}$ niederes Alkyl bedeuten und $R^1$, $R^2$, $R^3$, $R^4$, $R^5$ und n obige Bedeutung besitzen,

mit einer Verbindung der allgemeinen Formel

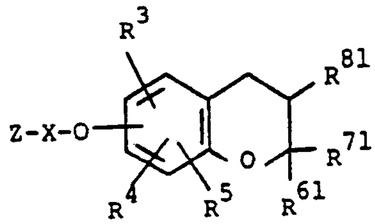

IV

und

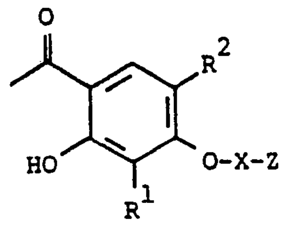

V

worin $R^1$, $R^2$, $R^3$, $R^4$, $R^{61}$, $R^{71}$, $R^{81}$ und X obige Bedeutung besitzen und Z ein Halogenatom bedeutet, umsetzt, oder

b) die Estergruppe in einer Verbindung der allgemeinen Formel

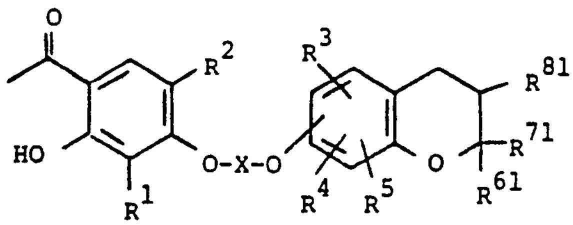

Ic

worin $R^1$, $R^2$, $R^3$, $R^4$, $R^5$, $R^{61}$, $R^{71}$, $R^{81}$ und X obige Bedeutung besitzen.

hydrolysiert und erwunschtenfalls ein erhaltenes Racemat in die optisch aktiven Isomeren aufspaltet und/oder eine erhaltene Verbindung der Formel I, worin $R^9$ Wasserstoff bedeutet, mit einer Base in ein pharmazeutisch annehmbares Salz überführt.

Die Reaktion einer Verbindung der Formel II mit einer Verbindung der Formel IV bzw. die Reaktion einer Verbindung der Formel III mit einer Verbindung der Formel V zu einer Verbindung der Formel Ic kann unter wasserfreien Bedingungen in einem inerten Lösungsmittel, z. B. in Aceton, Methyläthylketon, Diäthylketon, Dimethylformamid oder dergleichen, bei der Rückflusstemperatur der Reaktionsmischung (in Dimethylformamid vorzugsweise bei einer Temperatur im Bereich von etwa 70° bis etwa 100°C) und in Gegenwart eines säurebindenden Mittels, z. B. Kaliumcarbonat oder dergleichen, durchgeführt werden. Eine Mischung aus Aceton und Dimethylformamid ist das bevorzugte Lösungsmittel. Die erhaltene Verbindung der Formel Ic kann mittels herkömmlicher Methoden, beispielsweise mittels Kristallisation, Chromatographie oder dergleichen, isoliert werden.

Eine erhaltene Verbindung der Formel Ic kann mittels Hydrolyse in die entsprechende freie Carbonsäure übergeführt werden. Diese Hydrolyse wird vorzugsweise mit einem Alkalimetallhydroxid, z. B. Lithiumhydroxid, Natriumhydroxid, Kaliumhydroxid oder dergleichen, in einer Mischung aus Wasser und einem mit Wasser mischbaren Lösungsmittel, z. B. Methanol, Äthanol, Tetrahydrofuran oder dergleichen, bei einer Temperatur in einem Bereich von etwa Raumtemperatur bis Siedetemperatur durchgeführt. Die erhaltene Verbindung kann mittels herkömmlicher Methoden, z. B. mittels Kristallisation, Chromatographie oder dergleichen isoliert werden.

Die als Ausgangsstoffe verwendeten Verbindungen der Formeln II und V sind bekannt oder können mittels bekannter Verfahren hergestellt werden. Die als Ausgangsstoffe verwendeten Verbindungen der Formeln III und IV können gemäss den nachfolgenden Reaktionsschemata I-XIII hergestellt werden:

**Reaktionsschema I**

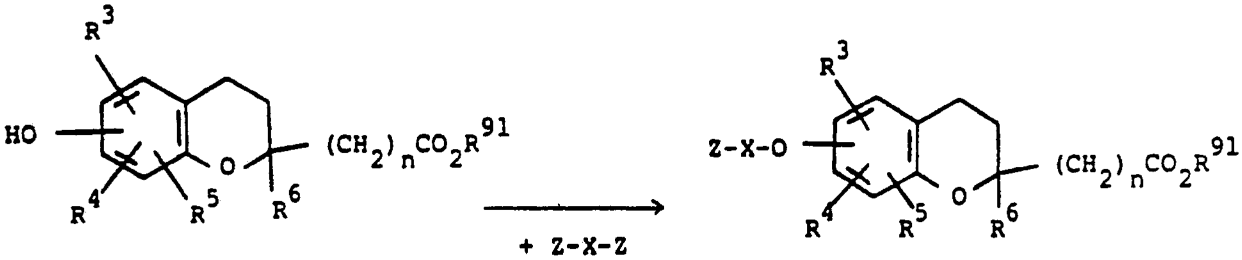


III'

VI

IV'

worin $R^3$, $R^4$, $R^5$, $R^6$, $R^{91}$, X, Z und n obige Bedeutung besitzen.

Die Reaktion einer Verbindung der Formel VI mit einer Verbindung der Formel III' zu einer Verbindung der Formel IV' kann in einem inerten organischen Lösungsmittel, wie Dimethylformamid, Aceton, Methyläthylketon oder dergleichen, vorzugsweise in Dimethylformamid, in Gegenwart einer Base, beispielsweise einem Alkalimetallcarbonat, wie Kaliumcarbonat, Natriumcarbonat oder dergleichen, oder einem Alkalimetallhydrid, wie Natriumhydrid oder dergleichen, bei einer Temperatur im Bereich von etwa 20° bis etwa 150°C, vorzugsweise bei Raumtemperatur, durchgeführt werden. Die erhaltene Verbindung der Formel IV' kann mittels herkömmlicher Methoden, beispielsweise mittel Kristallisation, Extraktion, Chromatographie oder dergleichen, isoliert werden. Die Verbindungen der Formel VI sind bekannt oder können nach an sich bekannten Verfahren hergestellt werden.

**Reaktionschema II**

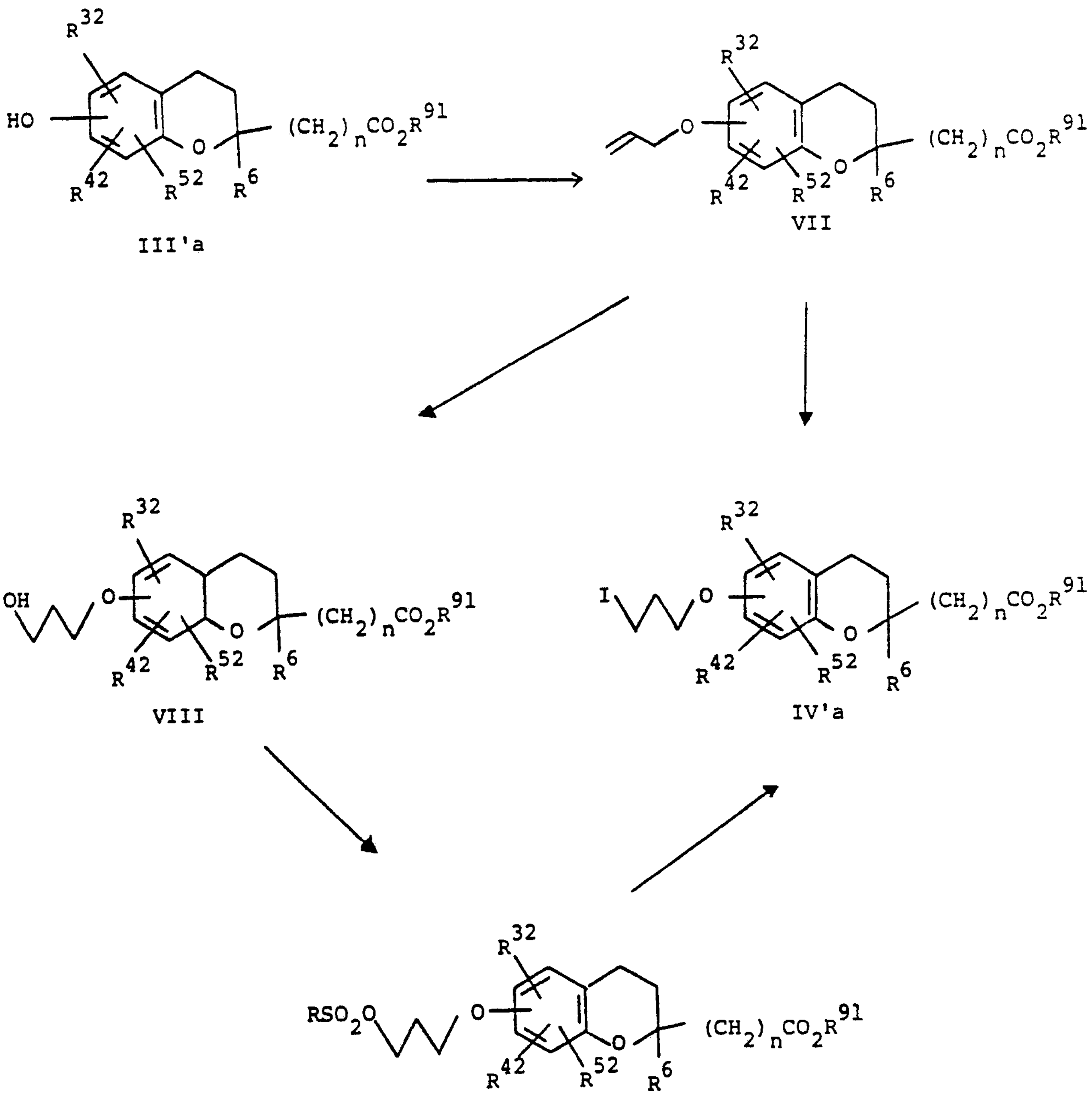

III'a

VII

VIII

IV'a

VIIIa

worin $R^6$, $R^{91}$ und n obige Bedeutung besitzen, $R^{32}$, $R^{42}$ und $R^{52}$ je Wasserstoff oder niederes Alkyl und R niederes Alkyl oder Aryl bedeuten.

Die Reaktion einer Verbindung der Formel III'a mit einem Allylhalogenid zu einer Verbindung der Formel VII gemäss Reaktionsschema II kann in einem inerten organischen Lösungsmittel, wie Dimethylformamid, Aceton, Methyläthylketon oder dergleichen, vorzugsweise in Dimethylformamid, in Gegenwart einer Base, beispielsweise einem Alkalimetallcarbonat, wie Kaliumcarbonat, Natriumcarbonat oder dergleichen, oder einem Alkalimetallhydrid, wie Natriumhydrid oder dergleichen, bei einer Temperatur in einem Bereich von etwa 20° bis etwa 150°C, vorzugsweise bei Raumtemperatur, durchgeführt werden. Die erhaltene Verbindung der Formel VII kann mittels herkömmlicher Methoden, beispielsweise mittels Kristallisation, Extraktion. Chromatographie oder dergleichen, isoliert werden.

Eine Verbindung der Formel VII kann unter Verwendung der für eine Hydroborierung eines Olefins üblichen Bedingungen in eine Verbindung der Formel VII übergeführt werden. Man kann beispielsweise ein Hydrid der Formel $(Q)_2BH$, worin Q Wasserstoff, niederes Alkyl oder niederes Cycloalkyl bedeutet, vorzugsweise Boran ($BH_3$), Dicyclohexylboran oder Boran-Dimethylsulfid-Komplex, in einem inerten Ätherlösungsmittel. vorzugsweise Tetrahydrofuran, bei einer Temperatur im Bereich von etwa 0°C bis etwa 100°C, vorzugsweise bei Raumtemperatur, verwenden. Das erhaltene Organoboran-Zwischenprodukt wird oxidiert, und zwar vorzugsweise unter Verwendung von alkalischem Wasserstoffperoxid oder dergleichen. Die erhaltene Verbindung der Formel VIII kann mittels herkömmlichen Methoden isoliert werden.

Eine Verbindung der Formel VIII wird mit einem Sulfonylchlorid der Formel $RSO_2Cl$, worin R Alkyl oder Aryl, wie Phenyl, bedeutet, zu einer Verbindung der Formel VIIIa umgesetzt. Man verwendet dabei die für die Überführung von Alkoholen in Sulfonatester üblichen Methoden; der Methansulfonatester wird bevorzugt. Die Sulfonierung wird beispielsweise in einem inerten Lösungsmittel, wie Methylenchlorid, unter Verwendung eines Sulfonylierungsmittels, beispielsweise eines Alkylsulfonylchlorides, vorzugsweise Methansulfonylchlorid, in Gegenwart einer Base, beispielsweise einem tri-niederen Alkylamin, Pyridin oder dergleichen, vorzugsweise Triäthylamin, bei einer Temperatur im Bereich von etwa 0° bis etwa 25°C, vorzugsweise bei 0°C, durchgeführt. Die erhaltene Verbindung der Formel VIIIa kann mittels herkömmlicher Methoden isoliert werden.

Eine Verbindung der Formel VIII kann in eine Verbindung der Formel IV'a übergeführt werden, und zwar unter Verwendung von Bedingungen, wie sie für die Überführung von Sulfonatester in Jodide üblicherweise angewendet werden. Man kann beispielsweise eine Verbindung der Formel VIIIa mit einem Alkalimetalljodid, beispielsweise Natriumjodid, Lithiumjodid, Kaliumjodid oder dergleichen, vorzugsweise mit Natriumjodid, in einem Lösungsmittel, beispielsweise in einem Dialkylketon, wie Äthylmethylketon, Aceton und dergleichen, vorzugsweise in Aceton, bei einer Temperatur in einem Bereich von etwa 20° bis etwa 100°C, vorzugsweise bei 20°C, umsetzen. Die erhaltene Verbindung der Formel IV'a kann mittels herkömmlicher Methoden isoliert werden.

Andererseits kann man auch eine Verbindung der Formel VII direkt in eine Verbindung der Formel IV'a überführen, und zwar unter Anwendung von Bedingungen, wie sie gewöhnlich für die Überführung von endständigen Olefinen in primäre Jodide mittels Hydroborierung und Jodierung angewendet werden. Die Reaktion kann beispielsweise unter Verwendung von Jod und einem Hydrid der Formel $(Q)_2BH$, worin Q Wasserstoff, niederes Alkyl oder niederes Cycloalkyl bedeutet, vorzugsweise Dicyclohexylboran, in Gegenwart eines inerten Äthers, wie Tetrahydrofuran, und einer Base, z. B. einem Alkalimetallcarboxylat, vorzugsweise Natriumacetat, bei einer Temperatur in einem Bereich von 0° bis etwa 20°C durchführen. Die erhaltene Verbindung der Formel IV'a kann mittels herkömmlicher Methoden isoliert werden.

**Reaktionsschema III**

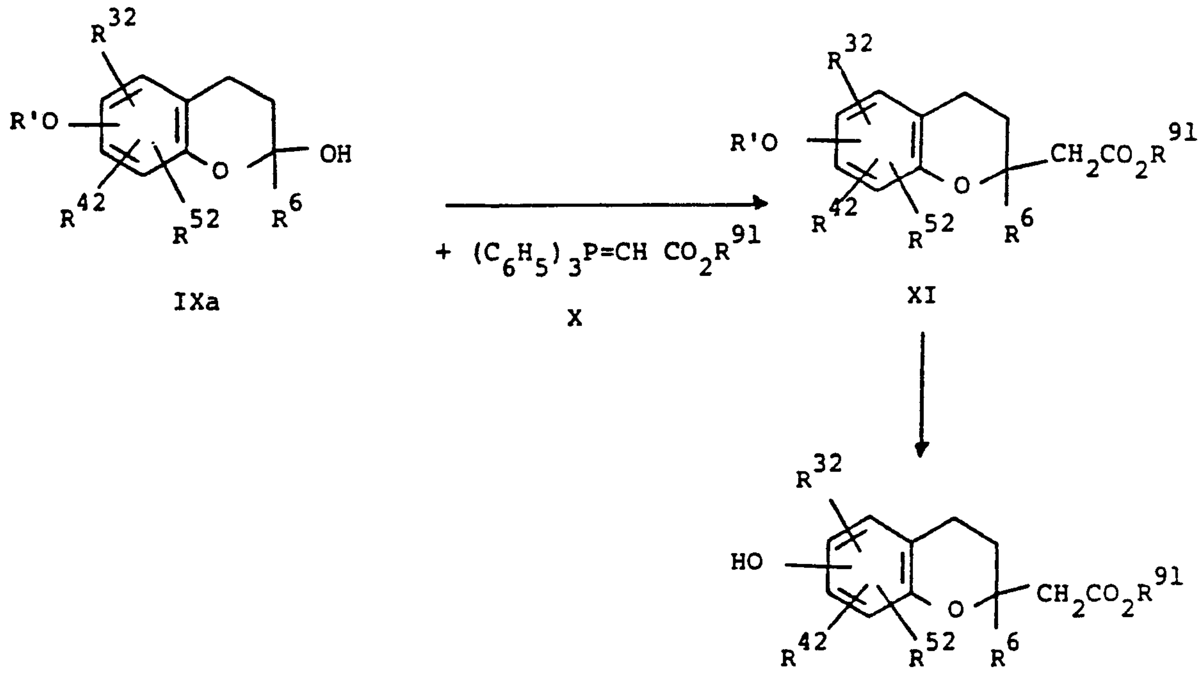


IXa

X

XI

III'b

worin $R^{32}$, $R^{42}$, $R^{52}$, $R^6$ und $R^{91}$ obige Bedeutung besitzen und $R^1$ Arylalkyl bedeutet.

Gemäss Reaktionsschema III kann man eine Verbindung der Formel IXa mit einer Verbindung der Formel $(C_6H_5)_3$-P$=$$CHCO_2R^{91}$, worin $R^{91}$ niederes Alkyl, vorzugsweise Äthyl bedeutet, umsetzen. Die Verbindungen der Formel IXa sind bekannt oder können nach an sich bekannten Methoden hergestellt werden. Man erhält bei dieser Reaktion eine Verbindung der Formel XI. Die Reaktion kann in einem inerten aromatischen Kohlenwasserstoff, vorzugsweise in Toluol, bei einer Temperatur im Bereich von etwa 20° bis etwa 150°C, vorzugsweise bei etwa 110°C, durchgeführt werden. Die erhaltene Verbindung der Formel XI kann mittels herkömmlicher Methoden isoliert werden.

Eine Verbindung der Formel XI kann mittels einer herkömmlichen katalytischen Hydrogenolyse in eine Verbindung der Formel III'b übergeführt werden. Man kann eine Verbindung der Formel XI beispielsweise in Gegenwart eines Katalysators, wie Palladium/Kohle, in einem Lösungsmittel, beispielsweise in einem Alkanol, wie Äthanol, vorzugsweise bei Raumtemperatur und bei einem Druck von einer Atmosphäre mit Wasserstoff behandeln. Die erhaltene Verbindung der Formel IIIb' kann mittels herkömmlicher Methoden isoliert werden.

**Reaktionsschema IV**

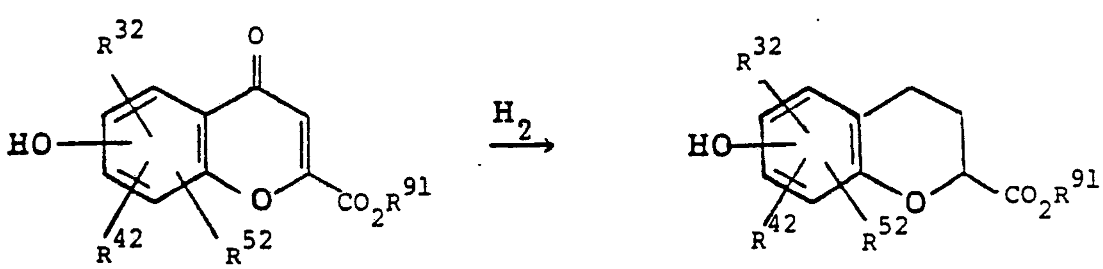


XII

III'c

worin $R^{32}$, $R^{42}$, $R^{52}$ und $R^{91}$ obige Bedeutung besitzen.

Gemäss Reaktionsschema IV wird eine Verbindung der Formel XII zu einer Verbindung der Formel III'c hydriert. Die Verbindungen der Formel XII sind bekannt oder können nach an sich bekannten Methoden hergestellt werden. Im speziellen wird die Reaktion mittels eines Katalysators, beispielsweise mittels

Palladium/Kohle, in einem Lösungsmittel, wie einer niederen Carbonsäure, vorzugsweise Essigsäure, bei einer Temperatur im Bereich von etwa 20° bis etwa 150°C, vorzugsweise bei 25°C, und bei erhöhtem Druck vorzugsweise bei 3,45 bar, durchgeführt. Die erhaltene Verbindung der Formel III'c kann mittels herkömmlicher Methoden isoliert werden.

**Reaktionsschema V**

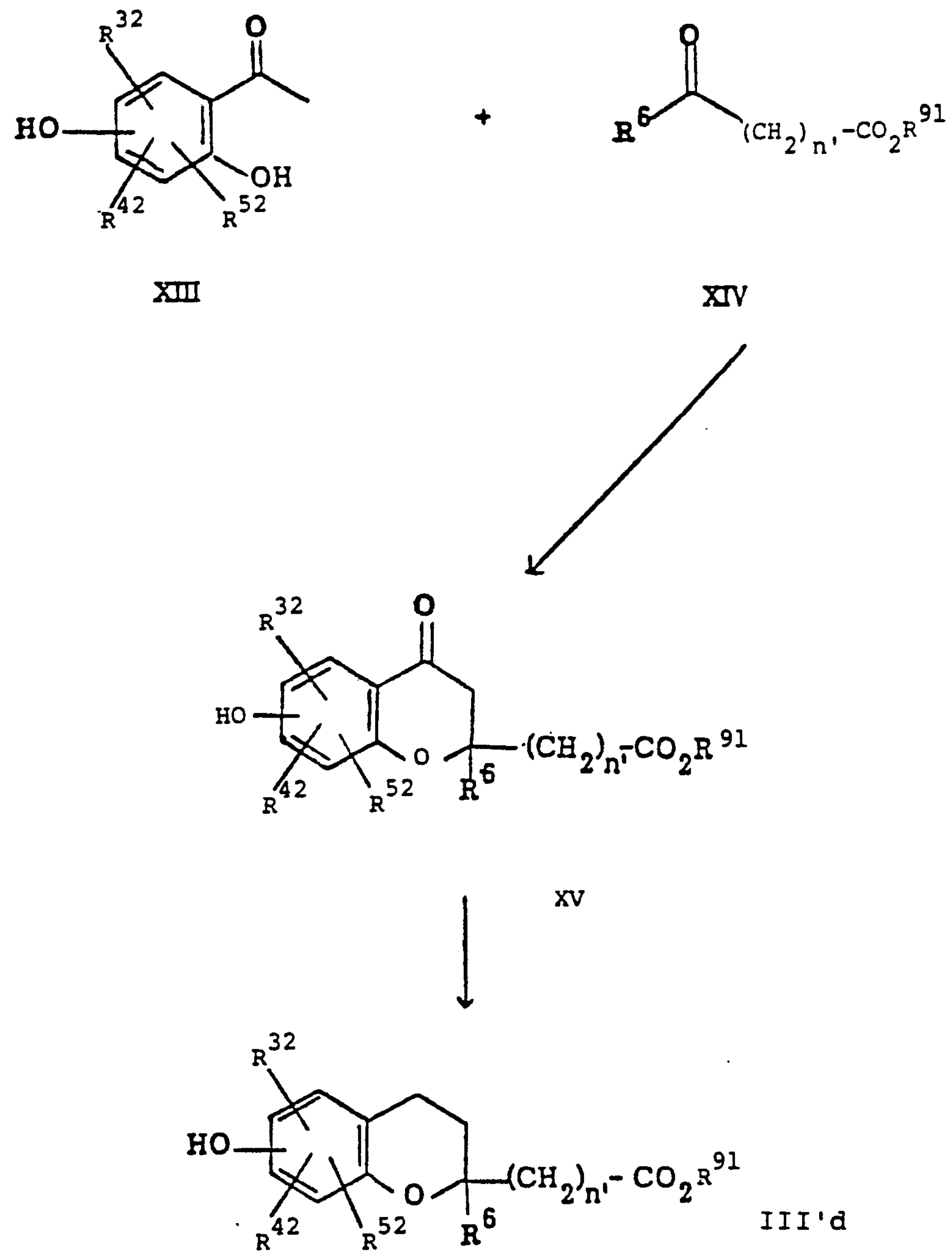

XIII

XIV

XV

III'd

worin $R^{32}$, $R^{42}$, $R^{52}$, $R^6$ und $R^{91}$ obige Bedeutung besitzen und n', 2, 3 oder 4 bedeutet.

Gemäss Reaktionsschema V wird eine Verbindung der Formel XIII mit einer Verbindung der Formel XIV zu einer Verbindung der Formel XV umgesetzt. Die Verbindungen der Formeln XIII und XIV sind bekannt oder können nach an sich bekannten Methoden hergestellt werden. Die Reaktion kann in Gegenwart eines Katalysators, beispielsweise eines cyclischen sekundären Amins, wie Pyrrolidin, und in einem inerten aromatischen Kohlenwasserstoff, vorzugsweise in Toluol, bei einer Temperatur im Bereich von etwa 25° bis etwa 150°C vorzugsweise bei 110°C, durchgeführt werden. Die erhaltene Verbindung der Formel XV kann mittels herkömmlicher Methoden isoliert werden.

Eine Verbindung der Formel XV kann beispielsweise unter Verwendung von Boran in Gegenwart einer Lewis-Säure, wie Bortrifluorid, Bortrichlorid, Aluminiumtrichlorid und dergleichen, vorzugsweise mit Bortrifluorid-Ätherat und in einem inerten Äther, wie z. B. Tetrahydrofuran, bei einer Temperatur im Bereich von etwa 0° bis etwa 25°C, vorzugsweise bei 0°C, zu einer Verbindung der Formel III'd reduziert werden. Die erhaltene Verbindung kann mittels herkömmlicher Methoden isoliert werden.

**Reaktionsschema VI**

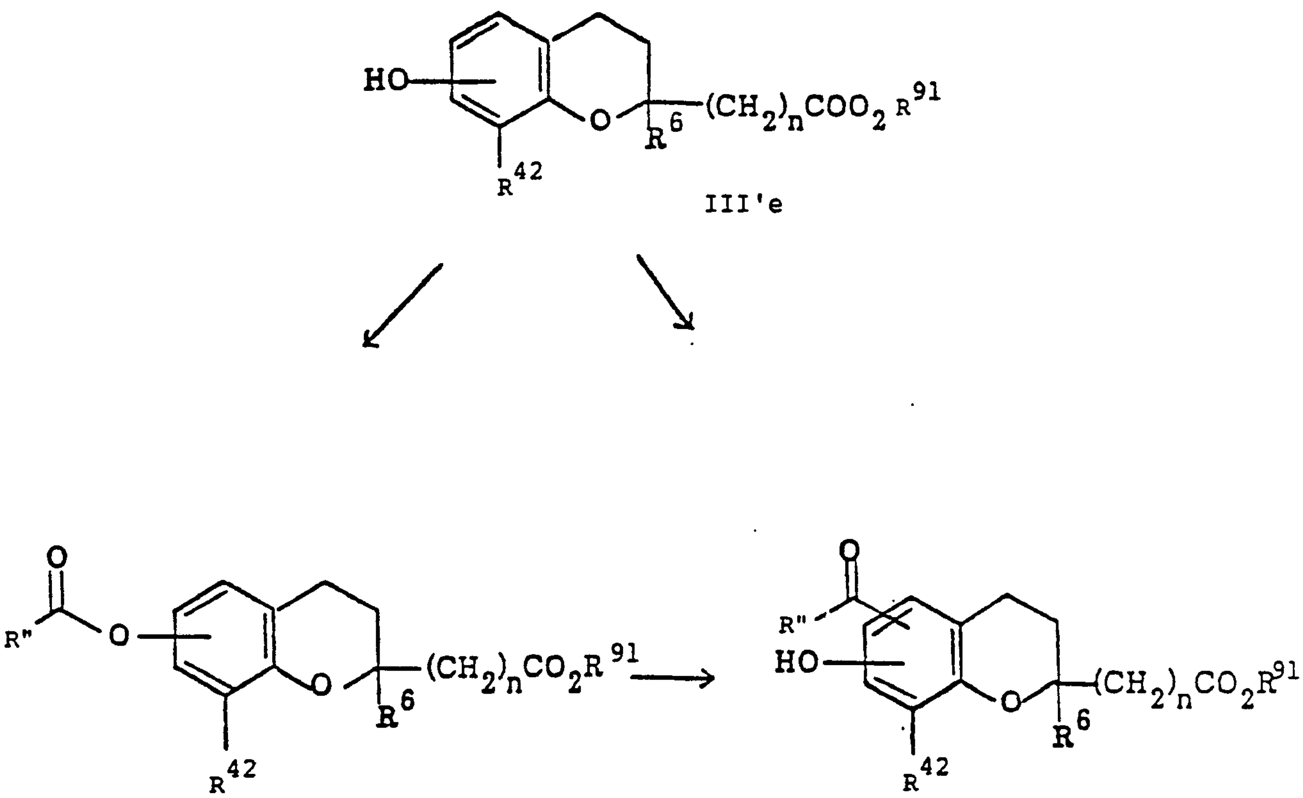

III'e

XIX

III'f

worin $R^{42}$, $R^6$, $R^{91}$ und n obige Bedeutung besitzen und R'' Wasserstoff oder niederes Alkyl bedeutet.

Gemäss Reaktionsschema VI wird ein Phenol der Formel III'e in das entsprechende Acylderivat der Formel XIX übergeführt. Man kann jedes beliebige Acylierungsmittel verwenden. Man kann beispielsweise ein niederes Alkanoylhalogenid oder Anhydrid, vorzugsweise Essigsäureanhydrid, in einem geeigneten basischen Lösungsmittel, wie z. B. Pyridin, einem niederen Alkylamin oder dergleichen, vorzugsweise in Pyridin, bei einer Temperatur im Bereich von etwa 0°C bis etwa 150°C, vorzugsweise bei 25°C, verwenden. Die erhaltene Verbindung der Formel XIX kann erwünschtenfalls mittels herkömmlicher Methoden isoliert werden.

Eine Verbindung der Formel XIX kann in eine Verbindung der Formel III'f übergeführt werden, und zwar unter Verwendung von Reaktionsbedingungen, wie sie für Fries-Umlagerungen von Phenylacylaten üblich sind. Beispielsweise kann man die Reaktion in Gegenwart einer Lewis-Säure, wie Aluminiumchlorid, Zinnchlorid, Bortrifluorid oder dergleichen, vorzugsweise in Gegenwart von Bortrifluorid-Ätherat, in einem Lösungsmittel, wie einem halogenierten Kohlenwasserstoff, einer niederen Fettsäure oder dergleichen, vorzugsweise in Essigsäure, bei einer Temperatur im Bereich von etwa 20° bis etwa 150°C, vorzugsweise bei 120°C, durchführen. Die erhaltene Verbindung der Formel III'f kann mittels herkömmlicher Methoden isoliert werden.

Andererseits kann man eine Verbindung der Formel III'e auch direkt, in eine Verbindung der Formel III'f überführen, und zwar unter Verwendung von Bedingungen, wie sie gewöhnlich für die Durchführung von Friedel-Crafts-Acylierungen von Phenolen angewendet werden. Beispielsweise kann man die Acylierung durchführen, indem man einen Lewis-Säure-Katalysator, wie Aluminiumchlorid, Zinnchlorid, Bortrifluorid oder dergleichen, vorzugsweise gasförmiges Bortrifluorid, und ein Acylierungsmittel, beispielsweise ein niederes Alkanoylhalogenid, ein niederes Alkanoylanhydrid, eine niedere Fettsäure oder dergleichen, vorzugsweise in Essigsäure, bei einer Temperatur im Bereich von etwa 20° bis etwa 150°C, vorzugsweise bei 80°C, verwendet. Die erhaltene Verbindung der Formel III'f kann mittels herkömmlichen Methoden isoliert werden.

**Reaktionsschema VII**

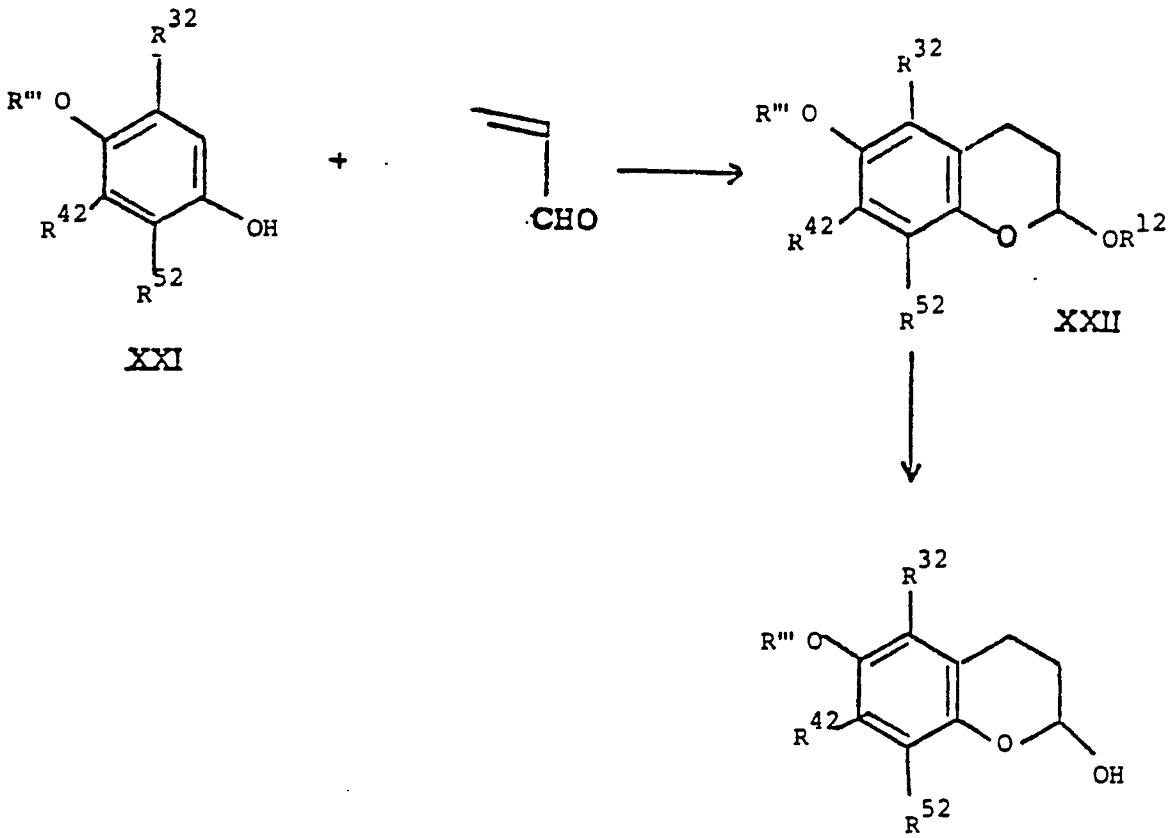

XXI

XXII

IX

worin $R^{32}$, $R^{42}$ und $R^{52}$ obige Bedeutung besitzen, R''' Wasserstoff oder Aryl-niederes Alkyl, vorzugsweise Benzyl, und $R^{12}$ niederes Alkyl bedeuten.

Gemäss Reaktionsschema VII kann man eine Verbindung der Formel XXI durch Behandeln mit Acrolein in eine Verbindung der formel XXII überführen. Man verwendet dabei als Katalysator beispielsweise eine starke Mineralsäure, wie Chlorwasserstoffsäure, Schwefelsäure, eine Aryl- oder Alkylsulfonsäure, wie p-Toluolsulfonsäure, oder dergleichen, vorzugsweise jedoch konzentrierte Schwefelsäure, in einem Lösungsmittel, z. B. in einem niederen Alkanol, wie Äthanol, Methanol oder dergleichen, vorzugsweise jedoch Methanol, bei einer Temperatur im Bereich von etwa 20° bis etwa 200°C, vorzugsweise bei 150°C. Die erhaltene Verbindung der Formel XXII kann mittels herkömmlicher Methoden isoliert werden. Die Verbindungen der Formel XXI sind bekannt oder können nach an sich bekannten Methoden hergestellt werden.

Eine Verbindung der Formel XXII kann unter Verwendung einer starken Mineralsäure, wie verdünnte Salzsäure, Schwefelsäure oder einer Aryl- oder Alkylsulfonsäure, wie p-Toluolsulfonsäure, oder dergleichen, als Katalysator, vorzugsweise unter Verwendung von verdünnter wässriger Salzsäure, in einem organischen Lösungsmittel, das mit Wasser mischbar ist, wie Aceton, Methanol, Äthanol, Tetrahydrofuran, Essigsäure oder dergleichen, vorzugsweise Aceton, bei einer Temperatur im Bereich von etwa 20° bis etwa 150°C, vorzugsweise bei 60°C, in eine Verbindung der Formel IX übergeführt werden,

**Reaktionsschema VIII**

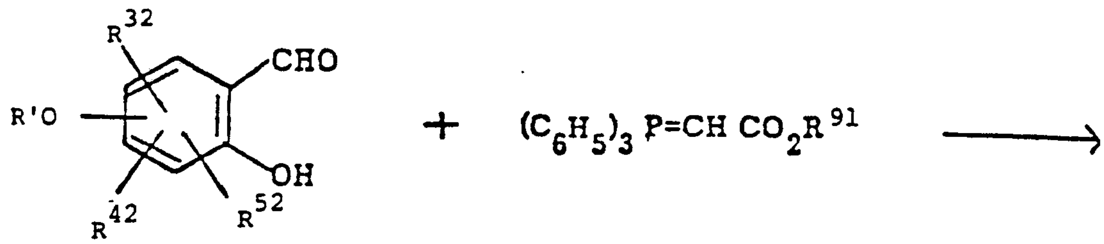

XXIII X

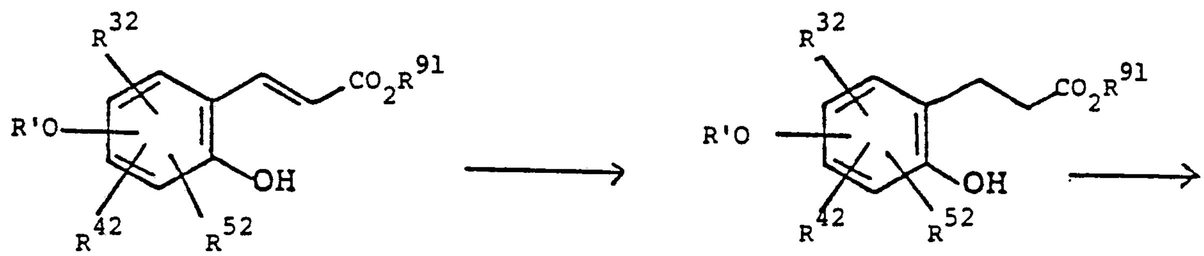

XXIV XXV

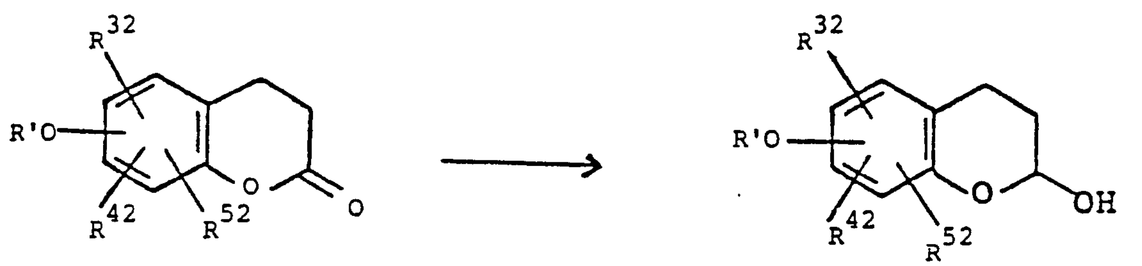

XXVI IXb

worin $R^{32}$, $R^{42}$, $R^{52}$, $R^{91}$ und R' obige Bedeutung besitzen.

Gemäss Reaktionsschema VIII kann man eine Verbindung der Formel XXIII mit einer Verbindung der Formel X umsetzen, wobei man eine Verbindung der Formel XXIV erhält. Die Verbindungen der Formeln XXIII und X sind bekannt oder können nach an sich bekannten Methoden hergestellt werden. Die Reaktion kann in einem inerten organischen Äther oder in einem aromatischen Kohlenwasserstoff, z. B. in Tetrahydrofuran, Dioxan, Glyme, Benzol, Toluol, Xylol oder dergleichen, vorzugsweise in Toluol bei einer Temperatur im Bereich von etwa 20° bis etwa 200°C, vorzugsweise bei 110°C, durchgeführt werden. Die erhaltene Verbindung der Formel XXIV kann mittels herkömmlicher Methoden isoliert werden.

Eine Verbindung der Formel XXIV kann zu einer Verbindung der Formel XXV hydriert werden. Man kann dabei einen herkömmlichen Katalysator verwenden, beispielsweise Palladium/Kohle, Platinoxid, Nickel oder dergleichen. Vorzugsweise verwendet man Platinoxid. Als Lösungsmittel kann man beispielsweise eine niedere Fettsäure, Äthylacetat oder dergleichen, vorzugsweise Äthylacetat, verwenden. Man arbeitet bei einer Temperatur im Bereich von etwa 20° bis etwa 100°C, vorzugsweise bei 20°C, und bei Atmosphärendruck. Die erhaltene Verbindung der Formel XXV kann mittels herkömmlicher Methoden isoliert werden.

Eine Verbindung der Formel XXV kann durch Verseifen und anschliessende Lactonisierung in eine Verbindung der Formel XXVI übergeführt werden. Die Verseifung kann durchgeführt werden, indem man ein Alkalimetallhydroxid, vorzugsweise Kaliumhydroxid, in einem Lösungsmittel, wie z. B. einem niederen Alkohol,

vorzugsweise Methanol, bei einer Temperatur im Bereich von etwa 20° bis etwa 100°C, vorzugsweise bei 65°C, verwendet. Die Lactonisierung kann beispielsweise mit dem Anhydrid einer niederen Fettsäure, wie Essigsäureanhydrid, das gleichzeitig auch als Lösungsmittel dienen kann, bei einer Temperatur im Bereich von etwa 20° bis etwa 200°C, vorzugsweise bei der Siedetemperatur von Essigsäureanhydrid, durchgeführt werden. Die erhaltene Verbindung der Formel XXVI kann mittels herkömmlicher Methoden isoliert werden.

Eine Verbindung der Formel XXVI kann in eine Verbindung der Formel IXb übergeführt werden, und zwar unter Verwendung eines herkömmlichen Reduktionsmittels. Man kann beispielsweise ein di-nieders Alkylaluminiumhydrid, vorzugsweise Diisobutylaluminiumhydrid, bei einer Temperatur im Bereich von etwa -50° bis etwa -100°C, vorzugsweise bei -75°C, in einem inerten Lösungsmittel, beispielsweise in einem aromatischen Kohlenwasserstoff, in einem chlorierten Alkan oder in einem niederen Alkan, vorzugsweise in Methylenchlorid, verwenden. Die erhaltene Verbindung der Formel IXb kann mittels herkömmlicher Methoden isoliert werden.

**Reaktionsschema IX**

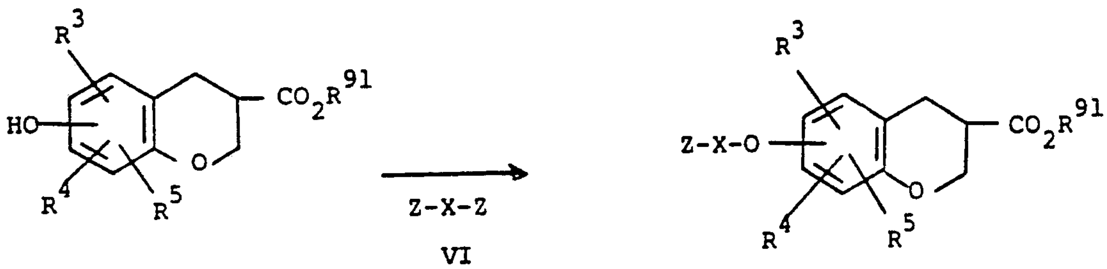


III"

IV"

worin $R^3$, $R^4$, $R^5$, $R^{91}$, X und Z obige Bedeutung besitzen.

Die Reaktion einer Verbindung der Formel VI mit einer Verbindung der Formel III zu einer Verbindung der Formel IV" gemäss Reaktionsschema IX kann in einem inerten organischen Lösungsmittel, wie Dimethylformamid, Aceton, Methyläthylketon oder dergleichen, vorzugsweise in Dimethylformamid, in Gegenwart einer Base, beispielsweise einem Alkalimetallcarbonat, wie Kaliumcarbonat, Natriumcarbonat oder dergleichen, oder einem Alkalimetallhydrid, wie Natriumhydrid oder dergleichen, bei einer Temperatur im Bereich von etwa 20° bis etwa 150°C, vorzugsweise bei Raumtemperatur, durchgeführt werden. Die erhaltene Verbindung der Formel IV" kann mittels herkömmlicher Methoden, beispielsweise mittels Kristallisation, Extraktion, Chromatographie oder dergleichen, isoliert werden.

**Reaktionsschema X**

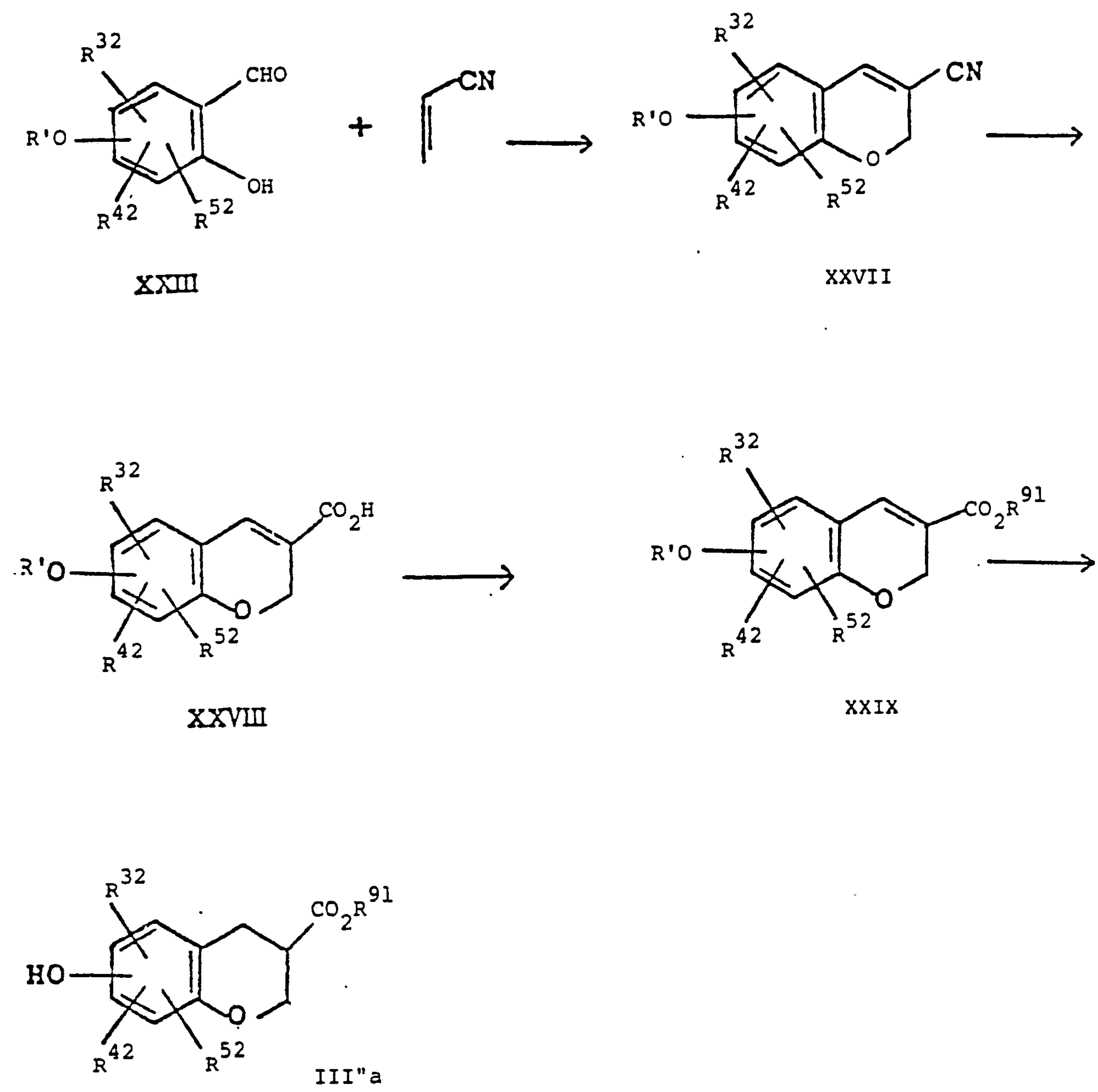

XXIII

XXVII

XXVIII

XXIX

III"a

worin $R^{32}$, $R^{42}$, $R^{52}$, $R^{91}$ und R' obige Bedeutung besitzen.

Gemäss Reaktionsschema X kann eine Verbindung der Formel XXIII durch Reaktion mit Acrylnitril zu einer Verbindung der Formel XXVII umgesetzt werden. Die Reaktion wird vorzugsweise in Gegenwart eines Katalysators. z. B. eines Amins wie Triäthylamin, Diazabicycloundecen und Diazabicyclo[2.2.2]octan (letzteres wird bevorzugt), bei einer Temperatur im Bereich von etwa 20° bis etwa 100°C, vorzugsweise bei 80°C, und ohne Lösungsmittel durchgeführt. Die erhaltene Verbindung der Formel XXVII kann mittels herkömmlicher Methoden isoliert werden.

Eine Verbindung der Formel XXVII kann durch Verseifen in eine Verbindung der Formel XXVIII übergeführt werden. Die Verseifung kann beispielsweise mit einem Alkalimetallhydroxid, wie Kaliumhydroxid, in einem Lösungsmittel, beispielsweise in einem mit Wasser mischbaren organischen Lösungsmittel, wie Tetrahydrofuran, Methanol, Äthanol oder dergleichen, vorzugsweise in Äthanol oder Tetrahydrofuran, bei einer Temperatur im Bereich von 20° bis etwa 100°C vorzugsweise zwischen 60° und 70°C, durchgeführt werden. Die erhaltene Verbindung der Formel XXVIII kann mittels herkömmlicher Methoden isoliert werden.

Durch Verestern einer Verbindung der Formel XXVIII kann man eine Verbindung der Formel XXIX herstellen. Die Veresterung kann beispielsweise so durchgeführt werden, dass man eine Verbindung der Formel XXVIII in einem Lösungsmittel, z. B. Äthanol, bei einer Temperatur im Bereich von etwa 20° bis etwa 100°C, vorzugsweise bei 78°C, und in Gegenwart eines Katalysators, beispielsweise einer starken Mineralsäure, wie

Schwefelsäure, p-Toluolsulfonsäure oder dergleichen, vorzugsweise p-Toluolsulfonsäure, mit einem niederen Alkohol umsetzt. Die erhaltene Verbindung der Formel XXIX kann mittels herkömmlicher Methoden isoliert werden.

Eine Verbindung der Formel XXIX kann mittels katalytischer Hydrierung in eine Verbindung der Formel III"a übergeführt werden. Man kann beispielsweise Palladium auf einem Träger, Nickel, Platin, vorzugsweise Palladium/Kohle, in Gegenwart eines Lösungsmittels, beispielsweise eines niederen Alkohols, Essigsäure, Äthylacetat oder dergleichen, vorzugsweise Äthanol, bei einer Temperatur im Bereich von etwa 20° bis 200° C, vorzugsweise bei 20° C, und bei Atmosphärendruck verwenden. Die erhaltene Verbindung der Formel III"a kann mittels herkömmlicher Methoden isoliert werden.

**Reaktionsschema XI**

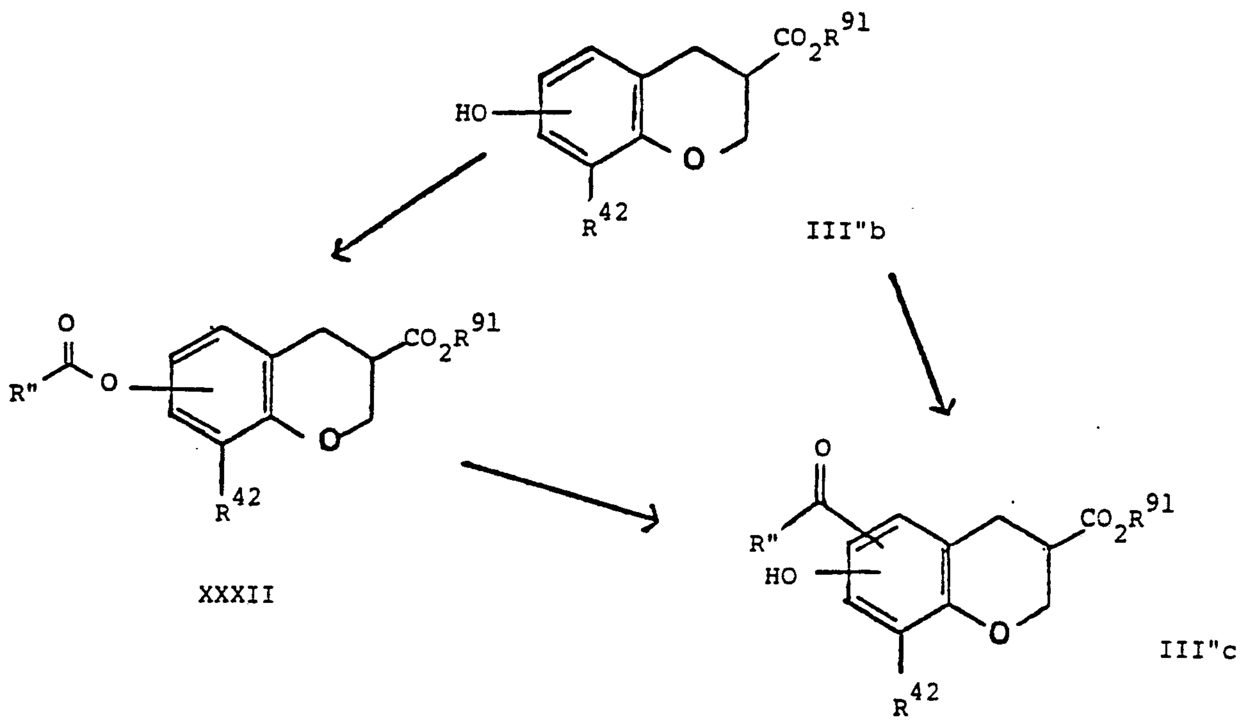

III"b

XXXII

III"c

worin $R^{42}$, $R^{91}$ und R" obige Bedeutung besitzen.

Gemäss Reaktionsschema XI kann eine Verbindung der Formel III"b in eine Verbindung der Formel III"c übergeführt werden, und zwar direkt oder über die Verbindung der Formel XXXII. Diese Reaktionen erfolgen in Analogie zur Herstellung von Verbindungen der Formel III'f aus Verbindungen der Formel III'e gemäss Reaktionsschema VI.

**Reaktionsschema XII**

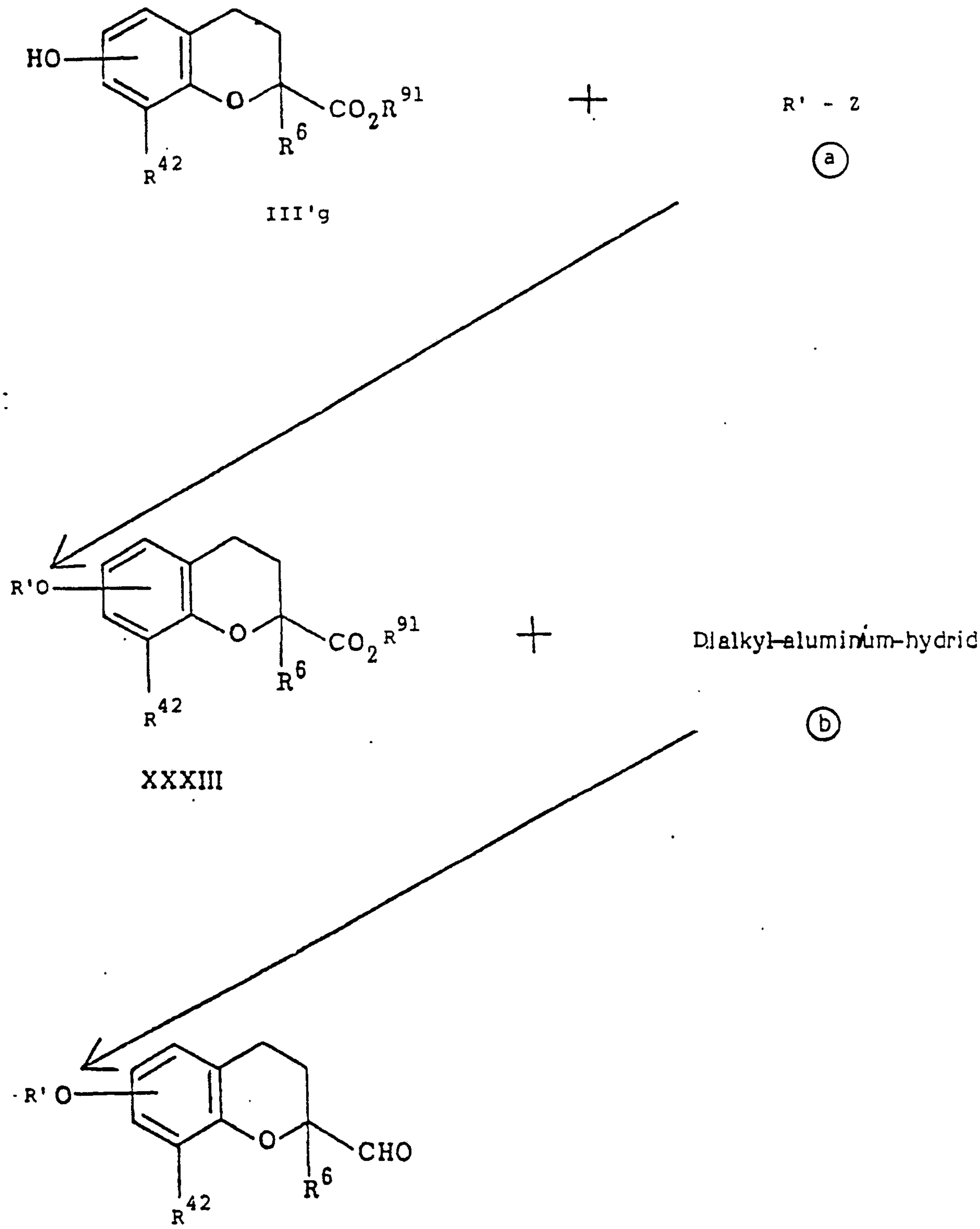


III'g

XXXIII

XXXIV

worin $R^{42}$, $R^{6}$, $R^{91}$ und R' obige Bedeutung besitzen und Z Halogen bedeutet.

Die Reaktion einer Verbindung der Formel III'g mit einer Verbindung der Formel (a) zu einer Verbindung der Formel XXXIII gemäss Reaktionsschema XII kann unter wasserfreien Bedingungen in einem inerten Lösungsmittel, wie Aceton, Methyläthylketon, Diäthylketon, Dimethylformamid oder dergleichen, bei der Rückflusstemperaatur der Reaktionsmischung, vorzugsweise bei einer Temperatur im Bereich von etwa 40° bis etwa 100°C, und in Gegenwart eines säurebindenden Mittels, wie Kaliumcarbonat oder dergleichen, durchgeführt werden. Das bevorzugte Lösungsmittel ist Aceton. Die erhaltene Verbindung der Formel XXXIII

kann mittels herkömmlicher Methoden. beispielsweise mittels Kristallisation, Chromatographie oder dergleichen, isoliert werden.

Eine Verbindung der Formel XXXIII kann in eine Verbindung der Formel XXXIV übergeführt werden, und zwar unter Verwendung eines herkömmlichen Reduktionsmittels, beispielsweise unter Verwendung eines diniederen Alkylaluminiumhydrids, vorzugsweise von Diisobutylaluminiumhydrid, bei einer Temperatur im Bereich von etwa -50° bis etwa -100° C, vorzugsweise bei -75° C, in einem inerten Lösungsmittel, beispielsweise einem aromatischen Kohlenwasserstoff, einem chlorierten Alkan oder einem niederen Alkan, vorzugsweise in Toluol. Die erhaltene Verbindung der Formel XXXIV kann mittels herkömmlicher Methoden isoliert werden.

**Reaktionsschema XIII**

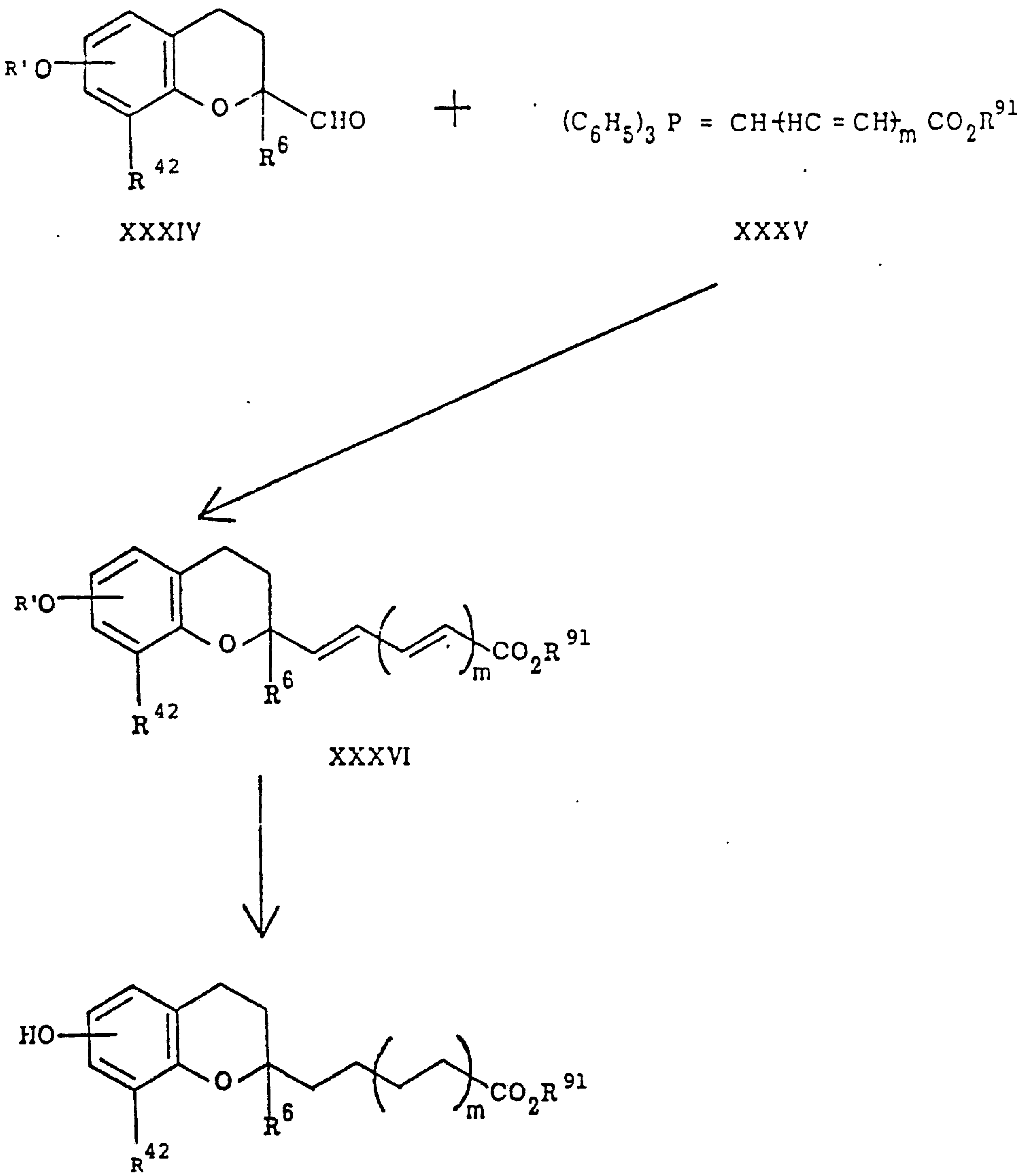

XXXIV

XXXV

XXXVI

III'h

worin m 0 oder 1 bedeutet und $R^{42}$, $R^6$, $R^{91}$ und R' obige Bedeutung besitzen.

Die Reaktion einer Verbindung der Formel XXXIV mit einer Verbindung der Formel $(C_6H_5)_3P=CH(CH=CH)_m-CO_2R^{91}$, worin $R^{91}$ niederes Alkyl, vorzugsweise Äthyl, und m die Zahl 0 oder 1 bedeuten, gemäss Reaktionsschema XIII zu einer Verbindung der Formel XXXVI kann in einem inerten aromatischen Lösungsmittel, vorzugsweise in Toluol, bei einer Temperatur im Bereich von etwa 20° bis etwa 150°C, vorzugsweise bei etwa 110°C, durchgeführt werden. Die erhaltene Verbindung der Formel XXXVI kann mittels herkömmlicher Methoden isoliert werden.

Eine Verbindung der Formel XXXVI kann mittels katalytischer Hydrierung in an sich bekannter Weise in eine Verbindung der Formel III'h übergeführt werden. Man kann beispielsweise eine Verbindung der Formel XXXVI in Gegenwart eines Katalysators, wie Palladium/Kohle, in einem Lösungsmittel, beispielsweise in einem Alkohol, wie Äthanol, oder in Äthylacetat, vorzugsweise bei Raumtemperatur und Atmosphärendruck mit Wasserstoff behandeln. Die erhaltene Verbindung der Formel III'h kann mittels herkömmlicher Methoden isoliert werden.

Die vorliegende Erfindung betrifft auch pharmazeutisch annehmbare Salze von Verbindungen der Formel I, worin $R^9$ Wasserstoff bedeutet. Diese Salze können dadurch hergestellt werden, dass man eine Carbonsäure der Formel I mit einer Base, welche ein nicht-toxisches, pharmakologisch und pharmazeutisch annehmbares Kation besitzt, umsetzt. Ganz allgemein umfasst die vorliegende Erfindung Salze mit sämtlichen Basen, welche mit den vorliegenden Carbonsäuren ein Salz bilden und deren pharmakologische Eigenschaften nicht zu nachteiligen physiologischen Reaktionen führen, wenn sie durch einen Warmblüter eingenommen werden. Geeignete Basen sind beispielsweise Alkalimetall- und Erdalkalimetallhydroxide und -carbonate, z. B. Natriumhydroxid, Kaliumhydroxid, Calciumhydroxid, Kaliumcarbonat und dergleichen, Ammoniak, primäre, sekundäre und tertiäre Amine, wie Monoalkylamine, Dialkylamine, Trialkylamine, stickstoffhaltige heterocyclische Amine, z. B. Piperidin, Aminosäuren, wie Lysin, und dergleichen. Die so erhaltenen pharmazeutisch annehmbaren Salze sind funktionelle Äquivalente der entsprechenden Carbonsäuren der Formel I. Dem Fachmann bereitet die Erkennung des Umfanges der therapeutisch verwertbaren, erfindungsgemässen Salze keine Schwierigkeiten, da die Vielfalt der durch die vorliegende Erfindung umfassten Salze nur durch die Kriterien beschränkt wird, dass die für die Herstellung dieser Salze verwendeten Basen nicht-toxisch und physiologisch annehmbar sind.

Die nützlichen antiallergischen Eigenschaften der Verbindungen der Formel I können mittels Standard-Tests in vitro und in Warmblütern ermittelt werden. Beispiele solcher Tests sind:

a) Meerschweinchen-Illeum-Test (in vitro):

Der Meerschweinchen-Illeum-Test wurde durch Orange und Austen in Adv. Immunol. 10, 105-144 (1969) beschrieben. Ein 1,5 cm grosses Illeum-Segment aus einem 300-400 g schweren Meerschweinchen wird in einem Organbad, das 10 ml Tyrodeslösung, $10^{-6}$M Atropinsulfat und $10^{-6}$M Pyrilaminmaleat enthält, suspendiert. Das Bad wird bei 37°C gehalten und mit einer Mischung aus 95 % Sauerstoff und 5 % Kohlendioxid belüftet. Das in diesem Versuch verwendete SRS-A erhält man, indem man zerkleinerte Lungenstücke von aktiv sensibilisierten Meerschweinchen in vitro mit Eieralbumin anregt. Man erstellt eine Dosis-Wirkungs-Kurve für die Wirkung von SRS-A auf das Illeum. Für die weiteren Versuche verwendet man diejenige Dosis von SRS-A, welche 50 % der maximalen Anzahl Kontraktionen bewirkt ($ED_{50}$). Man ermittelt dann diejenige Dosis an Testverbindung, welche die durch SRS-A bewirkten Kontraktionen des Meerschweinchen-Illeums um 50 % reduziert ($IC_{50}$). In diesem Test hat der Standard SRS-A-Antagonist 7[3-(4-Acetyl-3-hydroxy-2-propylphenoxy)-2-hydroxypropoxy]-4-oxo-8-propyl-4H-1-benzopyran-2-carbonsäure eine $IC_{50}$ von $3,5 \times 10^{-8}$M.

b) Meerschweinchen-Bronchokonstriktions-Test (in vivo):

Man anästhetisiert 300-500 g schwere, männliche Meerschweinchen intraperitoneal mit 2 g/kg Urethan und führt eine Polyäthylenkanüle in die Vena jugularis ein, durch welche Wirkstoffe verabreicht werden können. Mittels einer in die Trachea eingeführten Kanüle, welche mit einem Statham-Druck-Transduktor verbunden ist, wird der Trachealdruck aufgezeichnet. Nach chirurgischer Präparierung der Versuchstiere wartet man eine gewisse Zeit, damit die pulmonalen Funktionen sich stabilisieren können. Die zu testende Verbindung wird anschliessend gemäss dem nachfolgenden Protokoll verabreicht. Man verabreicht den Versuchstieren, welche spontan atmen, 0.1 mg/kg Propanolol intravenös. 5 Minuten später werden die Versuchstiere während 5 Minuten einer 1-proz. Aerosollösung der zu testenden Verbindung (Gew./Vol.) (auf einen alkalischen pH eingestellt, sofern für die Auflösung des Wirkstoffes notwendig) oder destilliertem Wasser mit einem geeigneten pH (zu Kontrollzwecken) ausgesetzt. Für alle Testverbindungen, welche durch Inhalation verabreicht werden, verwendet man ein Monaghan-Ultraschall-Sprühgerät (Modell 750). Die Ultraschallfrequenz des Sprühgerätes wird so eingestellt, dass der Durchmesser der produzierten Partikel 1-8 µ, durchschnittlich 3 µ beträgt. Wässrige Lösungen werden jeweils frisch hergestellt und in die Kammer des Sprühgerätes eingeführt. Der produzierte Sprühnebel wird den Versuchstieren verabreicht, indem man einen Strahl des Aerosols durch eine mit der Trachealkanüle verbundenen Y-Rohr leitet. Nach Ablauf der Behandlungszeit werden die Versuchstiere mit 1,2 mg/kg (i.v.) Succinylcholin paralysiert und mit einem Harvard-Rodent-Respirator bei 40 Atemzügen/Minute und einem Atemvolumen von 2,5 $cm^3$ mechanisch beatmet. 30 Sekunden nach der Verabreichung des Succinylcholins werden die Versuchstiere mit einem maximal konstringierenden Dosis von Leukotrien $E_4$ intravenös behandelt.

Die abgelesene Differenz (in cm $H_2O$) zwischen Vor- und Spitzenventilationsdruck wird gemittelt, und zwar

über 3 Werte bei Kontrolltieren und über 5 Werte bei Versuchstieren, welche mit Testsubstanz behandelt worden sind. Die prozentuale Hemmung wird mittels der folgenden Formel berechnet:

$$\frac{\text{Kontrollwert - Testsubstanzwert}}{\text{Kontrollwert.}} \times 100$$

Werden verschiedene Wirkstoffkonzentrationen untersucht, so wird die prozentuale Hemmung für jede Konzentration aufgezeichnet, und zwar wird der log Konzentration auf der Abszisse gegen die prozentuale Hemmung auf der Ordinate aufgetragen. Die $IC_{50}$ wird dann mittels linearer Regressionsanalyse ermittelt.

Wenn die in der nachfolgenden Tabelle I aufgeführten Verbindungen der Formel I als Testverbindungen verwendet werden, so erhält man die in der Tabelle I zusammengestellten Resultate:

**Tabelle I**

| | SRS-A Antagonismus | |
|---|---|---|
| Testverbindung | Meerschweinchen-Ileum-Test (in vitro) $IC_{50}$ (m) | Meerschweinchen-Bronchokonstriktions Test (in vivo; Aerosol) $IC_{50}$ (%) |
| rac-6-[3-(4-Acetyl-3-hydroxy-2-propylphenoxy)-propoxy]-3,4-dihydro-2,5,7,8-tetramethyl-2H-1-benzopyran-2-carbonsäure | $1 \times 10^{-7}$ | 1,0 |
| rac-7-[3-(4-Acetyl-3-hydroxy-2-propylphenoxy)-propoxyl]-3,4-dihydro-2-methyl-2H-1-benzopyran-2-propansäure | $1 \times 10^{-7}$ | >1,0 |
| rac-6-[3-(4-Acetyl-3-hydroxy-2-propylphenoxy)-propoxy]-3,4-dihydro-2,5,7,8-tetramethyl-2H-1-benzopyran-2-essigsäure | $2 \times 10^{-7}$ | 1,0 |
| rac-7-[3-(4-Acetyl-3-hydroxy-2-propylphenoxy)-propoxy]-3,4-dihydro-2-methyl-8-propyl-3H-1-benzopyran-2-essigsäure | $5 \times 10^{-7}$ | 0,71 |
| rac-6-[3-(4-Acetyl-3-hydroxy-2-propylphenoxy)-propoxy]-3,4-dihydro-2-methyl-2H-1-benzopyran-2-carbonsäure | $1 \times 10^{-7}$ | 0,20 |
| rac-6-[3-(4-Acetyl-3-hydroxy-2-propylphenoxy)-propoxy]-3,4-dihydro-2-methyl-2H-1-benzopyran-2-propansäure | $5 \times 10^{-7}$ | >1,0 |
| rac-6-Acetyl-7-[3-(4-acetyl-3-hydroxy-2-propylphenoxy) propoxy-2-methyl-8-propyl-2H-1-benzopypan-2-propansäure | $8 \times 10^{-8}$ | 0,12 |
| rac-6-[3-(4-Acetyl-3-hydroxy-2-propylphenoxy)-propoxy]-3,4-dihydro-5,7,8-trimethyl-2H-1-benzopyran-2-carbonsäure | $1 \times 10^{-7}$ | 1,0 |
| rac-7-[3-(4-Acetyl-3-hydroxy-2-propylphenoxy)-propoxy]-3,4-dihydro-2H-1-benzopyran-2-essigsäure | $1 \times 10^{-7}$ | 0,67 |
| rac-6-[3-(4-Acetyl-3-hydroxy-2-propylphenoxy)-propoxy]-3,4-dihydro-2H-1-benzopyran-2-essigsäure | $4 \times 10^{-7}$ | 1,0 |
| rac-6-[3-(4-Acetyl-3-hydroxy-2-propylphenoxy)-propoxy]-3,4-dihydro-2H-1-benzopyran-2-carbonsäure | $5 \times 10^{-7}$ | 0,50 |

| | | |
|---|---|---|
| rac-7-[3-(4-Acetyl-3-hydroxy-2-propylphenoxy)-propoxy]-3,4-dihydro-8-propyl-2H-benzopyran-2-carbonsäure | $1 \times 10^{-7}$ | 0,20 |
| rac-7-[3-(4-Acetyl-3-hydroxy-2-propylphenoxy)-propoxy]-3,4-dihydro-2H-1-benzopyran-2-carbonsäure | $6 \times 10^{-8}$ | 0,14 |
| rac-7-[3-(4-Acetyl-3-hydroxy-2-propylphenoxy)-propoxy]-3,4-dihydro-2H-1-benzopyran-3-carbonsäure | $1 \times 10^{-7}$ | >1,0 |
| rac-6-Acetyl-7-[3-(4-acetyl-3-hydroxy-2-propylphenoxy) propoxy]-3,4-dihydro-2H-1-benzopyran-2-carbonsäure | $1 \times 10^{-7}$ | 0,20 |
| rac-6-Acetyl-7-[3-(4-acetyl-3-hydroxy-2-propylphenoxy) propoxy]-3,4-dihydro-8-propyl-2H-l-benzopyran-2-carbonsäure | $1 \times 10^{-7}$ | 0,38 |
| rac-7-Acetyl-6-[3-(4-acetyl-3-hydroxy-2-propylphenoxy) propoxy]-3,4-dihydro-2H-1-benzopyran-2-carbonsäure | $2 \times 10^{-7}$ | 0,34 |
| rac-6-Acetyl-7-[5-(4-acetyl-3-hydroxy-2-propylphenoxy) pentyloxy]-3,4-dihydro-2H-1-benzopyran-2-carbonsäure | $6 \times 10^{-8}$ | 0,052 |
| rac-6-Acetyl-7-[[7-(4-acetyl-3-hydroxy-2-propylphenoxy) heptyl]oxy]-3,4-dihydro-2H-1-benzopyran-2-carbonsäure | $1 \times 10^{-6}$ | 1,0 |
| rac-8-Acetyl-7-[3-(4-acetyl-3-hydroxy-2-propylphenoxy) propoxy]-3,4-dihydro-2H-1-benzopyran-2-carbonsäure | $3 \times 10^{-7}$ | 0,59 |
| rac-7-Acetyl-6-[3-(4-acetyl-3-hydroxy-2-propylphenoxy) propoxy]-3,4-dihydro-2-methyl-2H-1-benzopyran-2-carbonsäure | $5 \times 10^{-7}$ | 1,0 |
| rac-7-[5-(4-Acetyl-3-hydroxy-2-propylphenoxy)-pentyloxy]-3,4-dihydro-2H-1-benzopyran-2-carbonsäure | $2 \times 10^{-6}$ | |
| rac-6-Acetyl-7-[5-(4-acetyl-3-hydroxyphenoxy)-pentyloxy]-3,4-dihydro-8-propyl-2H-1-benzopyran-2-carbonsäure | $1 \times 10^{-6}$ | |
| rac-6-Acetyl-7-[5-(4-acetyl-3-hydroxyphenoxy)-pentyloxy]-3,4-dihydro-2H-1-benzopyran-2-carbonsäure | $3 \times 10^{-6}$ | |
| rac-6-Acetyl-7-[5-(4-acetyl-3-hydroxy-2-propylphenoxy) pentyloxy]-3,4-dihydro-8-propyl-2H-1-benzopyran-2-carbonsäure | $5 \times 10^{-7}$ | |
| rac-6-[5-(4-Acetyl-3-hydroxy-2-propylphenoxy)-pentyloxy]-3,4-dihydro-2,5,7,8-tetramethyl-2H-1-benzopyran-2-carbonsäure | $1 \times 10^{-7}$ | |
| rac-7-[5-(4-Acetyl-3-hydroxyphenoxy)-pentyloxy]-3,4-dihydro-2H-1-benzopyran-2-carbonsäure | $5 \times 10^{-6}$ | |

| | |
|---|---|
| rac-6-Acetyl-7-[6-(4-acetyl-3-hydroxy-2-propylphenoxy) hexyloxy]-3,4-dihydro-2H-1-benzopyran-2-carbonsäure | $3 \times 10^{-6}$ |
| rac-6-Acetyl-7-[4-(4-acetyl-3-hydroxyy-2-propylphenoxy) butoxy]-3,4-dihydro-2H-1-benzopyran-2-carbonsäure | $2 \times 10^{-6}$ |
| rac-7-[5-(4-Acetyl-3-hydroxyphenoxy)pentyloxy]-3,4-dihydro-8-propyl-2H-1-benzopyran-2-carbonsäure | $6 \times 10^{-6}$ |
| rac-7-[5-(4-Acetyl-3-hydroxy-2-propylphenoxy)-pentyloxy]-3,4-dihydro-8-propyl-2H-1-benzopyran-2-carbonsäure | $8 \times 10^{-7}$ |
| (+)-(R)-6-[5-(4-Acetyl-3-hydroxy-2-propylphenoxy)-pentyloxy]-3,4-dihydro-2,5,7,8-tetramethyl-2H-1-benzopyran-2-carbonsäure | $5 \times 10^{-7}$ |
| (-)-(S)-6-[5-(4-Acetyl-3-hydroxy-2-propylphenoxy)-pentyloxy]-3,4-dihydro-2,5,7,8-tetramethyl-2H-1-benzopyran-2-carbonsäure | $8 \times 10^{-7}$ |
| (S)-6-Acetyl-7-[5-(4-acetyl-3-hydroxy-2-propylphenoxy)-pentyloxy]-3,4-dihydro-2H-1-benzopyran-2-carbonsäure | $6 \times 10^{-8}$ |
| (R)-6-Acetyl-7-[5-(4-acetyl-3-hydroxy-2-propylphenoxy)-pentyloxy]-3,4-dihydro-2H-1-benzopyran-2-carbonsäure | $1 \times 10^{-7}$ |

Wenn man in obigem Aerosol-Versuch Leukotrien-$E_4$ durch Leukotrien-$D_4$ ersetzt, erhält man für rac-6-Acetyl-7-[5-(4-acetyl-3-hydroxy-2-propylphenoxy)pentyloxy]-3,4-dihydro-2H-1-benzypyran-2-carbonsäure ein $IC_{50}$ von 0,0056 %, für (R)-(-)-6-Acetyl-7-[5-(4-acetyl-3-hydroxy-2-propylphenoxy)pentyloxy]-3,4-dihydro-2H-1-benzopyran-2-carbonsäure eine $IC_{50}$ von 0,018 % und für (S)-(+)-6-Acetyl-7-[5-(4-acetyl-3-hydroxy-2-propylphenoxy)pentyloxy]-3 4-dihydro-2H-1-benzopyran-2-carbonsäure eine $IC_{50}$ von 0,0018 %.

c) Orale Prüfung auf Leukotrien-Antagonismus

Man anästhetisiert männliche, 400-600 g schwere Meerschweinchen intraperitoneal mit 2 g/kg Urethan und führt eine Polyäthylenkanüle in die Vena jugularis ein, durch welche Wirkstoffe verabreicht werden können. Der tracheale Druck (in cm $H_2O$) wird mit einem Statham-Druck-Transduktor aufgezeichnet. Nach chirurgischen Präparierung der Versuchstiere wartet man eine gewisse Zeit, damit sich die spontane Atmung stabilisieren kann. Da frühere Studien gezeigt haben, dass Propanolol (0,1 mg/kg i.v,) einen potenzierenden Effekt auf die durch synthetische Leukotriene bewirkte Bronchokonstriktion hat, wird das Propanolol 5 Minuten vor der Behandlung mit Leukotrien verabreicht. 2 Minuten später paralysiert man die Atmung mit 1,2 mg/kg (i.v.) Succinylcholin-Chlorid und beatmet die Tiere mechanisch mit einem Beatmungsgerät für Kleintiere (Harvard Model 680) bei 40 Atemzügen/Minute und einem Beatmungsvolumen von 2,5 $cm^3$. Anschliessend behandelt man die Versuchstiere während 5 Minuten mit einer maximalkonstringierenden Dosis von Leukotrien-$C_4$, Leukotrien-$D_4$ oder Leukotrein-$E_4$ (25 µg/kg, i.v.). 2 Stunden vor der Behandlung mit dem Leukotrien verabreicht man 10 ml/kg (p.o.) der zu testenden Verbindung (in Form einer Lösung, welche auf einen alkalischen pH eingestellt wird, sofern für die Auflösung des Wirkstoffes notwendig) oder ein Kontrollvehikel. Um für eine Testverbindung die $ID_{50}$ zu bestimmen wird die Dosis von 10 mg/kg (p.o.) auf 100, 50, 30, 20, 5, 3 und 1 mg/kg (p.o.) variiert.

Um die orale Wirkungsdauer zu bestimmen, wird die Zeit zwischen Behandlung mit der zu testenden Verbindung und dem Leukotrien variiert.

Unter den Verbindungen die in diesem Test untersucht worden sind, zeigte rac-6-Acetyl-7-[5-(4-acetyl-3-hydroxy-2-propylphenoxy)pentyloxy]-3,4-dihydro-2H-1-benzopyran-2-pentansäure eine Hemmung der durch Leukotrien-$C_4$ bewirkten Bronchokonstriktion um 46 ± 14 % nach oraler Verabreichung von 10 mg/kg.

Die Verbindungen der Formel I oder, wenn $R^9$ Wasserstoff bedeutet, ihre Salze oder Arzneimittel, enthaltend eine therapeutisch wirksame Menge einer Verbindung der Formel I oder eines Salzes davon können mittels bestens bekannter Methoden verabreicht werden. Die Verbindungen der Formel I und ihre Salze können als Einzelsubstanzen verabreicht werden oder zusammen mit anderen Wirkstoffen, z. B. zusammen mit Antihistaminika, Inhibitoren von freigesetzten Vermittlersubstanzen, Methylxanthinen, β-Antagonisten oder antiasthmatischen Steroiden, wie Prednison oder Prednisolon. Sie können oral, parenteral, rektal oder durch Inhalation, z. B. in Form eines Aerosols, eines mikropulverisierten Puders oder in Form einer versprühten Lösung, verabreicht werden. Für die orale Verabreichung eignen sich Tabletten, Kapseln, z. B. in Mischungen mit Talk, Stärke, Milchzucker oder anderen inerten Hilfsstoffen, d. h. pharmazeutisch annehmbaren Trägern, oder wässrige Lösungen, Suspensionen, Elixiere oder wässrige alkoholische Lösungen, z. B. in Mischungen mit Zucker oder anderen Süsstoffen, Aromastoffen, Färbemitteln, Verdickungsmitteln und anderen herkömmlichen pharmazeutischen Hilfsstoffen. Für die parenterale Verabreichung eignen sich Lösungen oder Suspensionen, z. B. wässrige Lösungen oder Erdnussöl-Lösungen oder Suspensionen und Verwendung von üblichen Hilfsstoffen und Trägermaterialien. Für die Verabreichung in Form von Aerosolen können diese Substanzen in einem geeigneten, pharmazeutisch annehmbaren Lösungsmittel gelöst werden, z. B. in Äthanol oder Wasser oder in

Kombinationen von mischbaren Lösungsmitteln, und mit einem pharmazeutisch annehmbaren Treibgas vermischt werden. Solche Aerosole werden in geeignete Druckflaschen abgefüllt, welche mit einem für solche Zwecke geeigneten Ventil versehen sind. Dieses Ventil ist vorzugsweise ein Dosierungsventil, d. h. ein Ventil, das bei Betätigung eine vorbestimmte Menge einer wirksamen Dosis des Aerosols freigibt.

Die Verbindungen der Formel I werden ganz besonders bevorzugt durch Inhalation, z. B. in Form eines Aerosols, verabreicht und zwar insbesondere als antiasthmatisches Mittel.

Die Dosierung der zu verabreichenden Verbindung der Formel I oder des Salzes davon und die Häufigkeit der Verabreichung hängen ab von der Wirksamkeit und der Länge der Wirkung dieser Verbindung, und dar Art der Verabreichung, sowie von der Stärke der behandelten Krankheit, vom Alter des zu behandelnden Säugers und dergleichen. Im allgemeinen liegt die tägliche Dosis einer Verbindung der Formel I oder eines Salzes davon in einem Bereich von etwa 25 bis etwa 1000 mg, vorzugsweise zwischen 25 und etwa 250 mg, wobei die Verabreichung in einer einzigen Dosierung oder in mehreren Dosen erfolgen kann.

Da die erfindungsgemässen Verbindungen der Formel I, ein asymmetrisch substituiertes Kohlenstoffatom enthalten, fallen sie gewöhnlich in Form racemischer Mischungen an. Die Spaltung solcher Racemate in die optisch aktiven Isomeran kann mittels bekannter Methoden durchgeführt werden. Gewisse racemische Mischungen können als Eutektika ausgefällt und danach aufgetrennt werden. Man verwendet allerdings vorzugsweise die chemische Aufspaltung. Bei dieser Methode wird die racemische Mischung einer Verbindung der Formel I. worin $R^9$ Wasserstoff bedeutet, mit einem optisch aktiven Spaltungsmittel, beispielsweise einer optisch aktiven Base, wie d-(+)-α-Methylbenzylamin, das mit der Carboxylgruppe reagieren kann, in Diastereoisomere übergeführt. Die erhalten Diastereoisomeren können durch fraktionierte Kristallisation aufgetrennt und dann in die entsprechenden optischen Isomeren übergeführt werden. Die vorliegende Erfindung betrifft demnach sowohl die Racemate von Verbindungen der Formel I als auch ihre optisch akiven Formen (Enantiomeren).

Andererseits kann die Racematspaltung auch wie folgt durchgeführt werden: Man behandelt die im Ester der Formel IV entsprechende Carbonsäure mit einem optisch aktiven Amin, wie (R)- oder (S)-α-Methylbenzylamin, (R)- oder (S)-α-Naphthyläthylamin, Chinin, Chinidin, Ephedrin oder dergleichen, Vorzugsweise mit (R)- oder (S)-α-Methylbenzylamin. Nach Auftrennung der diastereoisomeren Salze wie oben beschrieben behandelt man das Salz mit einer Säure, stellt aus der erhaltenen Carbonsäure einen entsprechenden Ester her und führt den erhaltenen Ester in eine entsprechende optisch aktive Verbindung der Formel I über, und zwar wie weiter oben für die Herstellung der racemischen Verbindungen beschrieben.

Schliesslich kann die Spaltung auch wie folgt durchgeführt werden: Man behandelt eine einem Ester der Formel III oder IV entsprechende Carbonsäure mit einem optisch aktiven Alkohol und erhält eine Mischung von diastereoisomeren Estern, welche mittels Kristallisation oder Chromatographie aufgetrennt werden kann. Für die Aufspaltung geeignete optisch aktive Alkohole sind z. B. Menthol, Borneol, 2-Octanol, 2,3-Butandiol und dergleichen. In einer bevorzugten Ausführungsform verwendet man die mono-Ester von 2R,3R-Butandiol von Carbonsäuren, welche den Estern der Formeln III oder IV entsprechen. Diese Ester können mittels herkömmlicher Methoden hergestellt werden. Man verwendet vorzugsweise die Methode von Fisher unter Verwendung einer starken Säure als Katalysator. p-Toluolsulfonsäure ist der bevorzugte saure Katalysator. Die diastereoisomeren Ester von Carbonsäuren der Formel IV können hergestellt werden, indem man einen entsprechenden diastereoisomeren Ester der Formel III mit einem Dihaloalkan der Formel VI unter Verwendung von herkömmlichen Techniken, wie sie oben beschrieben sind, alkyliert. Die bevorzugte chromatographische Methode für die Auftrennung ist die Hochdruckflüssigkeitschromatographie (HPLC). Die aufgetrennten diastereoisomeren Ester werden in entsprechende optisch aktive Carbonsäuren der Formel I übergeführt, und zwar unter Verwendung von herkömmlichen Methoden, wie sie oben für die Herstellung von racemischen Verbindungen beschrieben sind.

Die nachfolgenden Beispiele wurden alle unter Inertgas (Argon) durchgeführt, Bezugnamen auf "übliches Aufarbeiten" umfasst 3 Extraktionen mit dem angegebenen Lösungsmittel. Die organischen Auszüge werden dann kombiniert, mit Wasser und Kochsalzlösung gewaschen, über wasserfreiem Magnesiumsulfat getrocknet, Filtriert und im Wasserstrahlvakuum eingedampft. Der Rückstand wird dann im Hochvakuum bei 40-50°C zu konstantem Gewicht getrocknet. Säulenchromatographie wird unter Verwendung von Kieselgel 0,063-0,2 mm durchgeführt.

Die folgenden Beispiele illustrieren die vorliegende Erfindung. Alle Temperaturen sind Celsius Graden angegeben, sofern nichts anderes erwähnt ist.

**Beispiel 1**

Eine Mischung von 30,4 g 2',4'-Dihydroxyacetophenon, 28,8 g Äthyllevulinat, 21,3 g Pyrrolidin und 400 ml Toluol wird unter Rühren und unter Rückfluss während 3 Stunden zum Sieden erhitzt, wobei man das gebildete Reaktionswasser mit einem Dean-Stark-Apparat entfernt. Man kühlt die erhaltene Mischung in einem Eisbad ab und behandelt dann mit 300 ml 1,2N-wässriger Salzsäure. Man rührt während 45 Minuten bei Raumtemperatur, behandelt die Mischung mit Äther und trennt die wässrige Phase ab. Die organische Lösung wird mit 1,2N-Salzsäure gewaschen. Die sauren wässrigen Lösungen werden vereinigt und zweimal mit Äther extrahiert. Die organischen Lösungen werden vereinigt, nacheinander mit Wasser, gesättigter wässriger

Natriumbicarbonatlösung und Kochsalzlösung gewaschen, über Magnesiumsulfat getrocknet, filtriert und unter vermindertem Druck eingedampft. Das verbleibende rote Öl (42 g) wird an 400 g Kieselgel chromatographiert. Man eluiert mit Toluol/Essigester (9:1) und erhält 32,3 g (58,1 %) rac-3.4-Dihydro-7-hydroxy-2-methyl-4-oxo-2H-1-benzopyran-2-propansäure-äthylester als orangefarbenes, viskoses Öl.

**Beispiel 2**

Zu einer in einem Eisbad gekühlten und gerührten Lösung von 1,5 g rac-3,4-Dihydro-7-hydroxy-2-methyl-4-oxo-2H-1-benzopyran-2-propansäure-äthylester in 8 ml trockenem Tetrahydrofuran gibt man 1,4 ml Bortrifluoridätherat, und zwar über einen Zeitraum von 2 Minuten. Die Mischung wird bei 0-5$^{c}$ während 5 Minuten gerührt, worauf man 5,4 ml 1M-Boran in Tetrahydrofuran dazugibt. Man rührt noch während 2,5 Stunden bei 0-5° und zersetzt dann die Mischung durch Zugabe von Eisessig. Man entfernt dann die Lösungsmittel im Vakuum und nimmt den Rückstand in Methylenchlorid auf. Die Lösung wird mit Natriumbicarbonatlösung und Kochsalzlösung gewaschen, getrocknet, filtriert und unter vermindertem Druck eingedampft. Das verbleibende orangefarbene Öl (1,4 g) wird an 50 g Kieselgel chromatographiert. Durch Eluieren mit Hexan/Essigester (4:1) erhält man 0,9 g (63,1 %) rac-3,4-Dihydro-7-hydroxy-2-methyl-2H-1-benzopyran-2-propansäure-äthylester als farbloses Öl.
Analyse für $C_{15}H_{20}O_4$: Berechnet: C, 68,16; H, 7,63: Gefunden: C, 67,86; H, 7,60.

**Beispiel 3**

Zu einer gerührten Aufschlämmung von 120 mg 50-proz. Natriumhydrid-Mineralöldispersion in 0,5 ml trockenem N,N-Dimethylformamid gibt man eine Lösung von 292 mg rac-3,4-Dihydro-7-hydroxy-2-methyl-2H-1-benzopyran-2-propansäure-äthylester in 3 ml trockenem N,N-Dimethylformamid. Die Mischung wird während 25 Minuten bei Raumtemperatur gerührt, worauf man eine Lösung von 364 mg 4'-(3-Brompropoxy)-2'-hydroxy-3'-n-propylacetophenon in 3 ml trockenem Dimethylformamid dazugibt. Man rührt noch während 21 Stunden bei Raumtemperatur, behandelt die Mischung mit Äther und verdünnter Salzsäure und arbeitet mit Äther wie üblich auf. Man erhält 523 mg eines orange farbenen Öls. Dieses Material wird an 50 g Kieselgel chromatographiert. Durch Eluieren mit Toluol/Essigester (19:1) erhält man 257 mg (46,5 %) rac-7-[3-(4-Acetyl-3-hydroxy-2-propylphenoxy) propoxy]-3,4-dihydro-2-methyl-2H-1-benzopyran-2-propansäure-äthylester als blassgelbes Öl.
Analyse für $C_{29}H_{38}O_7$: Berechnet: C, 69,86; H, 7,68. Gefunden: C, 69,65; H, 7,73.

**Beispiel 4**

Zu einer Mischung von 1,55 g des Esters aus Beispiel 3 und 35 ml Tetrahydrofuran/Wasser (1:1) gibt man 2,3 g Lithiumhydroxid-Monohydrat. Die Mischung wird dann noch während 22 Stunden bei Raumtemperatur gerührt und dann mit Wasser verdünnt, worauf man dreimal mit Äther extrahiert (die ätherischen Auszüge werden verworfen). Die wässrige Lösung wird durch Zugabe von gesättigter wässriger Oxalsäurelösung auf pH 1 angesäuert und mit Äther in üblicher Weise aufgearbeitet. Man erhält 1,4 g (95,8 %) rac-7-[3-(4-Acetyl-3-hydroxy-2-propylphenoxy)propoxy]-3,4-dihydro-2-methyl-2H-1-benzopyran-2-propansäure als viskoses gelbes Öl.
Analyse für $C_{27}H_{34}O_7$: Berechnet: C, 68,92; H, 7,28. Gefunden: C, 68,62; H, 7,23.

**Beispiel 5**

Eine Mischung von 3,88 g 2',4'-Dihydroxy-3'-n-propyl-acetophenon, 3,14 g Äthyllevulinat, 0,83 ml Pyrrolidin und 20 ml Toluol wird während einer Stunde bei Raumtemperatur gerührt und dann während 3 Stunden unter Rückfluss zum Sieden erhitzt, wobei das gebildete Reaktionswasser mit einem Dean-Stark-Apparat laufend entfernt wird. Man kühlt die erhaltene dunkelrotbraune Mischung ab, behandelt diese mit 25 ml 1N-Salzsäure und rührt während 30 Minuten bei Raumtemperatur. Man versetzt mit Wasser und arbeitet die Mischung in üblicher Weise mit Äther auf, worauf man 5,1 g eines dunkelroten Öls erhält. Dieses Material wird an 100 g Kieselgel chromatographiert. Durch Eluieren mit Toluol/Essigester (9:1 und 4:1) erhält man 2.9 g (45,3 %) rac-3,4-Dihydro-7-hydroxy-2-methyl-4-oxo-3-n-propyyl-2H-1-benzopyran-2-propansäure-äthylester als gelbes Öl.
Analyse für $C_{18}H_{24}O_5$ Berechnet: C, 67,48; H, 7,55. Gefunden: C, 67,31; H, 7,54.

**Beispiel 6**

In Analogie zu Beispiel 2 werden 257 mg rac-3,4-Dihydro-7-hydroxy-2-methyl-4-oxo-8-n-propyl-2H-1-benzopyran-2-propansäure-äthylester mit Boran in Tetrahydrofuran und Bortrifluoridätherat reduziert. Man erhält 195 mg rac-3,4-Dihydro-7-hydroxy-2-methyl-8-n-propyl-2H-1-benzopyran-propansäure-äthylester als blassgelbes Öl, dass man mittels Chromatographie an Kieselgel reinigt.

Analyse für $C_{18}C_{26}O_4$: Berechnet: C. 70,56; H, 8,55. Gefunden: C, 70,01; H, 8,28.

**Beispiel 7**

Zu einer gerührten Aufschlämmung von 108 mg 50-proz. Natriumhydrid-Mineralöldispersion (mit Hexan gewaschen) in 1 ml wasserfreiem N,N-Dimethylformamid gibt man eine Lösung von 268 mg rac-3,4-Dihydro-7-hydroxy-2-methyl-8-n-propyl-2H-1-benzopyran-2-propansäure-äthylester in 3 ml trockenem N,N-Dimethylformamid, und zwar über einen Zeitraum von einer Minute und bei Raumtemperatur. Man rührt während 30 Minuten bei Raumtemperatur, behandelt die Mischung tropfenweise mit einer Lösung von 313 mg 4'-(3-Brompropoxy)-2'-hydroxy-3'-n-propylacetophenon in 3 ml trockenem N,N-Dimethylformamid, versetzt mit Natriumjodid (0,3 g) und rührt die Mischung während 2,5 Stunden bei Raumtemperatur, bevor man die Reaktion durch Zugabe von 1 ml Wasser abbricht. Nach 30 Minuten versetzt man mit 0,3 g Lithiumhydroxid-Monohydrat und 1 ml Wasser und rührt noch während 3 Stunden. Die Mischung wird mit 1N-Salzsäure auf pH 1 angesäuert und mit Äther in üblicher Weise aufgearbeitet. Das erhaltene orangefarbene ölige Produkt (0,7 g) wird an 50 g Kieselgel chromatographiert. Man eluiert mit Toluol/Essigester (4:1) und erhält 310 mg (69,1 %) rac-7-[3-(4-Acetyl-3-hydroxy-2-n-propylphenoxy)propoxy]-3,4-dihydro-2-methyl-8-n-propyl-2H-1-benzopyran-2-propansäure als viskoses gelbes Öl, das man bei 60° im Hochvakuum trocknet.

Analyse für $C_{30}H_{40}O_7$: Berechnet: C, 70,29; H, 7,87. Gefunden: C, 70,19; H, 7,99.

**Beispiel 8**

In Analogie zu Beispiel 1 werden 15 g 2',5'-Dihydroxy-acetophenon mit 14,2 g Äthyllevulinat und 12,3 ml Pyrrolidin in 200 ml Toluol kondensiert. Das Rohprodukt (20,5 g) wird mittels Hochdruckflüssigkeitschromatographie gereinigt (Kieselgel; Hexan/Essigester 2:1). Man erhält 17,6 g (64,1%) rac-Dihydro-6-hydroxy-2-methyl-4-oxo-2H-1-benzopyran-2-propansäure-äthylester als gelbes Öl.

Analyse für $C_{15}H_{18}O_5$: Berechnet: C, 64,74; H, 6,52. Gefunden: C, 64,28; H, 6,64.

**Beispiel 9**

Zu einer Lösung von 8,25 g rac-3,4-Dihydro-6-hydroxy-2-methyl-4-oxo-2H-1-benzopyran-2-propansäure-äthylester in 50 ml trockenem Glyme gibt man 7,29 ml Bortrifluoridätherat, und zwar tropfenweise unter Rühren in einem Eisbad. Man rührt während 10 Minuten bei 0-5° und behandelt die Mischung mit 1,75 g Boran-Dimethylamin-Komplex. Man rührt noch während einer Stunde bei Raumtemperatur, worauf man 5 ml Eisessig zugibt, die Lösung auf kaltes Wasser giesst und mit Äther in üblicher Weise aufarbeitet (die vereinigten organischen Auszüge werden zusätzlich noch mit Natriumbicarbonatlösung gewaschen). Das Rohprodukt das noch grössere Mengen an Ausgangs-Keton enthält wird erneut der obigen Reduktion und Aufarbeitung unterworfen. Man erhält 8,1 g eines gelben Öls das man mittels präparativer Hochdruckflussigkeitschromatographie an Kieselgel unter Eluieren mit Hexan/Essigester (2:1) reinigt. Man erhält 5,8 g (74,1 %) rac-3,4-Dihydro-6-hydroxy-2-methyl-2H-1-benzopyran-2-propansäure-äthylester als gelbes Öl.

**Beispiel 10**

In Analogie zu Beispiel 7 werden 0,695 g rac-3,4-Dihydro-6-hydroxy-2-methyl-2H-1-benzopyran-2-propansäureäthylester mit 0,939 g 4'-(3-Brompropoxy)-2'-hydroxy-3'-n- propylacetophenon unter Verwendung von Natriumhydrid und Natriumjodid in trockenem N,N-Dimethylformamid alkyliert. Nach Verseifung mit 1,25 g Lithiumhydroxid-Monohydrat erhält man 1,4 g der rohen Säure. Mittels Säulenchromatographie an 50 g Kieselgel erhält man 0,837 g (67 %) rac-6-[3- (4-Acetyl-3-hydroxy-2-propylphenoxy)propoxy]-3,4-dihydro-2-methyl-2H-1-benzopyran-2-propansäure als farbloses Öl: als Elutionsmittel verwendet man Toluol/Essigester (9:1 und 4:1).

Analyse für $C_{27}H_{34}O_7$: Berechnet: C, 68,92; H, 7,28. Gefunden: C, 68,36; H, 7,20.

**Beispiel 11**

In Analogie zu Beispiel 7 werden 0,9 g des Äthylesters der rac-3,4-Dihydro-6-hydroxy-2-methyl-2H-1-benzopyran-2-carbonsäure mit 4'-(3-Brompropoxy)-2'-hydroxy-3'-n-propyl-acetophenon (1,5 g) unter Verwendung von 12,16 mMol Natriumhydrid und 10 mMol Natriumjodid in N,N-Dimethylformamid alkyliert. Nach Verseifen mit 1,5 g Lithiumhydroxid-Monohydrat wird die erhaltene rohe Säure mittels Chromatographie an Kieselgel gereinigt. Man erhält 1,16 g (68,9 %) rac-6-[3-(4-Acetyl-3-hydroxy-2-propylphenoxy)propoxy]-3,4-dihhydro-2-methyl-2H-1-benzopyran-2-carbonsäure als weisslichen Festkörper vom Schmelzpunkt 108-110,5°

Analyse für $C_{25}H_{30}O_7$: Berechnet: C, 67,86; H, 6,83. Gefunden: C, 67,92; H. 6,97.

**Beispiel 12**

Eine Portion von 365 mg 50-proz. Natriumhydrid-Mineralöldispersion wird mit Hexan gewaschen und in 32 ml wasserfreiem N,N-Dimethylformamid suspendiert. Zur gerührten Aufschlämmung gibt man 1 g rac-3,4-Dihydro-2,5,7,8-tetra-methyl-hydroxy-2H-1-benzopyran-2-carbonsäure-methylester. Man rührt während 45 Minuten bei Raumtemperatur und behandelt die Mischung tropfenweise mit 3,2 ml (4,47 g) Allylbromid. Man rührt noch während 90 Stunden bei Raumtemperatur, worauf man 3 ml Methanol hinzugibt. Man giesst die Mischung auf Wasser und arbeitet mit Äther in üblicher Weise auf. Man erhält 1,8 g eines bernsteinfarbenen Öls. Dieses Material wird mittels Säulenchromatographie an Kieselgel und präparativer Hochdruckflüssigkeitschromatographie an Kieselgel unter Eluieren mit Hexan/Essigester (19:1) gereinigt. Man erhält 772 ml (67,2 %) rac-3,4-Dihydro-2,5,7,8-tetramethyl-6-(2-propenyloxy)-2H-1-benzopyran-2-carbonsäure-methylester als blassgelbes Öl. Dieses Material wird bei einer Badtemperatur von 105-107° und einem Druck von 0,02 mm destilliert. Man erhält ein Öl das beim Stehen kristallisiert und dann einen Schmelzpunkt von 68-70° hat.

Analyse für $C_{18}H_{24}O_4$ Berechnet: C, 71.03; H, 7,95. Gefunden: C, 71,37; H, 7,90.

**Beispiel 13**

Zu einer in einem Eisbad gekühlten Lösung von 1,8 g rac-3,4-Dihydro-2,5,7,8-tetramethyl-6-(2-propenyloxy)-2H-1-benzopyran-2-carbonsäure-methylester in 36 ml wasserfreiem Tetrahydrofuran gibt man unter Rühren 1,98 ml Boran-Dimethylsulfid-Komplex. Die Reaktionsmischung wird während 1,5 Stunden bei 0-5° gerührt und dann durch tropfenweise Zugabe von 9 ml Wasser zersetzt. Man rührt dann noch während 10 Minuten bei 0-5° und behandelt dann die Mischung nacheinander tropfenweise mit 6,84 ml 3N-Natronlauge und 2,2 ml 30-proz. Wasserstoffperoxid. Man rührt die Mischung während einer Stunde bei 5-10° und säuert mit 7 ml 3N-Salzsäure an. Nach üblichem Aufarbeiten mit Äther erhält man 2 g eines Öls, das man mittels präparativer HPLC an Kieselgel unter Eluieren mit Hexan/Essigester (1:1) reinigt. Man erhält 1,22 g (64 %) rac-3,4-Dihydro-2,5,7,8-tetrametyl-6-(3-hydroxypropoxy)-2H-1-benzopyran-2-carbonsäure-methylester als farbloses Öl.

Analyse für $C_{18}H_{26}O_5$: Berechnet: C, 67,06; H, 8,13. Gefunden: C, 67,39; H, 8,19.

**Beispiel 14**

Zu einer Lösung, von 0,3 g rac-3,4-Dihydro-2,5,7,8-tetramethyl-6-(3-hydroxypropoxy)-2H-1-benzopyran-2-carbonsäuremethylester in 5 ml wasserfreiem Methylenchlorid und 0,33 ml Triäthylamin gibt man 0,17 ml Methansulfonylchlorid. Die Mischung wird während 45 Minuten bei Raumtemperatur gerührt, dann mit 1N-Schwefelsäure behandelt und mit Methylenchlorid in üblicher Weise aufgearbeitet. Man erhält 0,461 g rac-3,4-Dihydro-2,5,7,8-tetramethyl-6-(2-methan-solfonyloxypropoxy)-2H-1-benzopyran-2-carbonsäure-methylester als Öl, das man ohne weitere Reinigung in die nächste Stufe, einsetzt.

**Beispiel 15**

Eine Mischung aus dem rohen Methansulfonat aus Beispiel 14 (ungefähr 0,932 mMol), 595 mg Natriumjodid und 5 ml Aceton wird während 43,5 Stunden bei Raumtemperatur gerührt und dann mit Wasser behandelt und mit Äther in üblicher Weise aufgearbeitet (die ätherischen Auszüge werden zusätzlich noch mit verdünnter Natriumbisulfitlösung gewaschen). Das ölige Produkt (390 mg) wird an 20 g Kieselgel chromatographiert. Durch Eluieren mit Hexan/Essigester (19:1 und 9:1) erhält man 306 mg (76,0 %) rac-3,4-Dihydro-2,5,7,8-tetramethyl-6-(3-jodpropoxy)-2H-1-benzopyran-2-carbonsäure-methylester als farbloses Öl. Das in einem anderen Versuch auf diese Weise hergestellte Jodid kristallisierte zu einem Festkörper vom Schmelzpunkt 73-75,5°.

**Beispiel 16**

Eine Mischung aus 0,137 g 2',4'-Dihydroxy-3'-n-propyl-acetophenon, 0,195 g wasserfreiem Kaliumcarbonat und 3 ml wasserfreiem N,N-Dimethylformamid wird während einer Stunde bei Raumtemperatur gerührt. Eine Lösung von 0,306 g des Jodids aus Beispiel 15 in 5 ml N,N-Dimethylformamid wird hinzugefügt und die Mischung wird während 30 Minuten bei Raumtemperatur und während 31 Stunden bei 60° gerührt. Man kühlt ab, behandelt die Reaktionsmischung mit 1N-Salzsäure und arbeitet mit Äther in üblicher Weise auf. Das ölige Produkt (0,406 g) wird an 20 g Kieselgel chromatographiert. Durch Eluieren mit Hexan/Essigester (4:1) erhält man 0,291 g (82,5 %) rac-6-[3-(4-Acetyl-3-hydroxy-2-propylphenoxy)propoxy]-3,4-dihydro-2,5,7,8-tetramethyl-2H-1-benzopyran-2-carbonsäure-methylester als blassgelbes Öl. Das aus einem anderen Versuch in dieser Weise hergestellte Material kristallisierte und lieferte einen farblosen Festkörper vom Schmelzpunkt 121-123° (aus Äthanol).

Analyse für $C_{29}H_{38}O_7$: Berechnet: C, 69,86; H, 7,68. Gefunden: C, 69,31; H, 7,67.

**Beispiel 17**

Eine Lösung von 0,246 g des Methylesters aus Beispiel 16 und 0,227 g Natriumhydroxid in 8 ml Methanol und 2 ml Wasser wird während 1,5 Stunden bei Raumtemperatur gerührt, dann mit 1N-Salzsäure behandelt und in üblicher Weise mit Äther aufgearbeitet. Das Rohprodukt wird erneut in Äther aufgelöst, worauf man die Lösung mit verdünnter Natriumbicarbonatlösung wäscht. Die wässrige alkalische Phase wird mit 2N-Salzsäure sauer gestellt und mit Äther in üblicher Weise aufgearbeitet, worauf man 0,161 g (67,3 %) einer Säure als gelbes Öl erhält, das beim Stehenlassen kristallisiert. Durch Umkristallisieren aus Benzol/Hexan erhält man rac-6-[3-(4-Acetyl-3-hydroxy-2-propylphenoxy)propoxy]-3,4-dihydro-2,5,7,8-tetramethyl-2H-1-benzopyraran-2-carbonsäure als farblosen; Festkörper vom Schmelzpunkt 137-139°.

Analyse für $C_{28}H_{36}O_7$: Berechnet: C, 69,40; H, 7,49. Gefunden: C, 69,28, H, 7,58.

**Beispiel 18**

In Analogie zu Beispiel 12 werden 1,42 g rac-3,4-Dihydro-2,5,7,8-tetramethyl-6-hydroxy-2H-1-benzopyran-2-essigsäure-methylester mit 2,79 g Allylbromid und Natriumhydrid (5,58 mMol) in wasserfreiem N,N-Dimethylformamid alkyliert. Man erhält 1,35 g (83,1 %) rac,-3,4-Dihydro-2,5,7,8-tetra-methyl-6-(2-propenyloxy)-2H-1-benzopyran-2-essigsäure-methylester als gelbes Öl, das man mittels Säulenchromatographie an Kieselgel unter Eluieren mit Hexan/Essigester (9:1) reinigt.

Analyse für $C_{19}H_{26}O_4$: Berechnet: C, 71,67; H, 8,23. Gefunden: C, 71,54; H, 8,25.

**Beispiel 19**

Zu einer in einem Eisbad gekühlten Lösung von 1M-Boran in Tetrahydrofuran (10 ml) gibt man unter Rühren tropfenweise eine Lösung von 2 ml (1,64 g) Cyclohexen in 6 ml trockenem Tetrahydrofuran. Man rührt die Mischung während 1,5 Stunden bei 0-5°. Zur erhaltenen Aufschlämmung von Dicyclohexylboran gibt man tropfenweise eine Lösung von 1,59 g des Allyläthers aus Beispiel 18 in 4 ml Tetrahydrofuran. Man rührt die Mischung während einer Stunde bei 0-5° und während 18 Stunden bei Raumtemperatur, worauf man mit 20 ml 1M-methanolischem Natriumacetat (tropfenweise) und dann mit 23,8 ml 0,4M-methanolischem Jod versetzt. Die erhaltene Mischung wird während 4 Stunden bei Raumtemperatur gerührt und dann mit 3,1 ml wässrigem Natriumthiosulfat versetzt. Die Reaktionsmischung wird auf Wasser gegossen und in üblicher Weise mit Äther aufgearbeitet. Durch Chromatographieren des Rohproduktes (4,4 g) an 100 g Kieselgel erhält man 1,71 g (76,7 %) rac-3,4-Dihydro-6-(3-jodpropoxy)-2,5,7,8-tetramethyl-2H-1-benzopyran-2-essigsäure-methylester als Öl (als Elutionsmittel verwendet man Hexan-Essigester (19:1 und 9:1).

**Beispiel 20**

In Analogie zu Beispiel 16 werden 328 mg 2',4'-Dihydroxy-3'-n-propylacetophenon mit 756 mg des Jodides aus Beispiel 19 und wasserfreiem Kaliumcarbonat (467 g) in wasserfreiem N,N-Dimethylformamid alkyliert.

Man erhält 0,789 g (91,1 %) rac-6-[3-(4-Acetyl-3-hydroxy-2-propylphenoxy)propoxy]-3,4-dihydro-2,5,7,8-tetramethyl-2H-1-benzopyran-2-essigsäure-methylester als gelbes Öl, das man durch Säulenchromatographie an Kieselgel unter Eluieren mit Hexan/Äther (4:1) reinigt.

**Beispiel 21**

0,669 g des Methylesters aus Beispiel 20 werden unter Verwendung des in Beispiel 17 beshriebenen Verfahrens mit Natriumhydroxid verseift. Die rohe Säure wird an 30 g Kieselgel chromatographiert. Unter Eluieren mit Hexan/Essigester (2:1) erhält man 0,386 g (59,6 %) rac-6-[3-(4-Acetyl-3-hydroxy-2-propylphenoxy-propoxy]-3,4-dihydro-2,5,7,8-tetramethyl-2H-1-benzopyran-2-essigsäure als gelbe glasartige Substanz.

Analyse für $C_{29}H_{38}O_7$: Berechnet: C, 69,86; H, 7,68. Gefunden: C, 69,63; H: 7,89.

**Beispiel 22**

Zu einer Lösung von 0,8 g rac-3,4-Dihydro-7-hydroxy-2-methyl-8-n-propyl-2H-1-benzopyran-2-propansäure-äthylester in 8 ml Pyridin gibt man unter Rühren 0,37 ml Essigsäureanhydrid. Die Lösung wird während 5 Stunden bei Raumtemperatur gerührt, dann auf Eiswasser gegossen und mit Äther wie üblich aufgearbeitet (die vereinigten ätherischen Auszüge werden noch zusätzlich mit 1N-Salzsäure und gesättigter Natriumbicarbonatlösung gewaschen). Das ölige Produkt (1,0 g) wird an 50 g Kieselgel chromatographiert. Durch Eluieren mit Toluol/Essigester (19:1) erhält man 0,575 g (63,3 %) rac-Acetoxy-3,4-dihydro-2-methyl-8-n-propyl-2H-1-benzopyran-2-propansäure-äthylester als blassgelbes Öl.

Analyse für $C_{20}H_{28}O_5$: Berechnet: C, 68,94; H, 8,10 Gefunden: C, 68,73; H, 8,06.

**Beispiel 23**

Zu einer bei Raumtemperatur gerührten Lösung von 276 mg rac-7-Acetoxy-3,4-dihydro-2-methyl-8-n-propyl-2H-1-benzopyran-2-propansäure-äthylester in 2 ml Eisessig gibt man 0,21 ml Bortrifluoridätherat. Die Mischung wird während 24 Stunden bei 105° gerührt und dann abgekühlt und mit Äther verdünnt. Die Ätherlösung wird mit Wasser und Kochsalzlösung gewaschen und wie üblich weiterverarbeitet, wobei man 298 mg eines gelbbraunen Öls erhält. Dieses Material wird an 30 g Kieselgel chromatographiert. Durch Eluieren mit Toluol/Essigester (4:1) erhält man 183 mg (72,1 %) rac-6-Acetyl-3,4-dihydro-7-hydroxy-2-methyl-8-n-propyl-2H-1-benzopyran-2-propansäure als weisslichen Festkörper vom Schmelzpunkt 154-156°.

Analyse für $C_{18}H_{24}O_5$: Berechnet: C, 67,48; H, 7,55. Gefunden: C, 67,17; H, 7,72.

**Beispiel 24**

Eine Lösung von 692 mg rac-6-Acetyl-3,4-Dihydro-7-hydroxy-2-methyl-8-n-propyl-2H-1-benzopyran-2-propansäure und 100 mg p-Toluolsulfonsäure-Monohydrat in 10 ml absolutem Äthanol wird während 17 Stunden unter Rühren und unter Rückfluss zum Sieden erhitzt. Die erhaltene Lösung wird abgekühlt, mit wenig gesättigter Natriumbicarbonatlösung behandelt und dann im Vakuum eingedampft, um das Äthanol zu entfernen. Der Rückstand wird mit Äther behandelt, und die ätherische Lösung wird wie üblich weiterverarbeitet, Wobei man 752 mg eines gelben Öls erhält. Dieses Material wird an 50 g Kieselgel chromatographiert. Durch Eluieren mit Toluol/Essigester (4:1) erhält man 706 mg (93,9 %) rac-6-Acetyl-3,4-dihydro-7-hydroxy-2-methyl-8-n-propyl-2H-1-benzopyran-2-propansäure-äthylester als gelbes Öl.

Analyse für $C_{20}H_{28}O_5$: Berechnet: C, 68,94; H, 8,10. Gefunden: C, 68,86; H, 7,85.

**Beispiel 25**

Eine Mischung aus 263 mg rac-6-Acetyl-3,4-dihydro-7-hydroxy-2-methyl-8-n-propyl-2H-1-benzopyran-2-propansäureäthylester, 222 mg wasserfreiem Kaliumcarbonat, 0,39 ml 1,3-Dibrompropan, 6 ml wasserfreiem Aceton und 3 ml wasserfreiem N,N-Dimethylformamid wird unter Rühren und unter Rückfluss während, 16 Stunden zum Sieden erhitzt. Eine zusätzliche Portion von 0,25 g wasserfreiem Kaliumcarbonat und unter Rückfluss zum Sieden erhitzt. Die Mischung wird abgekühlt, und die flüchtigen Anteile werden zuerst im Wasserstrahlvakuum und am Hochvakuum. entfernt. Den Rückstand behandelt man mit Äther und Aceton und filtriert die Festkörper ab. Das Filtrat wird im Vakuum konzentriert, und man erhält 359 mg rac-6-Acetyl-7-(3-

brompropoxy)-3,4-dihydro-2-methyl-8-n-propyl-2H-1-benzopyran-2-propansäure-äthylester als orangefarbenes Öl.

**Beispiel 26**

Eine Mischung aus 335 mg rac-6-Acetyl-7-(3-Brompropoxy)-3,4-dihydro-2-methyl-8-n-propyl-2H-1-benzopyran-2-propansäure-äthylester, 143 mg 2',4'-Dihydroxy-3'-n-propyl-acetophenon, 217 mg wasserfreiem Kaliumcarbonat, 6 ml wasserfreiem Aceton und 3 ml wasserfreiem Dimethylformamid wird unter Rühren und unter Rückfluss während 17 Stunden zum Sieden erhitzt. Eine zusätzliche Portion von 100 mg Kaliumcarbonat wird dann hinzugefügt, worauf man noch während 4 Stunden unter Rühren und unter Rückfluss zum Sieden erhitzt. Nach Abkühlen auf Raumtemperatur wird die Mischung durch Zugabe von 1N-Salzsäure vorsichtig auf pH 1 gestellt. Man versetzt dann mit Wasser und arbeitet mit Äther wie üblich auf. Den Rückstand chromatographiert man an 50 g Kieselgel. Durch Eluieren mit Toluol/Essigester (9:1) erhält man 219 mg (52,6 %) rac-6-Acetyl-7-[3-(4-acetyl-3-hydroxy-2-n-propylphenoxy)propoxy]-3,4-dihydro-2-methyl-8-n-propyl-2H-1-benzopyran-1-propansäure-äthylester als gelbes Öl.

**Beispiel 27**

In Analogie zu Beispiel 4 werden 191 mg des Produktes aus Beispiel 26 mit 273 mg Lithiumhydroxid-Monohydrat in 5 ml Tetrahydrofuran/Wasser (3:2) verseift. Nach Säulenchromatographie an Kieselgel erhält man rac-6-Acetyl-7[-3-(-4-acetyl-3-hydroxy-2-n-propylphenoxy)propoxy]-3,4-dihydro-2-methyl-8-n-propyl-2H-1-benzopyran-2-propansäure als gelbes, viskoses Öl.

Analyse für $C_{32}H_{42}O_8$: Berechnet: C, 69,29; H, 7,63. Gefunden: C, 69,07; H, 7,79.

**Beispiel 28**

In Analogie zu Beispiel 7 werden 809 mg des Äthylesters der rac-3,4-Dihydro-6-hydroxy-5,7,8-trimethyl-2H-1-benzopyran-2-carbonsäure mit 1,2 g 4'-(3-Brompropoxy)-2'-hydroxy-3'-n-propylacetophenon, 448 mg 50-proz. Natriumhydrid-Mineralöldispersion und 1,2 g Natriumjodid in wasserfreiem Dimethylformamid alkyliert. Nach Verseifen mit 1,2 g Lithiumhydroxid-Monohydrat erhält man 1,6 g der rohen kristallinen Säure. Dieses Material wird an 50 g Kieselgel chromatographiert. Durch Eluieren mit Toluol/Essigester (4:1, 2:1 und 1:1) erhält man 1,2 g (83,2 %) rac-6-[3-(4-Acetyl-3-hydroxy-2-propylphenoxy)propoxy]-3,4-dihydro-5,7,8-trimethyl-2H-1-benzopyran-2-carbonsäure als weisslichen Festkörper vom Schmelzpunkt 144-147°.

Analyse für $C_{27}H_{34}O_7$: Berechnet: C, 68,92; H. 7,28. Gefunden: C, 68,68; H. 7,02.

**Beispiel 29**

Eine Mischung aus 25 g p-Benzyloxyphenol, 20 ml frisch destilliertem Acrolein, 70 ml Methanol und 0,5 ml konzentrierter Schwefelsäure wird in einem Druckgefäss während 2 Stunden bei 150° gerührt. Die Mischung wird dann abgekühlt und mit Äther verdünnt. Die ätherische Lösung wird nacheinander mit Wasser und gesättigter Natriumbicarbonatlösung gewaschen und dann in üblicher Weise aufgearbeitet. Der braune ölige Rückstand (47,9 g) wird an 250 g Kieselgel chromatographiert. Durch Eluieren mit Toluol erhält man 23,1 g eines orangefarbenen Öls, das man erneut an 300 g Kieselgel chromatographiert, durch Eluieren mit Hexan/Essigester (9:1) erhält man 12,8 g (37,9 %) rac-3,4-Dihydro-2-methoxy-6-(phenylmethoxy)-2H-1-benzopyran als gelbes Öl. Dieses Material wird in 200 ml Aceton gelöst, worauf man mit 120 ml 2N-Salzsäure versetzt. Die Mischung wird unter Rühren und unter Rückfluss während 2 Stunden zum Sieden erhitzt, über Nacht bei Raumtemperatur gehalten und während weiteren 2 Stunden unter Rückfluss zum Sieden erhitzt. Nach dem Abkühlen wird die Mischung im Wasserstrahlvakuum konzentriert, um das meiste Aceton zu entfernen. Der wässrige Rückstand wird mit Äther in üblicher Weise aufgearbeitet (die vereinigten ätherischen Auszüge werden zusätzlich mit gesättigter Natriumbicarbonatlösung gewaschen). Man erhält 12,1 g eines blassgelben Festkörpers. Dieses Material wird an 250 g Kieselgel chromatographiert. Durch Eluieren mit Toluol/Essigester (9:1 und 4:1) erhält man 9,8 g (80,7 %) rac-3,4-Dihydro-6-(phenylmethoxy)-2H-1-benzopyran-2-ol als blassgelben Festkörper vom Schmelzpunkt 92,5-95°.

Analyse für $C_{16}H_{16}O_3$: Berechnet: C, 74,98; H, 6,29. Gefunden: 74,78; H, 6,41.

**Beispiel 30**

Eine Lösung von 1,8 g rac-3,4-Dihydro-6-(phenylmethoxy)-2H-1-benzopyran-2-ol und 2,6 g Carbäthoxymethylen)triphenylphosphoran in 20 ml Toluol wird unter Rühren und unter Rückfluss während 22 Stunden zum Sieden erhitzt. Die Lösung wird abgekühlt und auf eine Säule aufgetragen, welche mit 50 g Kieselgel und Toluol bepackt worden ist. Durch Eluieren mit Toluol und Toluol/Essigester (19:1) erhält man 1,7 g (74,2 %) rac-3,4-Dihydro-6-(phenylmethoxy)-2H-1-benzopyran-2-essigsäure-äthylester als blassgelbes Öl. Dieses Material wird in 25 ml absolutem Äthanol aufgenommen, und die Lösung wird in der Gegenwart von 0,2 g 10-proz. Palladium/Kohle bei Raumtemperatur und unter einer Wasserstoffatmosphäre gerührt. Nach 1,5 Stunden ist die Wasserstoffaufnahme beendet, und der Katalysator wird mittels Filtration entfernt. Das Filtrat wird im Vakuum eingedampft. Man erhält 1,2 g (97,6 %) rac-3,4-Dihydro-6-hydroxy-2H-1-benzopyran-2-essigsäure-äthylester als blassgelbes Öl.

**Beispiel 31**

Eine Mischung aus 1,2 g rac-3,4-Dihydro-6-hydroxy-2H-1-benzopyran-1-essigsäure-äthylester, 1,8 g 4'-(3-Brompropoxy)-2'-hydroxy-3'-n-propylacetophenon, 1,6 g wasserfreiem Kaliumcarbonat, 30 ml trockenem Aceton und 15 ml trockenem Dimethylformamid wird unter Rühren und unter Rückfluss während 23 Stunden zum Sieden erhitzt. Man versetzt noch mit einer zusätzlichen Portion von 1,6 g Kaliumcarbonat und rührt noch während 2,5 Stunden unter Rückfluss zum Sieden. Nach dem Abkühlen wird die Mischung vorsichtig auf 1N-Salzsäure gegossen. Durch übliches Aufarbeiten mit Äther (die vereinigten ätherischen Auszüge werden zusätzlich noch mit gesättigter wässriger Natriumbicarbonatlösung gewaschen) erhält man 3,7 g eines braunen Öls, das man an 50 g Kieselgel chromatographiert. Durch Eluieren mit Toluol/Essigester (19:1) erhält man 1,6 g eines blassgelben Öls. Eine Lösung dieses Esters und 3,0 g Lithiumhydroxid-Monohydrat in 40 ml Tetrahydrofuran/Wasser (1:1) wird während 23 Stunden bei Raumtemperatur gerührt. Die erhaltene Lösung wird mit Wasser verdünnt und mit 2N-Salzsäure auf pH 1 gestellt. Man arbeitet dann mit Äther in üblicher Weise auf und erhält ein Öl, das man an 50 g Kieselgel chromatographiert. Durch Eluieren mit Toluol/Essigester (4:1, 2:1 und 1:1) erhält man 831 mg (37 %) rac-6-[3-(4-Acetyl-3-hydroxy-2-propylphenoxy)propoxy]-3,4-dihydro-2H-1-benzopyran-2-essigsäure als blassgelben Festkörper vom Schmelzpunkt 102,5-105°.

Analyse für $C_{25}H_{30}O_7$: Berechnet: C, 67,86; H, 6,83. Gefunden: C, 68,03; H, 6,75.

**Beispiel 32**

Eine Mischung aus 17,5 g (Carbäthoxymethylen)triphenylphosphoran und 11,4 g 4-Benzyloxy-2'-hydroxybenzaldehyd in 200 ml Toluol wird unter Rühren und unter Rückfluss während einer Stunde zum Sieden erhitzt. Nach Eindampfen des Lösungsmittels im Vakuum erhält man 28,2 g eines Rückstandes, der (4'-Benzyloxy-2'-hydroxy)benzolpropensäure-äthylester und Triphenylphosphinoxid enthält. Dieses Material wird in 300 ml Essigester gelöst und dann bei Atmosphärendruck und Raumtemperatur in Gegenwart von 0,35 g Platinoxid hydriert. Man entfernt den Katalysator durch Filtration, dampft das Filtrat im Vakuum ein und erhält 28,1 g eines Produktes, das (4'-Benzyloxy-2'-hydroxy)benzolpropansäure-äthylester enthält. Dieses Material wird mit 100 ml 5-proz. methanolischer Kaliumhydroxidlösung versetzt, und die Mischung wird dann während 2 Stunden unter Rückfluss zum Sieden erhitzt. Das meiste Methanol wird dann durch Eindampfen im Vakuum entfernt, und der Rückstand wird zwischen Äther und Wasser verteilt. Die ätherische Phase wird verworfen. Die wässrige Phase wird mit 2N-Salzsäure sauer gestellt und mit Äther/Tetrahydrofuran (1:1) in üblicher Weise aufgearbeitet. Man erhält 9,8 g (4'-Benzyloxy-2-hydroxy)benzolpropansäure. Dieses Material löst man in 98 ml Essigsäureanhydrid und erhitzt die erhaltene Lösung während 2 Stunden unter Rückfluss zum Sieden. Nach dem Abkühlen behandelt man den Rückstand mit 100 ml Methanol und entfernt die Lösungsmittel durch Eindampfen im Vakuum. Die Behandlung mit Methanol und das Eindampfen werden noch zweimal wiederholt, worauf der Rückstand mittels Hochdruckflüssigkeitschromatographie an Kieselgel unter Eluieren mit Hexan/Essigester (4:1) gereinigt wird. Man erhält 6,2 g (48,8 %) 3,4-Dihydro-7-(phenylmethoxy)-1-benzopyran-2-on als Festkörper.

**Beispiel 33**

Zu einer bei -76° gerührten Lösung von 6,2 g 3,4-Dihydro-7-(phenylmethoxy)-1-benzopyran-2-on in 80 ml Methylenchlorid gibt man tropfenweise 20 ml Diisobutylaluminiumhydrid (25-proz. Lösung in Toluol). Die Reaktionsmischung wird während einer Stunde bei dieser Temperatur gerührt, worauf man vorsichtig 5 ml Methanol dazugibt. Die Lösung wird auf 25 ml 1N-Schwefelsäure gegossen, und die Mischung wird mit Äther in üblicher Weise aufgearbeitet (die vereinigten organischen Auszüge werden zusätzlich noch mit gesättigter

Natriumbicarbonatlösung gewaschen). Der Rückstand (5,9 g) wird mittels Hochdruckflüssigkeitschromatographie an Kieselgel unter Eluieren mit Hexan/Essigester (3:1) gereinigt. Man erhält 4,8 g (76,8 %) rac-3,4-Dihydro-2-(phenylmethoxy)-2H-1-benzopyran-7-ol als farblosen Festkörper vom Schmelzpunkt 64-65,5°.
Analyse für $C_{16}H_{16}O_3$ Berechnet: C, 74,98; H, 6,29. Gefunden: C, 74,44; H, 6,38.

**Beispiel 34**

In Analogie zu Beispiel 30 werden 2,2 g rac-3,4-Dihydro-7-(phenylmethoxy)-2H-1-benzopyran-2-ol mit (Carbäthoxymethylen)triphenylphosphoran (3,2 g) kondensiert. Man erhält 2,6 g (92,8 %) rac-3,4-Dihydro-7-(phenylmethoxy)-2H-1-benzopyran-2-essigsäure-äthylester als gelbes Öl, das man dann mittels Säulenchromatographie reinigt. Nach Hydrogenolyse über 0,2 g 10-proz. Palladium/Kohle in Analogie zu Beispiel 30 erhält man 1,9 g rac-3,4-Dihydro-7-hydroxy-2-H 1-benzopyran-2-essigsäure-äthylester als blassgelbes Öl.

**Beispiel 35**

In Analogie zu Beispiel 31 werden 0,9 g rac-3,4-Dihydro-7-hydroxy-2H-1-benzopyran-2-essigsäure-äthylester mit 3 g 4'-(3-Brompropoxy)-2'-hydroxy-3'-n-propylacetophenon alkyliert. Das Rohprodukt (2,6 g) wird an 50 g Kieselgel chromatographiert, wobei man 1,2 g des gewünschten Esters als blassgelbes Öl erhält (als Elutionsmittel verwendet man Toluol/Essigester (19:1). Das erhaltene Material wird mit 2 g Lithiumhydroxid-Monohydrat in Analogie zu Beispiel 31 verseift. Die rohe Säure (1,1 g) wird an 50 g Kieselgel chromatographiert. Durch Eluieren mit Toluol/Essigester (2:1 und 1:1) erhält man 682 mg (40,5 %) rac-7-[3-(4-Acetyl-3-hydroxy-2-propylphenoxy)propoxy]-3,4-dihydro-2H-1-benzopyran-essigsäure als blassgelben Festkörper vom Schmelzpunkt 107-109,5°.
Analyse für $C_{25}H_{30}O_7$ Berechnet: C, 67,86. H, 6,83. Gefunden: C, 68,07; H, 6,87.

**Beispiel 36**

Eine Lösung von 8,75 g Äthyl-6-hydroxychromon-2-carboxylat in 200 ml Essigsäure wird in Gegenwart von 2 g 10-proz. Palladium/Kohle bei 3,45 bar und Raumtemperatur hydriert, und zwar indem man die Suspension während 17 Stunden schüttelt. Der Katalysator wird abfiltriert, und das Filtrat wird unter vermindertem Druck eingedampft. Man erhält 7,85 g Rohprodukt. Durch Reinigung dieser Substanz mittels Hochdruckflüssigkeitschromatographie unter Eluieren mit Hexan/Essigester (3:1) erhält man 4,9 g (59 %) reines kristallines rac-3,4-Dihydro-6-hydroxy-2H-1-benzopyran-2-carbonsäure-äthylester. Durch Umkristallisieren aus Essigester/Hexanerhält man einen farblosen Festkörper vom Schmelzpunkt 72,74°.
Analyse für $C_{12}H_{14}O_4$: Berechnet: C, 64,85; H, 6,35: Gefundenen: C, 64,57; H, 6,45.

**Beispiel 37**

In eine Lösung von 2,23 g rac-Äthyl-6-hydroxy-chroman-2-carboxylat in 30 ml Essigsäure wird bei 20-25° Bortrifluorid-Gas eingeleitet. Während der Einleitung des Gases (30-45 Minuten) wird mit einem kalten Wasserbad gekühlt um die Temperatur der exothermen Reaktion zu kontrollieren. Das kalte Bad wird anschliessend entfernt und die Reaktionsmischung wird während 5 Stunden auf 80° erhitzt. Anschliessend wird die Reaktionsmischung abgekühlt und vorsichtig auf eine Mischung aus gesättigter Natriumbicarbonatlösung und Eis gegossen. Das Produkt wird mit Tetrahydrofuran/Äther (1:1) ausgeschüttelt. Das nach dem Eindampfen des Lösungsmittels erhaltene rohe Material wird an Kieselgel chromatographiert, und man erhält 1,6 g (60 %) rac-7-Acetyl-6-hydroxy-3,4-dihydro-2H-1-benzopyran-2-carbonsäure-äthylester als viskoses Öl.

**Beispiel 38**

In Analogie zu Beispiel 42 wird 1,0 g rac-Äthyl-6-hydroxychroman-2-carboxylat in rac-6-[3-(4-Acetyl-3-hydroxy-2-propylphenoxy)propoxy]-3,4-dihydro-2H-1-benzopyran-2-carbonsäure übergeführt. Man erhält das gewünschte Produkt in einer Ausbeute von 27,3 % als weisses kristallines Material vom Schmelzpunkt 125-127°.

Analyse für $C_{24}H_{28}O_7$: Berechnet: C, 67,28; H, 6,59. Gefunden: C, 67,27; H. 6,67.

**Beispiel 39**

Eine Mischung aus 1,6 g rac-7-Acetyl-6-hydroxychroman-2-carbonsäure-äthylester, 3,0 g Kaliumcarbonat, 5 ml 1,3-Dibrompropan, 60 ml trockenem Aceton und 30 ml trockenem Dimethylformamid wird unter Rühren und unter Rückfluss während 20 Stunden zum Sieden erhitzt. Der Grossteil der Lösungsmittel wird im Vakuum entfernt, und der Rückstand wird mit Äther verdünnt und vorsichtig mit 1N-Salzsäure behandelt. Die organische Phase vird abgetrennt, mit gesättigter Natriumbicarbonatlösung und Wasser gewaschen, getrocknet und im Vakuum eingedampft. Das rohe Brompropyl derivat (2,1 g) wird in 30 ml Aceton gelöst, worauf man die erhaltene Lösung zu einer Mischung aus 1,2 g 2',4'-Dihydroxy-3'-propylacetophenon, 2,5 g wasserfreiem Kaliumcarbonat, 30 ml trockenem Aceton und 30 ml Dimethylformamid gibt. Das Reaktionsgemisch wird während 25 Stunden unter gelindem Sieden und Rückfluss erhitzt. Nach Entfernen des Grossteils des Lösungsmittels wird das Produkt mit Äther extrahiert. Die ätherischen Auszüge werden mit Kochsalzlösung gewaschen und getrocknet. Das rohe Material (3,1 g), das man nach Eindampfen der Lösung erhält, wird an Kieselgel chromatographiert, worauf man 1,3 g (43 %) des gewünschten Esters erhält. Dieses Material wird hydrolysiert, indem man es (1,3 g in 18 ml Tetrahydrofuran) mit einer wässrigen Lösung von Lithiumhydroxid (2,2 g Lithiumhydroxid-Monohydrat in 18 ml Wasser) behandelt. Das trübe Reaktionsgemisch wird während 21 Stunden bei Raumtemperatur gerührt und auf 1N-Salzsäure gegossen, worauf man das Produkt mit Äther extrahiert. Die organische Phase wird mit Kochsalzlösung gewaschen, getrocknet und eingedampft. Man erhält 1,2 g eines viskosen gelben Öls. Dieses wird mittels Säulenchromatographie an Kieselgel gereinigt, wobei man 0,778 g (27,3 %) rac-7-Acetyl-6-[3-(4-acetyl-3-hydroxy-2-propylphenoxy)propoxy]-3,4-dihydro-2H-1-benzopyran-2-carbonsäure als weisses kristallines Material vom Schmelzpunkt 140,5-142,5° (aus Essigester) erhält.

Analyse für $C_{26}H_{30}O_8$: Berechnet: C, 66,37; H, 6,43. Gefunden: C, 66,44; H, 6,51.

**Beispiel 40**

In Analogie zu Beispiel 36 werden 1,09 g Äthyl-7-hydroxychromon-2-carboxylat zu rac-Äthyl-7-hydroxychroman-2-carboxylat hydriert. Man erhält das Produkt in einer Ausbeute von 53 % als kristallines Material vom Schmelzpunkt 81-82,5° (aus Essigester/Hexan).

Analyse für $C_{12}H_{14}O_4$ Berechnet: C, 64,85; H, 6,35. Gefunden: C, 64,60; H 6,26.

**Beispiel 41**

1,1 g rac-Äthyl-7-hydroxychroman-2-carboxylat werden mit 10 ml Essigsäureanhydrid/Pyridin (1:1) versetzt, worauf man während 17 Stunden bei Raumtemperatur rührt. Man entfernt die Lösungsmittel, löst den Rückstand (1,2 g) in 12 ml Eisessig und behandelt dann mit 1,26 ml Bortrifluoridätherat. Das Reaktionsgemisch wird während 17 Stunden unter gelindem Sieden und unter Rückfluss erhitzt. Anschliessend kühlt man ab, giesst vorsichtig auf eine Mischung aus Natriumbicarbonat und Eis und extrahiert das Produkt mit Tetrahydrofuran/Äther (1:1). Nach Entfernen des Lösungsmittels erhält man 1,1 g rohe rac-7-Hydroxy-6-acetylchroman-2-carbonsäure. Dieses Material wird in den entsprechenden Äthylester übergeführt, und zwar indem man 1,1 g der rohen Säure in absolutem Äthanol und in Gegenwart von 10 mg p-Toluolsulfonsäure während 17 Stunden unter Rückfluss zum Sieden erhitzt. Man entfernt dann den Grossteil des Lösungsmittel, extrahiert das Produkt mit Tetrahydrofuran/Äther (1:1), wäscht mit gesättigter Natriumbicarbonatlösung und Wasser, trocknet und dampft ein. Man erhält 1,2 g des rohen Äthylesters als Mischung des 6-Acetyl- und 8-Acetylderivates in einem Verhältnis von etwa 7:1. Dieses Material wird mittels Säulenchromatographie an Kieselgel gereinigt. Man erhält 0,7 g (53,5 %) rac-6-Acetyl-3,4-dihydro-7-hydroxy-2H-1-benzopyran-2-carbonsäureäthylester als farbloses kristallines Material vom Schmelzpunkt 93,5-95,5° (aus Essigester/Hexan).

**Beispiel 42**

Eine Mischung aus 1 g rac-Äthyl-7-hydroxychroman-carboxylat, 1,6 g 4'-(3-Brompropoxy)-2'-hydroxy-3-propylacetophenon, 1,6 g wasserfreiem Kaliumcarbonat, 25 ml Aceton und 12 ml Dimethylformamid wird unter Rühren und unter Rückfluss während 23 Stunden zum Sieden erhitzt. Nach dem Abkühlen versetzt man vorsichtig mit 1N-Salzsäure und extrahiert das Produkt mit Äther. Die ätherische Phase wird mit Kochsalzlösung gewaschen, getrocknet und im Vakuum ein gedampft. Man erhält 3,6 g eines orangefarbenen

Öls. Durch chromatographische Reinigung an Kieselgel erhält man 1,4 g des alkylierten Äthylesters. Dieses Material wird in 20 ml Tetrahydrofuran auf genommen und dann mit wässrigem Lithium hydroxid (2,6 g Lithiumhydroxid-Monohydrat in 20 ml Wasser) versetzt, worauf man während 23 Stunden bei Raumtemperatur rührt. Das Reaktionsgemisch wird angesäuert, und das Produkt wird mit Äther extrahiert. Man wäscht mit Kochsalzlösung und trocknet. Nach Entfernen des Lösungsmittels erhält man 1,3 g der gewünschten Säure als blassgelben Körper, den man mittels Säulenchromatographie an Kieselgel reinigt. Man erhält 0,554 g (28,7 %) rac-7-[3-(4-Acetyl-3-hydroxy-2-propyl)phenoxy)propoxy]-3,4-dihydro-2H-1-benzopyran-2-carbonsäure als weisses, kristallines Material vom Schmelzpunkt 142,5-145°.

Analyse für $C_{24}H_{28}O_7$: Berechnet: C, 67,28; H, 6,59. Gefunden: C, 67,34; H, 6,62.

**Beispiel 43**

In Analogie ZU Beispiel 39 werden 0,84 g rac-Äthyl-6-acetyl-7-hydroxychroman-2-carboxylat in rac-6-Acetyl-7-[3-4-acetyl-3-hydroxy-2-propylphenoxy)propoxy]-3,4-dihydro-2H-1-benzopyran-2-carbonsäure übergeführt. Man erhält das gewünschte Produkt in einer Ausbeute von 49,5 % als farblosen Festkörper vom Schmelzpunkt 176-178,5° (Zersetzung; aus Essigester).

Analyse für $C_{26}H_{30}O_8$ Berechnet: C, 66,37; H, 6,43. Gefunden: C, 66,27; H, 6,39.

**Beispiel 44**

In Analogie zu Beispiel 36 werden 5,52 g Äthyl-7-hydroxy-8-propylchormon-2-carboxylat zu rac-Äthyl-7-hydroxy-8-propylchroman-2-carboxylat hydriert. Man erhält das gewünschte Produkt in einer Ausbeute von 43,5 % als viskoses, blassgelbes Öl.

**Beispiel 45**

In Analogie zu Beispiel 41 werden 1,3 g rac-3,4-Dihhydro-7-hydroxy-8-propyl-2H-1-benzopyran-carbonsäure-äthylester in das entsprechend 6-Acetylderivat übergeführt. Man erhält das gewünschte Produkt in einer Ausbeute von 79,5 % als viskoses Öl.

**Beispiel 46**

In Analogie zu Beispiel 42 werden 1,0 g rac-7-Hydroxy-8-propylchroman-2-carbonsäure-äthylester in rac-7-[3-(4-Acetyl-3-hydroxy-2-propyl)phenoxy)propoxy]-3,4-dihydro-8-propyl-2H-1-benzopyran-2-carbonsäure übergeführt. Man erhält das gewünschte Produkt als blassgelben Festkörper vom Schmelzpunkt 105-107°.

Analyse für $C_{27}H_{34}O_7$: Berechnet: C, 68,92; H, 7,28. Gefunden: C, 69,06; H, 7,29.

**Beispiel 47**

Eine Mischung aus 0,85 g rac-Äthyl-6-acetyl-7-hydroxy-8-propylchroman-2-carboxylat, 1,2 g wasserfreiem Kaliumcarbonat, 2,3 ml 1,3-Dibrompropan, 0,1 g 18-Crown-6-äther und 20 ml Acetonitril wird während 23 Stunden unter Rückfluss zum Sieden erhitzt. Nach dem Abkühlen wird das Reaktionsgemisch mit Äther verdünnt. Die organische Phase wird mit Kochsalzlösung gewaschen, getrocknet und eingedampft. Man erhält 1,2 g des rohen Propylderivates in Form eines gelben Öls. In Analogie zu Beispiel 39 wird dieses Bromid mittels Alkylierung von 2',4'-Dihydroxy-3'-propyl-acetophenon und anschliessende Verseifung in rac-6-Acetyl- 7-[3-(4-acetyl-3-hydroxy-2-propylphenoxy)propoxy]-3,4-dihydro-8-propyl-2H-1-benzopyran-2-carbonsäure übergeführt. Man erhält das gewünschte Produkt in einer Gesamtausbeute von 40 % als farblosen Festkörper vom Schmelzpunkt 108-110,5° (aus Essigester/Hexan).

Analyse für $C_{29}H_{36}O_8$ Berechnet: C, 67,95; H, 7,08. Gefunden: C, 68,16; H, 7,29.

**Beispiel 48**

In Analogie zu Beispiel 39 jedoch unter Verwendung von 1,5-Dibrompentan anstelle von 1,3-Dibrompropan wird rac-6-Acetyl-7-hydroxy-3,4-dihydro-2H-1-benzopyran-2-carbonäure-äthylester in rac-6-Acetyl-7-[5-(4-acetyl-3-hydroxy-2-pro-pylphenoxy)pentyloxy]-3,4-dihydro-2H-1-benzopyran-2-carbon-säure übergeführt. Man erhält das gewünschte Produkt in einer Ausbeute von 32,8 % als farblosen Festkörper vom Schmelzpunkt 113-115° (aus Essigester/Hexan).

Analyse für $C_{28}H_{34}O_8$ Berechnet: C, 67,45: H, 6,87. Gefunden: C, 67,61; H, 6,80.

**Beispiel 49**

In Analogie zu Beispiel 39, jedoch unter Verwendung von 1,7-Dibromheptan anstelle von 1,3-Dibrompropan, wird rac-6-Acetyl-7-hydroxy-3,4-dihydro-2H-1-benzopyran-2-carbonsäure-äthylester in rac-6-Acetyl-7-[7-(4-acetyl-3-hydroxy-2-propylphenoxy)heptyloxy]-3,4-dihydro-2H-1-benzopyran-2-carbonsäure übergeführt. Man erhält das gewünschte Produkt in einer Gesamtausbeute von 39,1 % als farblosen Festkörper vom Schmelzpunkt 133-135° (aus Essigester/Hexan).

Analyse für $C_{30}H_{38}O_8$ Berechnet: C, 68, 42; H, 7,27. Gefunden: C, 68,22; H, 7,18.

**Beispiel 50**

In Analogie zu Beispiel 39 werden 0,62 g rac-8-Acetyl-7-hydroxy-3,4-dihydro-2H-1-benzopyran-2-carbonsäure-äthylester in rac-8-Acetyl-7-[3-(4-acetyl-3-hydroxy-2propyl-phenoxy)propoxy]-3,4-dihydro-2H-1-benzopyran-2-carbonsäure übergeführt. Man erhält das gewünschte Produkt in einer Gesamtausbeute von 27,7 % als farblosen Festkörper vom Schmelzpunkt 146,5-148,5° (aus Essigester/Hexan).

Analyse für $C_{26}H_{30}O_8$: Berechnet: C, 66,37; H, 6,43. Gefunden: C, 66,51; H. 6,47.

**Beispiel 51**

In Analogie zu Beispiel 37 Verden 0,125 g rac-3,4-Dihydro-6-hydroxy-2-methyl-2H-1-benzopyran-carbonsäure-äthylester in rac-3,4-Dihydro-7-acetyl-6-hydroxy-2-methyl-2H-1-benzopyran-2-carbonsäure-äthylester übergeführt. Man erhält das gewünschte Produkt in einer Ausbeute von 65,6 % als viskoses Öl.

**Beispiel 52**

In Analogie zu Beispiel 39 werden 96,5 mg rac-3,4-Dihydro-7-acetyl-6-hydroxy-2-methyl-2H-1-benzopyran-2-carbonsäure-äthylester in rac-7-Acetyl-6-[3-(4-acetyl-3-hydroxy-2-propylphenoxy)propoxy]-3-,4-dihydro-2-methyl-2H-1-benzopyran-2-carbonsäure übergeführt. Man erhält das gewünschte Produkt in einer Ausbeute von 65,7 % als Schaum.

**Beispiel 53**

Eine Mischung aus 1,14 g 4-Benzyloxy-2-hydroxybenzaldehyd, 0,125 g 1,4-Diazabicyclo[2.2.2]octan und 0,65 g Acrylnitril wird während 2 Stunden unter Rühren auf 80-85° erhitzt. Man entfernt das Lösungsmittel, nimmt den Rückstand in Tetrahydrofuran-Äther (1:1) auf, wäscht mit 1N-Natriumhydroxid und Wasser, trocknet und entfernt die Lösungsmittel im Vakuum. Das Rohprodukt (1,1 g) wird mittels Säulenchromatographie an Kieselgel gereinigt. Man erhält 0,6 g (45,7 %) 7-(Phenylmethoxy)-2H-1-benzopyran-3-carbonitril als farblosen Festkörper vom Schmelzpunkt 95-98° (aus Essigester/Hexan).

Analyse für $C_{17}H_{13}O_2$: Berechnet C, 77,55; H, 4,98; N, 5,32. Gefunden: C, 77,41; H, 5,00: N, 4,80.

**Beispiel 54**

Man löst 2,2 g 7-(Phenylmethoxy)-2H-1-benzopyran-2-carbonitril in 40 ml einer Mischung aus Tetrahydrofuran und Äthylalkohol (1:1), versetzt mit 20 ml 2N-Kaliumhydroxid und erhitzt das Reaktionsgemisch während 21

Stunden unter Rückfluss zum Sieden. Man versetzt dann mit einer zusätzlichen Portion von 10 ml 2N-Kaliumhydroxid und erhitzt noch während zusätzlichen 27 Stunden unter Rühren und Rückfluss zum Sieden. Man stellt das Reaktionsgemisch dann mit 1N-Salzsäure sauer, extrahiert mit Essigester, wäscht mit Kochsalzlösung, trocknet und dampft im Vakuum ein. Man eshält 2,2 g eines orangefarbenen Festkörper. Man nimmt dieses Material in 30 ml Äthanol auf, versetzt mit 0,3 g p-Toluolsulfonsäure-Monohydrat und erhitzt während 18 Stunden unter Rückfluss zum Sieden. Nach Entfernen des Grossteils des Lösungsmittels extrahiert man mit Äther, wäscht die ätherische Phase mit gesättigter Natriumbicarbonatlösung und Wasser, trocknet und dampft ein. Man erhält 2,3 g des rohen Äthylesters. Durch Reinigung mittels Chromatographie an einer Kieselgelsäule erhält man 1,4 g 7-(Phenylmethoxy)-2H-1-benzopyran-3-carbonsäure-äthylester als kristallines Material.

**Beispiel 55**

Man löst 1,4 g des Esters aus Beispiel 54 in 25 ml absolutem Alkohol und hydriert bei Atmosphärendruck in Gegenwart von 0,2 g 10-proz. Palladium/Kohle. Man entfernt den Katalysator mittels Filtration durch Kieselgur und dampft das Filtrat dann ein. Man erhält 0,9 g rac-3,4-Dihydro-7-hydroxy-2H-1-benzopyran-3-carbonsäure-äthylester als weissen Festkörper.

**Beispiel 56**

In Analogie zu Beispiel 42 wird der Äthylester aus Beispiel 55 in rac-7-[-3-(4-Acetyl-3-hydroxy-2-propylphenoxy)propoxy]-3,4-dihydro-2H-1-benzopyran-3-carbonsäure übergeführt. Man erhält die gewünschte Säure in einer Gesamtausbeute von 17,7 % als farblosen Festkörper vom Schmelzpunkt 153,5-155,5° (aus Essigester/Äther/Hexan).

Analyse für $C_{24}H_{28}O_7$: Berechnet: C, 67,28; H, 6,59. Gefunden: C, 67,46; H, 6,66.

**Beispiel 57**

Man löst 0,9 g rac-7-Hydroxy-3,4-dihydro-2H-1-benzopyran-3-carbonsäure-äthylester in 5 ml Pyridin und versetzt mit 1,2 ml Essigsäureanhydrid. Man rührt während 3 Stunden bei Raumtemperatur, und verdünnt die Reaktionsmischung mit Äther. Die organische Phase wird mit 2H-Salzsäure, Natriumbicarbonatlösung und Kochsalzlösung gewaschen, getrocknet und eingedampft. Man erhält 1,2 g des rohen Acetates, das man in 10 ml Eisessig aufnimmt und dann mit 1 ml Bortrifluorid-Ätherat versetzt. Man erhitzt das Reaktionsgemisch während 6 Stunden auf 100°, kühlt ab und versetzt mit Äther. Die organische Phase wird mehrere Male mit Wasser gewaschen, getrocknet und im Vakuum eingedampft. Man erhält 1,0 g eines orangefarbenen Öles. Dieses Material wird in Analogie zu Beispiel 41 mit Äthanol verestert. Man erhält rac-6-Acetyl-7-hydroxy-3,4-dihydro-2H-1-benzopyran-3-carbonsäure-äthylester.

**Beispiel 58**

In Analogie zu Beispiel 39 wird rac-6-Acetyl-7-hydroxy-3,4-dihydro-2H-1-benzopyran-3-carbonsäure-äthylester in rac-6-Acetyl-7-[3-(4-acetyl-3-hydroxy-2-propylphenoxy)propoxy]-3,4-dihydro-2H-1-benzopyran-3-carbonsäure übergeführt. Man erhält die gewünschte Säure als farblosen Festkörper vom Schmelzpunkt 127-129° (Zersetzung; aus Essigester/Hexan).

Analyse für $C_{26}H_{30}O_8$: Berechnet: C, 66,37; H, 6,43. Gefunden: C, 66,45; H, 6,43.

**Beispiel 59**

In Analogie zu Beispiel 41 werden 11,3 g rac-7-Hydroxy-3-4-dihydro-2H-1-benzopyran-2-carbonsäure-äthylester in eine Ausbeute von 93 % (11 g) in die rohe rac-7-Hydroxy-6-acetyl-3-4-dihydro-2H-1-benzopyran-2-carbonsäure übergeführt. 1 g dieser rohen Säure wird in Gegenwart von 0,2 g p-Toluolsulfonsäure-Monohydrat mit 3,8 g (-)-(2R, 3R)-Butandiol in 30 ml Toluol behandelt. Man erhitzt während 18 Stunden unter Rückfluss zum Sieden, kühlt das Reaktionsgemisch ab, giesst es auf gesättigte Natriumbicarbonatlösung und extrahiert das Produkt mit Äther. Die ätherischen Auszüge werden zweimal mit Wasser gewaschen, getrocknet und eingedampft. Man erhält 1,8 g des Rohproduktes, bestehend aus einer Mischung des 6-Acetyl- und des 8-Acetyl-Monobutandiolester-Derivates im Verhältnis von etwa 7:1. Durch Reinigung dieses Materials mittels HPLC erhält man 0,8 g (61 %) [2R,S-2-[(1R*, 2R*)]]-5-Acetyl-7- hydroxy-3,4-dihydro-2H-1-benzopyran-2-carbonsäure-1-methyl-2-hydroxypropylester als viskoses blassgelbes Öl.

**Beispiel 60**

In Analogie zu Beispiel 25 erhält man 3,5 g [2R,S-2-[(1R*, 2R*)]]-6-Acetyl-7-hydroxy-3,4-dihydro-2H-1-benzopyran-2-carbonsäure-1-methyl-2-hydroxypropylester, 12 ml 1,5-Dibrompentan, 7,2 g wasserfreiem Kaliumcarbonat in 100 ml Aceton und 50 ml NN-Dimethylformamid 2,6 g (50 %) [2R, S-2-[(1R*, 2R*)]]-6-Acetyl-7-[(5-brompentyl)oxy]-3,4-dihydro-2H-1-benzopyran-2-carbonsäure-2-hydroxymethyl-propylester. Dieses Material wird dann mittels HPLC unter Eluieren mit Toluol/Essigester (2:1) und Recyclieren gereinigt. Die weniger polare diastereoisomere Form wird aus Diathyläther umkristallisiert und liefert 786 mg [2S-[2β-(1R*, 2R*)]]-6-Acetyl-7-](5-brompentyl)oxy]-3,4-dihydro- 2H-1-benzopyran-2-carbonsäure-2-hydroxy-1-methylpropylesterals farblosen Festkörper vom Schmelzpunkt 94,5-98°;
$[\alpha]_D^{25}$ -7,99° (c 1. $CHCl_3$)
Analyse für $C_{21}H_{29}BrO_6$ Berechnet: C, 55,15; H, 6,39 Gefunden: C, 54,79; H, 6,20.
Durch umkristallisieren der starker polaren diastereoisomeren Form aus Diäthyläther erhält man 1,03 g [2R] [2α-(1R*,2R*)]]-5-Acetyl-7-[(5brompentyl)oxy]-3,4-dihydro-2H-1-benzopyran-2-carbonsäure-2-hydroxy-1-methylpropylester als farblosen Festkörper vom Schmelzpunkt 80-83°;
$[\alpha]^{25}_D$ -21,49° (c 1, $CHCl_3$), Analyse für $C_{21}H_{29}BrO_6$ Berechnet: C, 55,15: H, 6,39 Gefunden: C, 55,06; H, 6,39.

**Beispiel 61**

In Analogie zu Beispiel 26 werden 199,8 mg [2S-(2β-(1R*, 2R*]]-6-Acetyl-7-[(5-Brompentyl)oxy]-3,4-dihydro-2H-1-benzopyran-2-carbonsäure-2-hydroxy-1-methylpropylester mit 2',4'-Dihydroxy-3'-n-propylacetophenon alkyliert. Nach Chromatographischer Reinigung an einer Kieselgelsäule erhält man 106 mg (42,8 %) [2S-[2β(1R*,2R*)]]-6-Acetyl-7-[5-(4-acetyl-3-hydroxy-2-propylphenoxy)pentyloxy]-3,4-dihydro-2H-1-benzopyran-2-carbonsäure2-hydroxy-1-methylpropylester vom Schmelzpunkt 124-127,5° (aus Essigester/Hexan); $[\alpha]^{25}_D$ -7,25° (c 1, $CHCl_3$).
Analyse für $C_{32}H_{42}O_9$ Berechnet: C, 67,35: H, 7,42 Gefunden: C, 67,14; H, 7,51.

**Beispiel 62**

In Analogie zu Beispiel 61 werden 554 mg des 2R-Brom-Esters aus Beispiel 60 in den [2R-(2α(1R*, 2R*)]-6-Acetyl-7-[5-(4-acetyl-3-hydroxy-2-propylphenoxy)pentyloxy]-3,4-dihydro-2H-1-benzopyran-2-carbonsäure-2-hydroxy-1-methylpropylester übergeführt. Man erhält das gewünschte Produkt in einer Ausbeute von 52,6 % als Stoff mit einem Schmelzpunkt von 100-105° (aus Essigester/Hexan);
$[\alpha]^{25}_D$ -15,75° (c 1, $CHCl_3$),
Analyse für $C_{32}H_{42}O_9$ Berechnet: C, 67,35; H, 7,42 Gefunden: C, 66,98; H, 7,39.

**Beispiel 63**

In Analogie zu Beispiel 4 werden 405 mg des 2S-isomeren Esters aus Beispiel 61 mit 0,6 g Lithiumhydroxid-Monohydrat in 10 ml Tetrahydrofuran/Wasser (1:1) verseift. Nach Kristallisieren aus Essigester/Hexan erhält man 185 mg (52,5 %) (S)-6-Acetyl-7-[5-(4-acetyl-3-hydroxy-2-propylphenoxy)-pentyloxy]-3,4-dihydro-2H-1-benzopyran-2-carbonsäure vom Schmelzpunkt 94-97,5°; $[\alpha]^{25}_D$ +1,26° (c 1, $CHCl_3$).
Analyse für $C_{28}H_{34}O_8$ Berechnet: C, 67,45; H, 6,87 Gefunden: C, 66,60; H, 6,70.

**Beispiel 64**

In Analogie zu Beispiel 63 erhält man aus 502 mg des 2R-isomeren Esters aus Beispiel 62 185 mg (40,7 %) (R)-6-Acetyl-7-[5-(4-acetyl-3-hydroxy-2-propylphenoxy)pentyloxy]-3,4-dihydro-2H-1-benzopyran-2-carbonsäure, das aus Essigester/Hexan umkristallisiert wird und dann einen Schmelzpunkt von 102,5-108° hat; $[\alpha]^{25}_D$ -1,02° (c 1, $CHCl_3$).
Analyse für $C_{28}H_{34}O_8$ Berechnet: C, 67,45; H, 6,87 Gefunden: C, 67,26; H, 6,78.

**Beispiel 65**

Eine Mischung aus 1 g (4,5 mMol) rac-3,4-Dihydro-7- hydroxy-2H-1-benzopyran-2-carbonsäure-äthylester, 3,7 ml (27,2 mMol) 1,5-Dibrompentan, 2 g (14,5 mMol) wasserfreiem Kaliumcarbonat. 30 ml Aceton und 15 ml N,N-Dimethylformamid werden unter Ruhren und Rückfluss während 20 Stunden zum Sieden erhitzt. Die Mischung wird dann abgekühlt und mit Äther verdünnt. Die ätherische Lösung wird mit Wasser und gesättigter Kochsalzlösung gewaschen, über wasserfreiem Magnesiumsulfat getrocknet, filtriert und unter vermindertem Druck eingedampft. Man erhält 7,2 g einer gelben Flüssigkeit. Dieses Material wird an 50 g Kieselgel chromatographiert. Durch Eluieren mit Toluol/Essigester (19:1 und 9:1) erhält man 1,2 g (72 %) rac-7-[(5-Brompentyl)oxy]-3,4- dihydro-2H-1-benzopyran-2-carbonsäure-äthylester als blassgelbes Öl.

**Beispiel 66**

In Analogie zu Beispiel 65 (gleiche Bedingungen und gleiche molaren Verhältnisse) können die nachfolgend aufgeführten Verbindungen aus den angegebenen Ausgangsstoffen hergestellt werden. Alle Verbindungen werden mittels Chromatographie an Kieselgel gereinigt und als blassgelbe Öle isoliert:

rac-6-Acetyl-7-[(5-brompentyl)oxy]-3,4-dihydro-8-propyl-2H-1-benzopyran-2-carbonsäure-äthylester aus rac-6-Acetyl-7-hydroxy-3,4-dihydro-8-propyl-2H-1-benzopyran-2-carbonsäure-äthylester und 1,5-Dibrompentan.

rac-6-Acetyl-7-[(5-brompentyl)oxy]-3,4-dihydro-2H-1-benzopyran-2-carbonsäure-äthylester aus rac-6-Acetyl-3,4-dihydro-7-hydroxy-2H-1-benzopyran-2-carbonsäure-äthylester und 1,5-Dibrompentan.

rac-6-Acetyl-7-[(6-bromhexy)oxy]-3,4-dihydro-2H-1-benzopyran-2-carbonsäure-äthylester aus rac-6-Acetyl-3,4-dihydro-7-hydroxy-2H-1-benzopyran-2-carbonsäure-äthylester und 1,6-Dibromhexan.

rac-6-Acetyl-7-[(4-brombutyl)oxy]-3,4-dihydro-2H-1-benzopyran-2-carbonsäure-äthylester aus rac-6-Acetyl-3,4-dihydro-7-hydroxy-2H-1-benzopyran-2-carbonsäure-äthylester und 1,4-Dibrombutan.

rac-7-[(5-Brompentyl)oxy]-3,4-dihydro-8-propyl-2H-1-benzopyran-2-carbonsäure-äthylester aus rac-3,4-dihydro-7-hydroxy-8-propyl-2H-1-benzopyran-2-carbonsäure-äthylester und 1,5-Dibrompentan.

rac-8-Acetyl-7-[(5-brompentyl)oxy]-3,4-dihydro-2H-1-benzopyran-2-carbonsäure-methylester aus rac-8-Acetyl-3,4-dihydro-7-hydroxy-2H-1-benzopyran-2-carbonsäure-methylester und 1,5-Dibrompentan.

**Beispiel 67**

Eine Mischung aus 1,2 g (3,23 mMol) rac-7-[(5-Brompentyl)oxy]-3,4-dihydro-2H-1-benzopyran-2-carbonsäure-äthylester, 1,0 g (5,15 mMol) 2',4'-Dihydroxy-3'-propylacetophenon, 1,8 g (13,0 mMol) wasserfreiem Kaliumcarbonat, 30 ml Aceton und 15 ml N,N-Dimethylformamid wird unter Rühren und unter Rückfluss während 6 Stunden zum Sieden erhitzt. Nach dem Abkühlen wird die Mischung mit Äther verdünnt. Die ätherische Phase wird mit Wasser und Kochsalzlösung gewaschen, über wasserfreiem Magnesiumsulfat getrocknet, filtriert und unter vermindertem Druck eingedampft. Den Rückstand (2,1 g) chromatographiert man an 50 g Kieselgel. Durch Eluieren mit Toluol/Essigester (19:1) erhält man 1,3 g (83 %) rac-7-[5-(4-Acetyl-3-hydroxy-2- propylphenoxy)pentyloxy]-3,4-dihydro-2H-1-benzopyran-2-carbonsäure-äthylester als gelbes Öl.

**Beispiel 68**

In Analogie zu Beispiel 67 (gleiche Reaktionsbedingungen und gleiche molaren Verhältnisse) können die nachfolgend aufgeführten Verbindungen aus den angegebenen Ausgangasstoffen hergestellt werden. Alle Verbindungen werden durch Chromatographie an Kieselgel gereinigt und als blassgelbe Öle isoliert:

rac-6-Acetyl-7-[5-(4-acetyl-3-hydroxyphenoxy)pentyloxy]-3,4-dihydro-8-propyl-2H-1-benzopyran-2-carbonsäureäthylester aus rac-6-Acetyl-7-[(5-brompentyl)oxy]-3,4-dihydro-8-propyl-2H-1-benzopyran-2-carbonsäure-äthylester und 2',4'-Dihydroxyacetophenon.

rac-6-Acetyl-7-[5-(4-acetyl-3-hydroxyphenoxy)pentyloxy]-3,4-dihydro-2H-1-benzopyran-2-carbonsäure-äthylester aus rac-6-Acetyl-7[(5-brompentyl)oxy]3,3-dihydro-2H-1-benzopyran-2-carbonsäure-äthylester und 2',4'-Dihydroxyacetophenon.

rac-6-Acetyl-7-[5-(4-acetyl-3-hydroxy-2-propylphenoxy)pentyloxy]-3,4-dihydro-8-propyl-2H-1-benzopyran-2-carbonsäure-äthylester aus rac-6-Acetyl-7-[(5-brompentyl)oxy]-3,4-dihydro-8-propyl-2H-1-benzopyran-2-carbonsäure-äthylester und 2',4'-Dihydroxy-3'-propylacetophenon.

rac-7-[5-(4-Acetyl-3-hydroxyphenoxy)pentyloxy]-3,4-dihydro-2H-1-benzopyran-2-carbonsäure-äthylester aus rac-7-[(5-Brompentyl)oxy]-3,4-dihydro-2H-1-benzopyran-2-carbonsäure-äthylester und 2',4'-Dihydroxyacetophenon.

rac-6-Acetyl-7-[6-(4-acetyl-3-hydroxy-2-propylphenoxy)hexyloxy]-3,4-dihydro-2H-1-benzopyran-2-carbonsäure-äthylester aus rac-6-Acetyl-7-[(6-bromhexyl)oxy]-3,4-dihydro-2H-1-benzopyran-2-carbonsaure-

äthylester und 2',4'-Dihydroxy-3'-propylacetophenon.

rac-6-Acetyl-7-[4-(4-acetyl-3-hydroxy-2-propylphenoxy)butoxy]-3,4-dihydro-2H-1-benzopyran-2-carbonsäure-äthylester aus rac-6-Acetyl-7-[(4-brombutyl)oxy]-3,4-dihydro-2H-1-benzopyran-2-carbonsäure-äthylester und 2',4'-Dihydroxy-3 -propylacetophenon.

rac-7-[5-[4-Acetyl-3-hydroxyphenoxy)pentyloxy]-3,4-dihy-dro-8-propyl-2H-1-benzopyran-2-carbonsäure-äthylester aus rac-7-[(5-Brompentyl)oxy]-3,4-dihydro-8-propyl-2H-1-benzo-pyran-2-carbonsäure-äthylester und 2',4 '-Dihydroxyacetophenon.

rac-7-[5-(4-Acetyl-3-hydroxy-2-propylphenoxy)pentyloxy]-3,4-dihydroxy-8-propyl-2H-1-benzopyran-2-carbonsäureäthylester aus rac-7-[(5-Brompenyl)oxy]-3,4-dihydro-8-propyl-2H-1-benzopyran-2-carbonsäure-äthylester und 2',4'-Dihydroxy-3'-propylacetophenon.

rac-8-Acetyl-7-[[5-(4-acetyl-3-hydroxy-2-propylphenoxy)pentyloxy]-3,4-dihydro-2H-1-benzopyran-2-carbonsäure-methylester aus rac-8-Acetyl-7-[(5-brompentyl)oxy]-3,4-dihydro-2H-1-benzopyran-2-carbonsäure-methylester und 2',4'-Dihydroxy-3'-propylacetophenon.

**Beispiel 69**

Eine Mischung aus 1,3 g (2,68 mMol) des Esters aus Beispiel 67, 60 ml Tetrahydrofuran/Wasser (1:1) und 2,4 g (57,1 mMol) Lithiumhydroxid-Monohydrat wird während 5 Stunden bei Raumtemperatur gerührt. Die Mischung wird mit Wasser verdünnt und dann dreimal mit Äther ausgeschüttelt. Die wässrige Phase wird mit 2N-Salzsäure auf pH 1 gestellt und dreimal mit Essigester extrahiert. Die Essigesterauszüge werden vereinigt, mit gesättigter Kochsalzlösung gewaschen, über wasserfreiem Magnesiumsulfat getrocknet, filtriert und unter vermindertem Druck eingedampft. Der Rückstand wird aus Essigester umkristallisiert, wobei man 0,725 g rac-7-[5-(4-Acetyl-3-hydroxy-2-propylphenoxy)pentyloxy]-3,4-dihydro-2H-1-benzopyran-2-carbonsäure als farblosen Festkörper vom Schmelzpunkt 124-126,5° erhält.

Analyse für $C_{26}H_{32}O_7$
Berechnet: C, 68,40; H, 7,07
Gefunden: C, 68,34: H, 7,29.

**Beispiel 70**

In Analogie zu Beispiel 69 (gleiche Reaktionsbedingungen und gleiche molare Verhältnisse) können die in der folgenden Tabelle aufgeführten Carbonsäuren durch Verseifen der entsprechenden Ester aus Beispiel 68 hergestellt werden;

| | Smp. (°C) | Umkrist. aus | Formel | Mikroanalys Ber. % C/H | Gef. % C/H |
|---|---|---|---|---|---|
| A | 105-107 | Essig-ester/Hexan | $C_{28}H_{34}O_8$ | 67,45/6,87 | 67,24/6,91 |
| B | 183-185,5 | Essig-ester/ | $C_{25}H_{28}O_8$ | 65,78/6,18 | 65,80/6,08 |
| C | Öl | -- | $C_{31}H_{40}O_8$ | 68,87/7,46 | 68,76/7,70 |
| D | 146.5]149 | Essig-ester/Hexan | $C_{23}H_{26}O_7$ | 66,65/6,32 | 66,51/6,29 |
| E | 138,5-142 | Essig-ester/Hexan | $C_{29}H_{36}O_8$ | 67,95/7,08 | 67,99/7,14 |
| F | 137-140 | Essig-ester/Hexan | $C_{27}H_{32}O_8$ | 66,93/6,66 | 67,21/6,77 |
| G | 120-123 | Essig-ester/Hexan | $C_{26}H_{32}O_7$ | 68,40/7,07 | 68,70/7,44 |
| H | oil | -- | $C_{29}H_{38}O_7$ | 69,86/7,68 | 70,02/7,80 |
| I | 134-136 | Aceto-nitril | $C_{28}H_{34}O_8$ | 67,45/6,87 | 67,20/6,84 |

A = rac-6-Acetyl-7-[5-(4-acetyl-3-hydroxyphenoxy)pentyloxy]-3,4-dihydro-8-propyl-2H-1-benzopyran-2-carbonsäure
B = rac-6-Acetyl-[5-(4-acetyl-3-hydroxyphenoxy)pentyloxy]-3,4-dihydro-2H-1-benzopyran-2-carbonsäure
C = rac-6-Acetyl-7-[5-(4-acetyl-3-hydroxy-2-propylphenoxy)pentyloxy]-3,4-dihydro-8-propyl-2H-1-benzopyran-2-carbonsäure
D = rac-7-[5-(4-Acetyl-3-hydroxyphenoxy)pentyloxy]-3,4-dihydro-2H-1-benzopyran-2-carbonsäure
E = rac-6-Acetyl-7-[6-(4-acetyl-3-hydroxy-2-propylphenoxy)-hexyloxy]-3,4-dihydro-2H-1-benzopyran-2-carbonsäure
F = rac-Acetyl-7-[4-(4-acetyl-3-hydroxy-2-propylphenoxy)butoxyl]-3,4-dihydro-2H-1-benzopyran-2-carbonsäure
G = rac-7-[5-(4-Acetyl-3-hydroxy-2-propylphenoxy)pentyloxy]-3,4-dihydro-8-propyl-2H-1-benzopyran-2-carbonsäure
H = rac-7-[5-(4-Acetyl-3-hydroxy-2-propylphenoxy)pentyloxy]-3,4-dihydro-8-propyl-2H-1-benzopyran-2-carbonsäure
I = rac-8-Acetyl-7-[[5-(4-acetyl-3-hydroxy-2-propylphenoxy)pentyl]oxy]-3,4-dihydro-2H-1-benzopyran-carbonsäure.

**Beispiel 71**

Eine Mischung aus 6,5 g (24,6 mMol) (R)-(+)-6-Hydroxy-3,4-dihydro-2,5,7,8-tetramethyl-2H-1-benzopyran-2-carbonsäuremethylester, 10,37 g (75,1 mMol) Vasserfreiem Kaliumcarbonat, 1,12 g 18-Crown-6, 39,3 ml (0,2 Mol) 1,5-Dibrompen-tan und 150 ml Acetonitril wird unter Rühren und unter Rückfluss während 21 Stunden zum Sieden erhitzt. Nach dem Abkühlen wird die Mischung mit Äther und Wasser versetzt. Die organische Phase wird abgetrennt, mit Wasser und Kochsalzlösung gewaschen, über wasserfreiem Magnesiumsulfat getrocknet, filtriert und unter vermindertem Druck einge dampft. Der Rückstand (68 g) wird zuerst mittels Säulenchromatographie an 400 g Kieselgel unter Eluieren mit Toluol/Essigester (49:1) und dann mittels präparativer HPLC an Kieselgel unter Verwendung von Hexan/Essigester (9:1) als mobile Phase gereinigt. Man erhält 9,71 g (95,6 %) (R)-(+)-6-[(5-Brompentyl)oxy]-3,4-dihydro-2,5,7,8-tetra-methyl-2H-1-benzypropan-2-carbonsäure-methylester als Festkörper. Durch Umkristallisieren aus Äthanol erhält man das gewünschte Produkt als farblosen Festkörper vom Schmelzpunkt 64-65,5°; $[\alpha]^{25}_D$ +36,37° (c 2, $CHCl_3$).
Analyse für $C_{20}H_{29}BrO_4$ Berechnet: C, 56,12; H, 7,07; Br, 19,33 Gefunden: C, 58,01; H, 7,07; Br, 19,20.

**Beispiel 72**

In Analogie zu Beispiel 71 wird (S)-(-)-6-Hydroxy-3,4-dihydro-2,5,7,8-tetramethyl-2H-1-benzopyran-2-carbonsäuremethylester mit 1,5-Dibrompentan zu (S)-(-)-6-[(5-Brompentyl)oxy]-3,4-dihydro-2,5,7,8-tetramethyl-2H-1-benzo-pyran-2-carbonsäure-methylester alkylert. Man erhält das gewünschte Produkt in einer Ausbeute von 88,5 %;
$[\alpha]^{25}_{D}$ -34,60° (c 2, $CHCl_3$).
Analyse für $C_{20}H_{29}BrO_4$ Berechnet: C, 58,12; H, 7,07; Br, 19,33 Gefunden: C, 58,00; H, 7,19; Br, 19,33.

**Beispiel 73**

In Analogie zu Beispiel 71 (gleiche Bedingungen und molaren Verhältnissen) werden 1 g rac-6-Hydroxy-3,4-dihydro-2,5,7,8-tetramethyl-2H-1-benzopyran-2-carbonsäure-methylester-mit 1,5-Dibrompentan zu rac-6-[(5-Brompentyl)oxy]-3,4- dihydro-2,5,7,8-tetramethyl-2H-1-benzopyran-2-carbon-säuremethylester alkyliert. Nach Reinigung mittels Chromatographie an Kieselgel erhält man das gewünschte Produkt in einer Ausbeute von 90 % als farblosen Festkörper vom Schmelzpunkt 72,5-74,5°.
Analyse für $C_{20}H_{29}BrO_4$ Berechnet: C, 58,12; H, 7,07; Br, 19,33 Gefunden: C, 58,13; H, 7,15; Br, 19,30.

**Beispiel 74**

Eine Mischung aus 7,35 g (17,8 mMol) des (R)-(-)-Bromesters aus Beispiel 71, 3,85 g (19,8 mMol) 2',4'-Dihydroxy-3'-propylacetophenon, 8,93 g (64,7 mMol) wasserfreiem Kaliumcarbonat, 154 ml trockenem Aceton und 77 ml trockenem N,N-Dimethylformamid wird unter Rühren und unter Rückfluss während 5,5 Stunden zum Sieden erhitzt. Die Mischung wird abgekühlt und mit Essigester verdünnt. Die organische Phase wird mit Wasser und gesättigter Kochsalzlösung gewaschen, über wasserfreiem Magnesiumsulfat getrocknet, filtriert und unter vermindertem Druck eingedampft. Man erhält 10,8 g eines Öls, das man mittels präparativer HPLC an Kieselgel unter Verwendung von Hexan/Essigester (2:1) als mobile Phase gereinigt wird. Man erhält 9,1 g (97 %) (R)-(+)-6-[5-(4-Acetyl-3-hydroxy-2-propylphenoxy)pentyloxy]-3,4-dihydro-2,5,7,8-tetramethyl-2H-1-benzopyran-2-carbonsäure-methylester als gelbes Öl; $[\alpha]^{26}_{D}$ +26,62° (c 2, $CHCl_3$).
Analyse für $C_{31}H_{42}O_7$ Berechnet: C, 70,70; H, 8,04 Gefunden: C, 70,06; H, 8,18.

**Beispiel 75**

In Analogie zu Beispiel 74 werden 8,4 g des (S)-(-)-Bromesters aus Beispiel 72 mittels Alkylierung von 2',4'-Dihydroxy-3'-propylacetophenon (4,4 g) in einer Ausbeute von 91 % in den (S)-(-)-6-[5-(4-Acetyl-3-hydroxy-2-propylphenoxy)pentyloxy]-3,4-dihydro-2,5,7,8-tetramethyl-2H-1-benzppyran-2-carbonsäure-methylester übergeführt. Dieses Material erhält man als gelbes Öl.
Analyse für $C_{31}H_{42}O_7$ Berechnet: C, 70,70; H, 8,04 Gefunden: C, 70,13; H, 7,73.

**Beispiel 76**

In Analogie zu Beispiel 74 (gleiche Reaktionsbedingungen und molare Verhältnisse) werden 1,2 g des rac-Bromesters aus Beispiel 73 mittels Alkylierung von 0,834 g 2'-4'-Dihydroxy-3-propylacetophenon in den rac-6-[5-(4-acetyl-3-hydroxy-2-propylphenoxy)pentyloxy]-3,4-dihydroxy-2,5,7,8-tetramethyl-2H-1-benzopyran-2-carbonsäure-methylester übergeführt. Man erhält das gewünschte Produkt in einer Ausbeute von 72 % als blassgelbes Öl.
Analyse für $C_{31}H_{42}O_7$ Berechnet: C, 70,70; H, 8,04 Gefunden: C, 70,44; H, 7,97.

**Beispiel 77**

Eine Mischung aus 7,55 g (14,3 mMol) des (R)-(+)-Esters aus Beispiel 74, 140 ml Tetrahydrofuran, 90 ml Wasser und 12,6 g (0,21 Mol) Lithiumhydroxid-Monohydrat wird während 7 Stunden bei Raumtemperatur gerührt und dann während 17 Stunden bei 0° gehalten. Die Mischung wird auf kalte 3H-Salzsäure gegossen. Die organischen Substanzen werden dann dreimal mit Essigester extrahiert. Die Extrakte werden gereinigt, mit Wasser und gesättigter Kochsalzlösung gewaschen, über wasserfreiem Magnesiumsulfat getrocknet, filtriert

und unter vermindertem Druck eingedampft. Die verbleibende gelbe glasartige Substanz (7,3 g) wird an 350 g Kieselgel chromatographiert. Unter Eluieren mit Toluol/Essigester (4:1, 2:1 und 1:1) erhält man die gewünschte Säure (5 g) welche man aus Acetonitril Umkristallisiert. Man erhält 2,49 g (34 %) (R)-(+)-6-[5-(4-Acetyl-3-hydroxy-2-propylphenoxy)pentylooxy]-3,4-dihydro-2,5,7,8-tetramethyl-2H-1-benzopyran-2-carbonsäure als farblosen Festkörper vom Schmelzpunkt 92,5-95°; $[\alpha]^{26}_D$ +37,86° (c 2, $CHCl_3$).

Analyse für $C_{30}H_{40}O_7$ Berechnet: C, 70,29; H, 7,87 Gefunden: C, 70,25; H, 7,92.

**Beispiel 78**

In Analogie zu Beispiel 77 werden 9,35 g (17,8 mMol) des (S)-(-)-Esters aus Beispiel 75 verseift. Man erhält (S)-(-)-6-[5-(4-Acetyl-3-hydroxy-2-propylphenoxy)pentyl-oxy]-3,4-dihydro-2,5,7,8-tetramethyl-2H-1-benzopyran-2-car-bonsäure als farblosen Festkörper vom Schmelzpunkt 90-93,5° (aus Acetonitril); $[\alpha]^{25}_D$ -38,18° (c 2, $CHCl_3$).

Analyse für $C_{30}H_{40}O_7$ Berechnet: C, 70,29; H, 7,87 Gefunden: C, 70,50; H, 8,11.

**Beispiel 79**

In Analogie zu Beispiel 77 werden 5,8 g (11 mMol) des racemischen Esters aus Beispiel 76 verseift. Man erhält 2,77 g (49 %) rac-6-[5-(4-Acetyl-3-hydroxy-2-propylphenoxy)pentyloxy]-3,4-dihydro-2,5,7,8-tetramethyl-2H-1-benzypyran-2-carbonsäure als farblosen Festkörper vom Schmelzpunkt 92,5-95°.

Analyse für $C_{30}H_{40}O_7$ Berechnet; C, 70,29; H, 7,87 Gefunden: C, 70,28; H, 7,98

**Beispiel 80**

Eine Mischung aus 2 g (4 mMol) rac-6-Acetyl-7-[5-(4-acetyl-3-hydroxy-2-propylphenoxy)pentyloxy]-3,4-dihydro-2H-1-benzopyran-2-carbonsäure und 155 ml Wasser wird mit 40 ml (4 mMol) 0,1N-wässrige N-triumhydroxidlösung versetzt. Die Mischung wird während 2 Stunden bei Raumtemperatur gerührt und dann genutscht. Das Filtrat wird gefriergetrocknet, worauf man den Festkörper in heissem Essigester, das wenig Äthanol enthält, aufnimmt. Nach Zugabe von Hexan erhält man einen Niederschlag. Man lässt die Mischung über Nacht bei 0° stehen und nutscht dann das ausgefallene Material ab. Der Festkörper wird aus Essigester/Hexan (2:1) zweimal umkristallisiert. Nach dem Trocknen im Hochvakuum bei 60° erhält man 1,6 g (77 %) des Mononatriumsalzes der eingesetz ten Säure als farblosen Festkörper.

Analyse für $C_{28}H_{33}NaO_8$ Berechnet: C, 64,61; H, 6,39; Na, 4,42 Gefunden: C, 64,50; H, 6,55; Na, 4,17.

**Beispiel 81**

Man versetzt eine Lösung von 2 g (4 mMol) rac-6-Acetyl-7-[5-(4-acetyl-3-hydroxy-2-propylphenoxy)pentyloxy]-3,4-dihydro-2H-1-benzopyran-2-carbonsäure in 15 ml Äthanol mit einer Lösung von 0,242 g (2 mMol) d-(+)-α-Methylbenzylamin in 4 ml Äther. Man verdünnt die erhaltene Lösung mit 10 ml Äther und lässt dann bei 0° stehen. Das ausgefallene Material wird abgenutscht und mit Äther gewaschen. Durch Umkristallisieren aus Äthanol erhält man 0,742 g (60 %) eines farblosen Festkörpers vom Schmelzpunkt 167-170°, der zur Hauptsache aus dem d-Aminsalz der (S)-Säure besteht. Dieses Salz wird in Essigester suspendiert und mit 1N-Salzsäure geschüttelt. Die organische Phase wird abgetrennt, mit Wasser gewaschen, über wasserfreiem Magnesiumsulfat getrocknet, filtriert und unter vermindertem Druck eingedampft. Man erhält 0,58 g (S)-6-Acetyl-7-[5-(4-acetyl-3-hydroxy-2-propylphenoxy)pentyloxy]-3,4-dihydro-2H-1-benzopyran-2-carbonäure als Festkörper. Eine Probe dieses Materials wird mit Diazomethan verestert. Der erhaltene Methylester wird mittels Flüssigkeitschromatographie an einer 30 cm-Prikle-Säule (kovalentes Phenylglycin) auf Enantiomeren hin untersucht. Die Analyse zeigt, dass die mit diesem Spaltungsverfahren erhaltene Säure aus 95,3 % des (S)-Enantiomeren (weniger polarer Methylester) und 4,7 % des (R)-Enantiomeren (stärker polarer Methylester) besteht.

**Beispiel 82**

Eine Mischung aus 6 g (27 mMol) rac-3,4-Dihydro-7-hydroxy-2H-1-benzopyran-2-carbonsäure-äthylester, 6,8 g (54 mMol) Benzylchlorid, 7,5 g (54 Mol) wasserfreiem Kaliumcarbonat und 4,5 g (27 mMol) Kaliumjodid in 100

ml Aceton wird unter Rühren und unter Rückfluss während 7 Stunden zum Sieden erhitzt. Der Grossteil des Acetons wird unter Vermindertem Druck entfernt, und der Rückstand wird mit Essigester verdünnt. Die organische Phase wird mit Wasser gewaschen, über wasserfreiem Magnesiumsulfat getrocknet, filtriert und unter vermindertem Druck eingedampft. Man erhält 17,4 g eines gelben Öls. Dieses Material wird an Kieselgel chromatographiert. Unter Eluieren mit Toluol/Essigester (19:1) erhält man 6,9 g (78 %) rac-3,4-Dihydro-7-(phenylmethoxy)-2H-1-benzopyran-2-carbonsäure-äthylester als gelbes Öl.

**Beispiel 83**

Man löst 6,9 g (22 mMol) des Produktes aus Beispiel 82 in 70 ml Toluol und kühlt die Lösung auf 78° ab (Trockeneis/Aceton-Bad). Unter Rühren werden 15,6 ml einer 25-proz. Lösung von Diisobutylaluminiumhydrid in Toluol tropfenweise zugegeben. Das Reaktionsgemisch wird während 2 Stunden bei -78° gerührt, worauf man zuerst vorsichtig 2,5 ml Methanol und dann 2N-Salzsäure und Eis dazugibt. Man extrahiert das Gemisch mit Essigester, wäscht die vereinigten organischen Auszüge mit gesättigter Kochsalzlösung, trocknet diese über wasserfreiem Magnesiumsulfat, filtriert und dampft unter vermindertem Druck ein. Der Rückstand (7,6 g) wird an Kieselgel chromatographiert Unter Eluieren mit Toluol/Essig ester (9:1) erhält man 5,6 g (70 %) rac-3,4-Dihydro-7-(phenylmethoxy)-2H-1-benzopyran-2-carboxaldehyd als blassgelbes Öl.

**Beispiel 84**

In Analogie zu Beispiel 30 werden 2,7 g (10 mMol) des Aldehydes aus Beispiel 83 mit 3,7 g (10,6 mMol) (Carbäthoxymethylen)triphenylphosphoran in 30 ml Toluol kondensiert (1,5 Stunden bei 100°). Nach Chromatographie des Rohproduktes an 50 g Kieselgel unter Eluieren mit Toluol erhält man 3,0 g (88,8%) rac-3,4-Dihydro-7-(phenylmethoxy)- 2H-1-benzopyran-2-propensäure-äthylester als blassgelbes Öl (Mischung der E- und Z-Isomeren).

**Beispiel 85**

3 g (8,9 mMol) des Propensäureesters aus Beispiel 84 werden bei Atmosphärendruck und Raumtemperatur über 0,3 g 10-proz. Palladium/Kohle in 35 ml Essigester zu 2,4 g rac-3-4-Dihydro-7-hydroxy-2H-1-benzopyran-2-propansäure-äthylester hydriert. Man erhält das gewünschte Produkt nach Abfiltrieren vom Katalysator und Eindampfen als viskoses Öl.

**Beispiel 86**

In Analogie zu Beispiel 41 wird der Propansäureester aus Beispiel 85 in 1,4 g (54 %) rac-6-Acetyl-3,4-dihydro-7-hydroxy-2H-1-benzopyran-2-propansäure-äthylester übergeführt. Nach Chromatographieren an Kieselgel unter Eluieren mit Toluol/Essigester (19:1) erhält man das gewünschte Produkt als gelbes Öl das beim Stehenlassen kristallisiert.

**Beispiel 87**

In Analogie zu Beispiel 65 wird das Produkt aus Beispiel 86 (1,4 g, 4,79 mMol) in rac-6-Acetyl-7-[(5-brom-pentyl)oxy]-3,4-dihydro-2H-1-benzopyran-2-propansäure-äthylester übergeführt. Nach Reinigung des Rohproduktes mittels Chromatographie an Kieselgel unter Eluieren mit Toluol/Essigester (19:1) erhält man das gewünschte Produkt in einer Ausbeute von 76 % (1,6 g) als gelbes Öl das beim Stehenlassen kristallisiert.

**Beispiel 88**

In Analogie zu Beispiel 67 wird der Bromester aus Beispiel 87 (1,6 g, 3,63 mMol) in rac-6-Acetyl-7-[(-5(-4-acetyl-3-hydroxy-2-propylphenoxy)pentyloxy]-3,4-dihydro-2H-1-benzopyran-2-propansäure-äthylester übergeführt, und zwar durch Alkylieren von 2'-4'-Dihydroxy-3-propylacetophenon. Nach Reinigung durch Chromatographieren an Kieselgel unter Eluieren mit Toluol/Essigester (9:1 und 4:1) erhält man das gewünschte

Produkt in einer Ausbeute von 79% (1,6 g) als blassgelbes Öl.

**Beispiel 89**

In Analogie zu Beispiel 69 wird das Produkt aus Beispiel 88 (1,6 g, 2,89 mMol) zu rac-6-Acetyl-7-[5-(4-acetyl-3-hydroxy-2-propylphenoxy)pentyloxy]-3,4-dihydro-2H-1-benzopyran-2-propansäure verseift. Nach Umkristallisieren aus Essigester/Hexan und Acetonitril erhält man das gewünschte Produkt in einer Ausbeute von 66 % (1 g) als farblosen Festkörper vom Schmelzpunkt 126,5-129°.

Analyse für $C_{30}H_{38}O_8$ Berechnet: C, 68,42; H, 7,27 Gefunden: C, 68,42; H, 7,49.

**Beispiel 90**

In Analogie zu Beispiel 30 werden 2,7 g (10 mMol) des Aldehydes aus Beispiel 83 mit 4,4 g (12 mMol) Äthyl-4-(triphenylphosphoranyliden)-2-butenoat in 30 ml Toluol kondensiert (3,5 Stunden bei 100°). Durch Chromatographieren des Rohproduktes an 100 g Kieselgel unter Eluieren mit Toluol und Toluol/Essigester (19:1) erhält man 1,1 g (30 %) rac-5-[3,4-dihydro-7-(phenylmethoxy)-2H-1-benzopyran-2-yl]-2,4-pentadiencarbonsäure-äthylester als gelbes Öl (Mischung der E und Z-Isomeren).

**Beispiel 91**

Das Produkt aus Beispiel 90 (1,1 g, 3,02 mMol) wird bei Atmosphärendruck- und Raumtemperatur in 15 ml Essigester über 0,95 g 10-proz. Palladium/Kohle hydriert. Der Katalysator wird abfiltriert und das Filtrat wird unter vermindertem Druck eingedampft. Man erhält 0,9 g (100 %) rac-3,4-Dihydro-7-hydroxy-2H-1-benzopyran-2-pentansäure-äthylester als blassgelbes Öl.

**Beispiel 92**

In Analogie zu Beispiel 41 wird das Produkt aus Beispiel 91 in rac-6-Acetyl-3,4-dihydro-7-hydroxy-2H-1-benzo pyran-2-pentansäure-äthylester (0,4 g, 41 %) übergeführt, das man als gelbes Öl erhält, das beim Stehenlassen kristallisiert.

**Beispiel 93**

In Analogie zu Beispiel 65 wird das Produkt aus Beispiel 92 (0,4 g, 1,25 mMol) in rac-6-Acetyl-7-[(5-brompentyl)oxy]-3,4-dihydro-2H-1-benzopyran-2-pentansäure-äthylester übergeführt. Nach Säulenchromatographie an Kieselgel unter Eluieren mit Toluol/Essigester (19:1) erhält man das gewünschte Produkt in einer Ausbeute von 51 % (0,3 g) als gelbes Öl.

**Beispiel 94**

In Analogie zu Beispiel 67 wird das Produkt aus Beispiel 93 (0,3 g, 0,64 mMol) in rac-6-Acetyl-7-[5-(4-acetyl-3-hydroxy-2-propylphenoxy)pentyloxy]-3,4-dihydro-2H-1-benzopyran-2-pentansäure-äthylester übergeführt, und zwar durch Alkylierung von 2',4'-Dihydroxy-3'-propylacetophenon. Nach Reinigung durch Chromatographie an Kieselgel unter Eluieren mit Toluol/Essigester (19:1) erhält man das gewünschte Produkt in einer Ausbeute von 80 % (0,3 g) als gelbes Öl.

**Beispiel 95**

In Analogie zu Beispiel 69 wird das Produkt aus Beispiel 94 (0,3 g, 0,51 mMol) zu rac-6-Acetyl-7-[5-(4-acetyl-3-hydroxy-2-propylphenoxy)pentyloxy]-3,4-dihydro-2H-1-benzopyran-2-pentansäure verseift. Man erhält das gewünschte Produkt in einer Ausbeute von 0,137 g (48 %) als farblosen Festkörper vom Schmelzpunkt 116-120,5° (aus Acetonitril).
Analyse für $C_{32}H_{42}O_8$ Berechnet: C, 69,29; N, 7,63 Gefunden: C, 69,35; H, 7,66.

**Beispiel A**

Rac-6-Acetyl-7-[3-(4-acetyl-3hydroxy-2-propylphenoxy)propoxy]-2-methyl-8-propyl-2H-1-benzopyran-2-propansäure kann wie folgt als Wirkstoff für die Herstellung von Kapseln verwendet werden:

| Bestandteile | mg/Kapsel | | | |
|---|---|---|---|---|
| Wirkstoff | 25 | 50 | 100 | 200 |
| Lactose | 375 | 155 | 200 | 140 |
| Stärke | 30 | 30 | 35 | 40 |
| Talk | 20 | 15 | 15 | 20 |
| Kapselfüllgewicht | 450 mg | 250 mg | 350 mg | 400 mg |

Verfahren:
Man mischt den Wirkstoff, die Lactose und die Stärke in einem geeigneten Mixer, mahlt, gibt den Talk hinzu, mischt gut und füllt auf einer geeigneten Apparatur in Kapseln ab

**Beispiel B**

Rac-7-[3-(4-Acetyl-3-hydroxy-2-propylphenoxy)propoxyl]-3,4-dihydro-2H-1-benzopyran-2-carbonsäure kann wie folgt als Wirkstoff für die Herstellung von Tabletten (Nassgranulie rung) verwendet werden:

| Bestandteile | mg/Tablette | | | |
|---|---|---|---|---|
| Wirkstoff | 25 | 50 | 100 | 200 |
| Lactose | 280 | 153 | 187 | 171 |
| Modifizierte Stärke | 55 | 25 | 35 | 45 |
| Vorgelatinisierte Stärke | 35 | 20 | 25 | 30 |
| Destilliertes Wasser q.s. | - | - | - | - |
| Magnesiumstearat | 5 | 2 | 3 | 4 |
| Tablettengewicht | 400 mg | 250 mg | 350 mg | 450 mg |

Verfahren:
Man mischt den Wirkstoff, die Lactose, die modifizierte Stärke und die vorgelatinisierte Stärke in einem geeigneten Mixer, granuliert mit genügend Wasser, um eine geeignete Konsistenz zu erhalten, mahlt, trocknet in einem geeigneten Ofen, mahlt und vermischt mit Magnesiumstearat während 3 Minuten und verpresst auf einer geeigneten Presse zu Tabletten entsprechender Grösse.

**Beispiel C**

Rac-6-Acetyl-7-[5-(4-acetyl-3-hydroxy-2-propylphenoxy)-pentyloxy]-3,4-dihydro-2H-1-benzopyran-2-carbonsäure kann wie folgt als Wirkstoff für die Herstellung von Tabletten (direkte Verpressung) verwendet werden:

| Bestandteile | mg/Tablette |
|---|---|
| Wirkstoff | 25 |
| Lactose | 181 |
| Avicel | 55 |
| Stärke für die direkte Verpressung | 35 |
| Magnesiumstearat | 4 |
| Tablettengewicht | 300 mg |

Verfahren:
Man vermischt den Wirkstoff mit einer äquivalenten Menge Lactose, mischt gut, mischt mit Cellulose und

der Stärke für die direkte Verpressung und den verbleibenden Teil an Lactose, mischt gut, fügt das Magnesiumstearat hinzu, mischt während 3 Minuten und verpresst auf einer geeigneten Presse zu Tabletten entsprechender Grösse.

**Beispiel D**

Rac-6-Acetyl-7-[5-(4-acetyl-3-hydroxy-2-propylphenoxy)-pentyloxy]-3,4-dihydro-2H-1-benzopyran-2-carbonsäure kann wie folgt als Wirkstoff für die Herstellung von Aerosolen (Freon-Suspension-Aerosol) verwendet werden:

| Bestandteile | mg/100 ml | | |
|---|---|---|---|
| Wirkstoff (mikronisiert) | 1 | 10 | 25 |
| Glyceryltrioleat | 0,03 | 3,0 | 7,5 |
| Freon 114*: 30 Teile auffüllen | | | |
| Freon 12 : 70 Teile auf | 100µl | 100µl | 100µl |

* Freon 11 kann auch Verwendet werden.

Verfahren:
Man mikronisiert rac-6-Acetyl-7-[5-(4-acetyl-3-hydroxy-2-propylphenoxy)pentyloxy]-3-3,4-dihydro-2H-1-benzopyran-2-carbonsäure mittels Druckluft zu 1-10 mikron grossen Partikeln, legt dieses Material in einem geeigneten Gefäss vor, versetzt mit dem Glyceryltrioleat bei Raumtemperatur, gibt auf -30° abgeschrecktes Freon dazu und verschliesst unmittelbar danach mit einem geeigneten, präzisen Volumetrischen Ventil. Der Behälter wird dann einer Ultraschallbehandlung ausgesetzt um den Wirkstoff zu dispergieren.

**Beispiel E**

Das Natriumsalz der rac-6-Acetyl-7-[5-(4-acetyl-3-hydroxy-2-propylphenoxy)pentyloxy]-3,4-dihydro-2H-1-benzopyran-2-carbonsäure kann wie folgt als Wirkstoff für die Herstellung von Aerosolen (Natrium-Freon-Suspension-Aerosol) verwendet werden:

| Bestandteile: | mg/100 ml | | |
|---|---|---|---|
| Wirkstoff (mikronisiert) | 1 | 10 | 25 |
| Oleinsäure | 0,01 zu<br>0,03 | 1,0 zu<br>3,0 | 3,0 zu<br>7,5 |
| Freon 114*: 30 Teile auffüllen | | | |
| Freon 12:70 Teile auf | 100 µl | 100 µl | 100 µl |

* Freon 11 kann auch Verwendet werden.

Verfahren:
Man mikronisiert rac-6-Acetyl-7-[5-(4-acetyl-3-hydroxy-2-propylphenoxy)pentyloxy]-3,4-dihydro-2H-1-benzopyran-2-carbonsäure mittels Druckluft zu 1-10 mikrongrossen Partikeln, legt dieses Material in einem geeigneten Gefäss vor, versetzt mit dem Glyceryltrioleat bei Raumtemperatur, gibt auf -30° abgeschrecktes Freon dazu und verschliesst unmittelbar danach mit einem geeigneten, präzisen Volumetrischen Ventil. Der Behälter wird dann einer Ultrawchallbehandlung ausgesetzt um den Wirkstoff zu dispergieren.

**Beispiel F**

Rac-6-Acetyl-7-[5-(4-acetyl-3-hydroxy-2-propylphenoxy)-pentyloxy]-3,4-dihydro-2H-1-benzopyran-2-carbonsäure kann wie folgt als Wirkstoff für die Herstellung von Aerosolen (Freon-Lösungs-Aerosol) verwendet werden:

Bestandteile

| | | | |
|---|---|---|---|
| Wirkstoff | 1,0 mg | 10,0 mg | 25,0 mg |
| Dimethylsulfoxid | 3,0 µl | 10,0 µl | 20,0 µl |
| Äthanol (99,9 %) | 6,0 µl | 6,0 µl | 6,0 µl |
| Methylenchlorid | 10 µl | 10 µl | 10 µl |
| Freon 12 : auffüllen auf | 100 µl | 100 µl | 100 µl |

Verfahren:
Man löst rac-Acetyl-7-[5-(4-acetyl-3-hydroxy-2-propyl-phenoxy)pentyloxy]-3,4-dihydro-2H-1-benzypyran-2-carbonsäure bei Raumtemperatur und in einem geeigneten Behälter in Dimethylsulfoxid, versetzt mit dem Äthanol und dem Methylenchlorid, gibt dann Freon dazu, das vorher auf -30° abgeschreckt worden ist, und verschliesst en Kontainer mit einem geeigneten, präzisen volumetrischen Ventil.

**Beispiel G**

Rac-6-Acetyl-7-[5-(4-acetyl-3-hydroxy-2-propylphenoxy)pentyloxy]-3,4-dihydro-2H-1-benzopyran-2-carbonsäuren kann wie folgt als Wirkstoff für die Herstellung von Aerosolen (Freon-Lösungs-Aerosol) verwendet werden:

Bestandteile

| | | |
|---|---|---|
| Wirkstoff | 50,0 | mg |
| Dimethylsulfoxid | 150 | µl |
| Äthanol (99,9 %) | 0,3 | ml |
| Methylenchlorid | 0,5 | ml |
| Freon 12 | 4,2 | ml |
| Total Volumen | 5,0 | ml |
| Wirkstoffkonzentration | 1 mg | /0,1 ml |

Verfahren:
Man verwendet das gleiche Verfahren wie für Beispiel F beschrieben.

**Beispiel H**

Rac-6-Acetyl-7-[5-(4-acetyl-3-hydroxy-2-propylphenoxy)pentyloxy]-3,4-dihydro-2H-1-benzypropan-2-carbonsäure kann wie folgt als Wirkstoff für die Herstellung von Sprühlösungen verwendet werden:

| Bestandteile | | mg/ml | |
|---|---|---|---|
| Wirkstoff | 25,0 | 50,0 | 100,0 |
| Phosphatpuffer, enthaltend Natriumhydroxid, vom pH 7,8: auffüllen auf | 1,0 ml | 1,0 ml | 1,0 ml |

Verfahren:
Man löst rac-6-Acetyl-7-[5-(4-acetyl-3-hydroxy-2-propylphenoxy)pentyloxy]-3,4-dihydro-2H-1-benzopyran-2-carbonsäure im Phosphatpuffer. Man erhält eine Lösung mit einem pH von 7,8.

**Patentansprüche** für die Vertragsstaten: BE, CH, DE, FR, GB, IT, LI, LU, NL, SE

1. Verbindungen der allgemeinem Formel

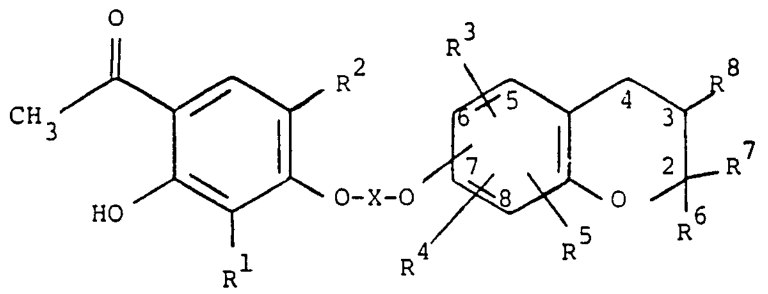

I

worin $R^1$ Wasserstoff oder niederes Alkyl, $R^2$ Wasserstoff oder Halogen und $R^3$, $R^4$ und $R^5$ unabhängig voneinander Wasserstoff, Acyl oder niederes Alkyl bedeuten, wobei höchstens einer von $R^3$, $R^4$ und $R^5$ Acyl bedeuten kann, und entweder $R^6$ und $R^7$ je Wasserstoff und $R^8$ die Gruppe -COOR$^9$ bedeuten, oder $R^6$ Wasserstoff oder niederes Alkyl, $R^7$ die Gruppe $-(CH_2)_n-COOR^9$, $R^8$ Wasserstoff, $R^9$ Wasserstoff oder niederes Alkyl, X $(C_{3-7})$Alkylen und n eine Zahl von 0-4 bedeuten, wobei "Acyl" niederes Alkanoyl oder Arylcarbonyl, "Aryl" gegebenenfalls durch Halogen, Trifluormethyl, niederes Alkyl, niederes Alkoxy, Nitro, Amino, niederes Acrylamino und di- niederes Alkylamino einfach oder mehrfach substituiertes Phenyl bedeuten und die als "neder" bezeichneten gruppen bis zu 7 Kohlenstoffatome aufweisen,

und, wenn $R^9$ Wasserstoff bedeutet, Salze davon mit pharmazeutisch annehmbaren Basen.

2. Verbindungen nach Anspruch 1, worin $R^6$ Wasserstoff oder niederes Alkyl, $R^7$ die Gruppe $-(CH_2)_n-COOR^9$ und $R^8$ Wasserstoff bedeuten und $R^9$ und n die in Anspruch 1 angegebene Bedeutung besitzen.

3. Verbindungen der allgemeinen Formel

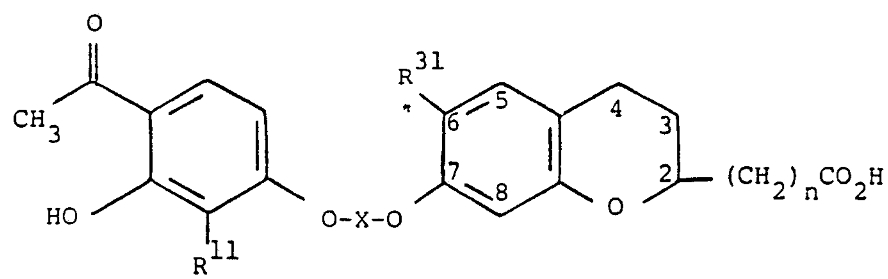

Ib

worin $R^{11}$ niederes Alkyl, $R^{31}$ Acyl, X $(C_{3-7})$- Alkylen und n eine Zahl von 0 bis 4 bedeuten.

4. Verbindungen nach Anspruch 2, worin $R^1$ niederes Alkyl, $R^2$ Wasserstoff, $R^3$ Acyl und $R^4$, $R^5$, $R^6$ und $R^9$ je Wasserstoff bedeuten.

5. Verbindungen nach Anspruch 4, worin X Alkylen mit 3-5 Kohlenstoffatomen bedeutet.

6. Verbindungen nach Anspruch 5, worin $R^3$ Acetyl bedeutet.

7. Verbindungen nach Anspruch 6, worin $R^1$ Propyl bedeutet.

8. Rac-6-Acetyl-7-[5-(4-acetyl-3-hydroxy-2-propyl-phenoxy)pentyloxy]-3,4-dihydro-2H-1-benzopyran-2-carbon-säure.

9. Rac-7-[3-(4-Acetyl-3-hydroxy-2-propylphenoxy)propoxy]-3,4-dihydro-2H-1-benzopyran-2-carbonsäure.

10. Rac-6-Acetyl-7-[3-(4-acetyl-3-hydroxy-2-propylphenoxy)propoxy]-2-methyl-8-propyl-2H-1-benzopyran-2-propansäure.

11. Rac-6-[3-(4-Acetyl-3-hydroxy-2-propylphenoxy)propoxy]-3,4-dihydro-2-methyl-2H-1-benzopyran-2-carbonsäure.

12. Rac-7-[3-(4-Acetyl-3-hydroxy-2-propylphenoxy)propoxy]-3,4-dihydro-8-proyl-2H-1-benzopyran-2-carbonsäure.

13. Rac-6-Acetyl-7-[3-(4-acetyl-3-hydroxy-2-propylphenoxy)propoxy]-3,4-dihydro-2H-benzopyran-2-carbonsäure.

14. Verbindungen der allgemeinen Formel

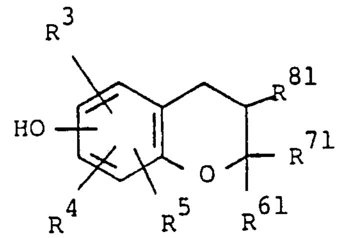

III

worin $R^3$, $R^4$ und $R^5$ unabhängig voneinander je Wasserstoff, Acyl oder niederes Alkyl bedeuten, wobei höchstens einer von $R^3$, $R^4$ und $R^5$ Acyl bedeuten kann, und entweder $R^{61}$ und $R^{71}$ beide Wasserstoff und $R^{81}$ die Gruppe $-COOR^{91}$ oder $R^{61}$ Wasserstoff oder niederes Alkyl, $R^{71}$ die Gruppe $(CH_2)_n-COOR^{91}$, $R^{81}$ Wasserstoff, $R^{91}$ niederes Alkyl, und n eine Zahl von 0-4 bedeuten, wobei "Acyl" gegebenenfalls durch Halogen, Trifluormethyl, niederes Alkyl, niederes Alkanoyl oder Arylcarbonyl, "Aryl" niederes Alkoxy, Nitro, Amino, niederes Alkylamino und di- niederes Alkylamino einfach oder mehrfach substituiertes Phenyl bedeuten und die als "nieder" bezeichneten gruppen bis 7 Kohlenstoffatome aufweisen.

15. Verbindungen der allgemeinen Formel

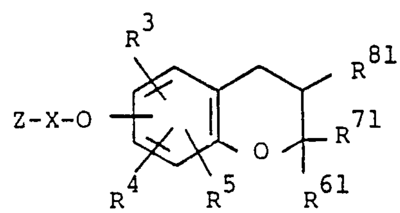

IV

worin $R^3$, $R^4$ und $R^5$ unabhängig voneinander Wasserstoff, Acyl oder niederes Alkyl bedeuten, wobei höchstens einer von $R^3$, $R^4$ und $R^5$ Acyl bedeuten kann, und entweder $R^{61}$ und $R^{71}$ beide Wasserstoff und $R^{81}$ die Gruppe $-COOR^{91}$ oder $R^{61}$ Wasserstoff oder niederes Alkyl, $R^{71}$ die Gruppe $-(CH_2)_n-COOR^{91}$, Wasserstoff, $R^{91}$ niederes Alkyl, X $(C_{3-7})$-Alkylen, Z ein Halogenatom und n eine Zahl von 0-4 bedeuten, wobei "Acyl" niederes Alkanoyl oder Arylcarbonyl, "Aryl" gegebenenfalls durch Halogen, Trifluormethyl, niederes Alkyl, niederes Alkoxy, Nitro, Amino, niederes Alkylamino und di- niederes Alkylamino einfach oder mehrfach substituiertes Phenyl bedeuten und die als "nieder" bezeichneten gruppen bis 7 Kohlenstoffatome aufweisen.

16. Verbindungen nach einem der Ansprüche 1-13 zur Anwendung als therapeutische Wirkstoffe.

17. Verbindungen nach einem der Ansprüche 1-13 zur Anwendung als antiallergische Wirkstoffe.

18. Rac-6-Acetyl-7-[5-(4-acetyl-3-hydroxy-2-propylphenoxy)pentyloxy]-3,4-dihydro-2H-1-benzopyran-2-carbonsäure zur Anwendung als therapeutischen Wirkstoff.

19. Rac-6-Acetyl-7-[5-(4-acetyl-3-hydroxy-2-propylphenoxy)pentyloxy]-3,4-dihydro-2H-1-benzopyran-2-carbonsäure zur Anwendung als antiallergischer Wirkstoff.

20. Verfahren zur Herstellung von Verbindungen nach einem der Ansprüche 1-13, dadurch gekennzeichnet, dass man

a) eine Verbindung der allgemeinen Formel

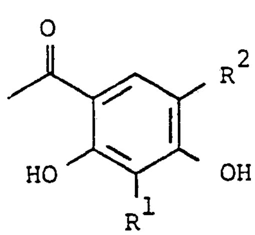

II

oder

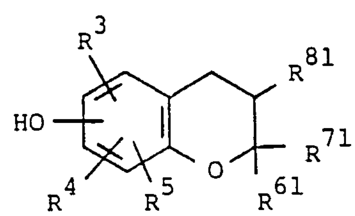

III

worin entweder $R^{61}$ und $R^{71}$ je Wasserstoff und $R^{81}$ die Gruppe $-COOR^{91}$ oder $R^{61}$ Wasserstoff oder niederes Alkyl, $R^{71}$ die Gruppe $-(CH_2)_n-COOR^{91}$, $R^{81}$ Wasserstoff, und $R^{91}$ niederes Alkyl bedeuten und $R^1$, $R^2$, $R^3$, $R^4$, $R^5$ und n obige Bedeutung besitzen, wobei "niederes Alkyl" bis zu 7 Kohlenstoffatome aufweist, mit einer Verbindung der allgemeinen Formel

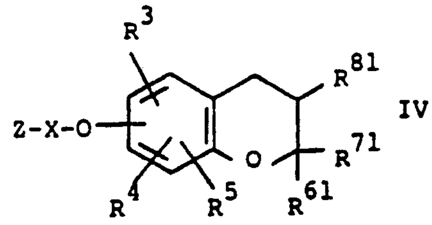

IV

und

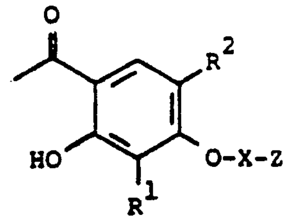

V

worin $R^1$, $R^2$, $R^3$, $R^4$. $R^5$, $R^{61}$, $R^{71}$, $R^{81}$ und X obige Bedeutung besitzen und Z ein Halogenatobedeutet, umsetzt, oder

b) die Estergruppe in einer Verbindung der allgemeinem Formel

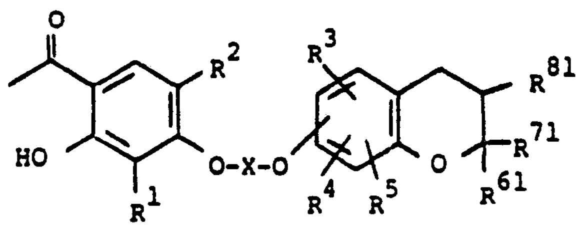

Ic

worin $R^1$, $R^2$, $R^3$, $R^4$, $R^5$, $R^{61}$, $R^{71}$, $R^{81}$ und X obige Bedeutung besitzen, hydrolysiert und erwünschtenfalls ein erhaltenes Racemat in die optisch aktiven Isomeren aufspaltet und/oder eine erhaltene Verbindung der Formel I, worin $R^9$ Wasserstoff bedeutet, mit einer Base in ein pharmazeutisch annehmbares Salz überführt.

21. Arzneimittel, enthaltend eine Verbindung nach einem der Ansprüche 1-13 und ein therapeutisch inertes Trägermaterial.

22. Antiallergische Mittel, enthaltend eine Verbindung nach einem der Ansprüche 1-13 und ein therapeutisch inertes Trägermaterial.

23. Arzneimittel enthaltend rac-6-Acetyl-7-[5-(4-acetyl-3-hydroxy-2-propylphenoxy)pentyloxy]-3,4-dihydro-2H-1Trägermaterial.

24. Antiallergisches Mittel, enthaltend rac-6-Acetyl-7-[5-(4-acetyl-3-hydroxy-2-propylphenoxy)pentyloxy]-3,4-dihydro-2H-1-benzopyran-2-carbonsäure und ein therapeutisch inertes Trägermaterial.

**Patentansprüche** für den Vertragstaat: AT

1. Verbindungen der allgemeinem Formel

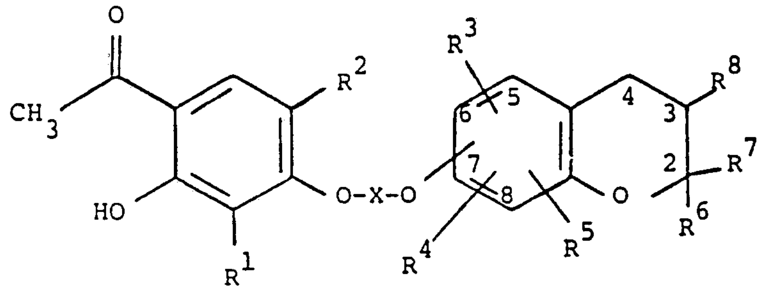

I

worin $R^1$ Wasserstoff oder niederes Alkyl, $R^2$ Wasserstoff oder Halogen und $R^3$, $R^4$ und $R^5$ unabhängig voneinander Wasserstoff, Acyl oder niederes Alkyl bedeuten, wobei höchstens einer von $R^3$, $R^4$ und $R^5$ Acyl bedeuten kann, und entweder $R^6$ und $R^7$ je Wasserstoff und $R^8$ die Gruppe $-COOR^9$ bedeuten, oder $R^6$ Wasserstoff oder niederes Alkyl, $R^7$ die Gruppe $-(CH_2)_n-COOR^9$, $R^8$ Wasserstoff, $R^9$ Wasserstoff oder niederes Alkyl, X $(C_{3-7})$-Alkylen und n eine Zahl von 0-4 bedeuten, wobei "Acyl" niederes Alkanoyl oder Arylcarbonyl, "Aryl" gegebenenfalls durch Halogen, Trifluormethyl, niederes Alkyl, niederes Alkoxy, Nitro, Amino, niederes Acrylamino und di- niederes Alkylamino einfach oder mehrfach substituiertes Phenyl bedeuten und die als "neder" bezeichneten gruppen bis zu 7 Kohlenstoffatome aufweisen,

und, wenn $R^9$ Wasserstoff bedeutet, Salze davon mit pharmazeutisch annehmbaren Basen.

2. Verbindungen nach Anspruch 1, worin $R^6$ Wasserstoff oder niederes Alkyl, $R^7$ die Gruppe $-(CH_2)_n-COOR^9$

und $R^8$ Wasserstoff bedeuten und $R^9$ und n die in Anspruch 1 angegebene Bedeutung besitzen.

3. Verbindungen der allgemeinen Formel

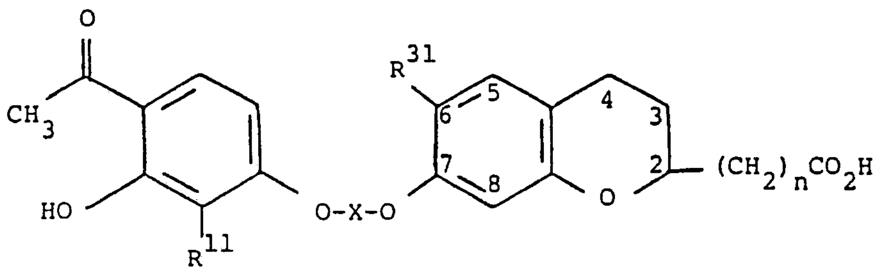

Ib

worin $R^{11}$ niederes Alkyl, $R^{31}$ Acyl, X $(C_{3-7})$- Alkylen und n eine Zahl von 0 bis 4 bedeuten.

4. Verbindungen nach Anspruch 2, worin $R^1$ niederes Alkyl, $R^2$ Wasserstoff, $R^3$ Acyl und $R^4$, $R^5$, $R^6$ und $R^9$ je Wasserstoff bedeuten.

5. Verbindungen nach Anspruch 4, worin X Alkylen mit 3-5 Kohlenstoffatomen bedeutet.

6. Verbindungen nach Anspruch 5, worin $R^3$ Acetyl bedeutet.

7. Verbindungen nach Anspruch 6, worin $R^1$ Propyl bedeutet.

8. Rac-6-Acetyl-7[-[5-(4-acetyl-3-hydroxy-2-propylphenoxy)pentyloxy]-3,4-dihydro-2H-1-benzopyran-2-carbon-säure.

9. Rac-7-[3-(4-Acetyl-3-hydroxy-2-propylphenoxy)propoxy]-3,4-dihydro-2H-1-benzopyran-2-carbonsäure.

10. Rac-6-Acetyl-7-[3-(4-acetyl-3-hydroxy-2-propyl-phenoxy)propoxy]-2-methyl-8-propyl-2H-1-benzopyran-2-propansäure.

11. Rac-6-[3-(4-Acetyl-3-hydroxy-2-propylphenoxy)propoxy]-3,4-dihydro-2-methyl-2H-1-benzopyran-2-carbonsäure.

12. Rac-7-[3-(4-Acetyl-3-hydroxy-2-propylphenoxy)propoxy]-3,4-dihydro-8-proyl-2H-1-benzopyran-2-carbonsäure.

13. Rac-6-Acetyl-7-[3-(4-acetyl-3-hydroxy-2-propylphenoxy)propoxy]-3,4-dihydro-2H-benzopyran-2-carbonsäure.

14. Verbindungen der allgemeinen Formel

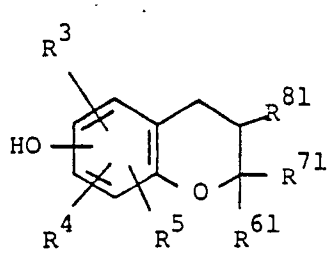

III

worin $R^3$, $R^4$ und $R^5$ unabhängig voneinander je Wasserstoff, Acyl oder niederes Alkyl bedeuten, wobei höchstens einer von $R^3$, $R^4$ und $R^5$ Acyl bedeuten kann, und entweder $R^{61}$ und $R^{71}$ beide Wasserstoff und $R^{81}$ die Gruppe -COOR$^{91}$ oder $R^{61}$ Wasserstoff oder niederes Alkyl, $R^{71}$ die Gruppe $(CH_2)_n$ COOR$^{91}$, $R^{81}$ Wasserstoff, $R^{91}$ niederes Alkyl, und n eine Zahl von 0-4 bedeuten, wobei "Acyl" niederes Alkanoyl oder Arylcarbonyl, "Aryl" gegebenenfalls duch Halogen, Trifluormethyl, niederes Alkyl, niederes Alkoxy, Nitro, Amino, niederes Alkylamino und di-nieders Alkylamino einfach oder mehrfach substituiertes Phenyl bedeuten und die als "nieder" bezeichneten gruppen bis 7 Kohlenstoffatome aufweisen.

15. Verbindungen der allgemeinen Formel

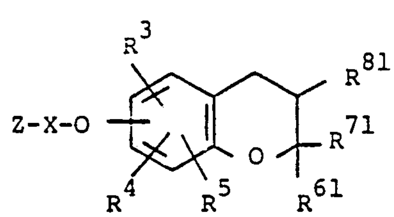

IV

worin $R^3$, $R^4$ und $R^5$ unabhängig voneinander Wasserstoff, Acyl oder niederes Alkyl bedeuten, wobei höchstens einer von $R^3$, $R^4$ und $R^5$ Acyl bedeuten kann, und entweder $R^{61}$ und $R^{71}$ beide Wasserstoff und $R^{81}$ die Gruppe $-COOR^{91}$ oder $R^{61}$ Wasserstoff oder niederes Alkyl, $R^{71}$ die Gruppe $-(CH_2)_n-COOR^{91}$, Wasserstoff, $R^{91}$ niederes Alkyl, X $(C_{3-7})$-Alkylen, Z ein Halogenatom und n eine Zahl von 0-4 bedeuten, wobei "Acyl" niederes Alkanoyl oder Arylcarbonyl, "Aryl" gegebenenfalls durch Halogen, Trifluormethyl, niederes Aklyl, niederes Alkoxy, Nitro, Amino, niederes Alkylamino und di-nieders Alkylamino einfach oder mehrfach substituiertes Phenyl bedeuten und die als "nieder" bezeichneten gruppen bis 7 Kohlenstoffatome aufweisen.

16. Verbindungen nach einem der Ansprüche 1-13 zur Anwendung als therapeutische Wirkstoffe.

17. Verbindungen nach einem der Ansprüche 1-13 zur Anwendung als antiallergische Wirkstoffe.

18. Rac-6-Acetyl-7-[5-(4-acetyl-3-hydroxy-2-propylphenoxy)pentyloxy]-3,4-dihydro-2H-1-benzopyran-2-carbonsäure zur Anwendung als therapeutischen Wirkstoff.

19. Rac-6-Acetyl-7-[5-(4-acetyl-3-hydroxy-2-propylphenoxy)pentyloxy]-3,4-dihydro-2H-1-benzopyran-2-carbonsäure zur Anwendung als antiallergischer Wirkstoff.

20. Arzneimittel, enthaltend eine Verbindung nach einem der Ansprüche 1-13 und ein therapeutisch inertes Träger material.

21. Antiallergische Mittel, enthaltend eine Verbindung nach einem der Ansprüche 1-13 und ein therapeutisch inertes Trägermaterial.

22. Arzneimittel enthaltend rac-6-Acetyl-7-[-5(4-actyl-3-hydroxy-2-propylphenoxy)pentyloxy]-3,4-dihydro-2H-1-benzopyran-2-carbonsäure und ein therapeutisch inertes Trägermaterial.

23. Antiallergisches Mittel, enthaltend rac-6-Acetyl-7-[5-(4-acetyl-3-hydroxy-2-propylphenoxy)pentyloxy]-3,4-dihydro-2H-1-benzopyran-2-carbonsäure und ein therapeutisch inertes Trägermaterial.

24. Verfahren zur Herstellung von Verbindungen der allgemeinen Formel

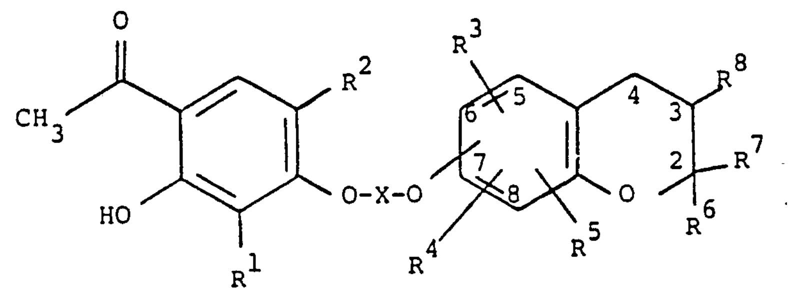

I

worin $R^1$ Wasserstoff oder niederes Alkyl, $R^2$ Wasserstoff oder Halogen und $R^3$, $R^4$ und $R^5$ unabhängig voneinander Wasserstoff, Acyl oder niederes Alkyl bedeuten, wobei höchstens einer von $R^3$, $R^4$ und $R^5$ Acyl bedeutet, und entweder $R^6$ und $R^7$ je Wasserstoff und $R^8$ die Gruppe $-COOR^9$ bedeuten, oder $R^6$ Wasserstoff oder niederes Alkyl, $R^7$ die Gruppe $-(CH_2)_n-COOR^9$, Wasserstoff, $R^9$ Wasserstoff oder niederes Alkyl, X $(C_{3-7})$Alkylen und n eine Zahl von 0-4 bedeuten, wobei "Acyl" niederes Alkanoyl oder Arylcarbonyl, "Aryl" gegebenenfalls durch Halogen, Triflouromethyl, niederes Alkyl, niederes Alkony, Nitro, Amino, niederes Alkylamino und di-nieders Alkylamino einfach oder mehrfach substituiertes Phenyl bedeuten und die als "Nieder" bezeichneten Gruppen bis zu 7 Kohlenstoffatome aufweisen,

und, wenn $R^9$ Wasserstoff bedeutet, Salzen davon mit pharmazeutisch annehmbaren Basen, dadurch gekennzeichnet, dass man

a) eine Verbindung der allgemeinen Formel

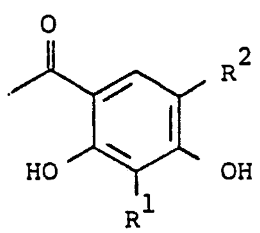

II

oder

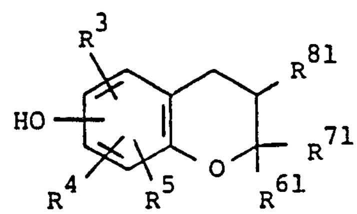

III

worin und $R^{61}$ und $R^{71}$ je Wasserstoff und $R^{81}$ die Gruppe $-COOR^{91}$ oder $R^{61}$ Wasserstoff oder niederes Alkyl, $R^{71}$ die Gruppe $-(CH_2)_n-COOR^{91}$, $R^{81}$ Wasserstoff, und $R^{91}$ niederes Alkyl bedeuten und $R^1$, $R^2$, $R^3$, $R^4$, $R^5$ und n obige Bedeutung besitzen, mit einer Verbindung der allgemeinen Formel

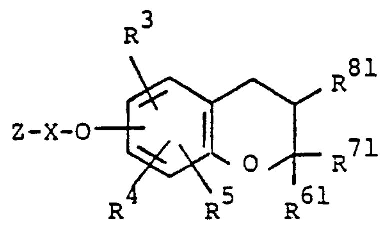

IV

und

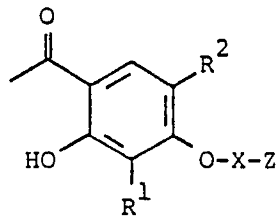

V

worin $R^1$, $R^2$, $R^3$, $R^4$, $R^5$, $R^{61}$, $R^{71}$, $R^{81}$ und X obige Bedeutung besitzen und Z ein Halogenatom bedeutet, umsetzt, oder

b) die Estergruppe in einer Verbindung der allgemeinen Formel

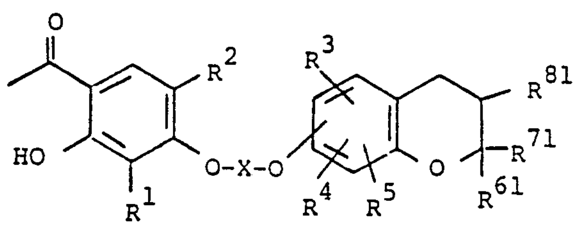

Ic

worin $R^1$, $R^2$, $R^3$, $R^4$, $R^5$, $R^{61}$, $R^{71}$, $R^{81}$ und X obige Bedeutung besitzen, hydrolysiert und erwünschtenfalls ein erhaltenes Racemat in die optisch aktiven Isomeren aufspaltet und/oder eine erhaltene Verbindung der Formel I, worin $R^9$ Wasserstoff bedeutet, mit einer Base in ein pharmazeutisch annehmbares Salz überführt.

25. Verfahren nach Anspruch 24, worin $R^6$ Wasserstoff oder niederes Alkyl, $R^7$ die Gruppe $-(CH_2)_n-COOR^9$ und $R^8$ Wasserstoff bedeuten, und und n die in Anspruchs angegebene Bedeutung besitzen.

26. Verfahren nach Anspruch 24, dadurch gekennzeichnet, dass man Verbindungen der allgemeinen Formel

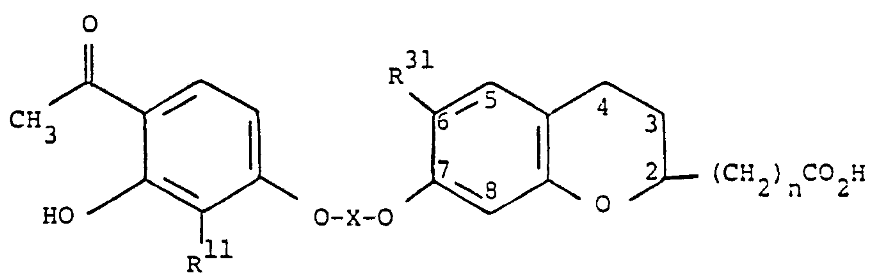

Ib

worin $R^{11}$ niederes Alkyl, $R^{31}$ Acyl, X $(C_{3-7})$-Alkylen und n eine Zahl von 0-4 bedeuten, herstellt.

27. Verfahren nach Anspruch 25, worin $R^1$ niederes Alkyl, $R^2$ Wasserstoff, $R^3$ Acyl und $R^4$, $R^5$, $R^6$ und $R^9$ je Wasserstoff bedeuten.

28. Verfahren nach Anspruch 27, dadurch gekennzeichnet, dass X Alkylen mit 3-5 Kohlenstoffatomen bedeutet.

29. Verfahren nach Anspruch 28, worin $R^3$ Acetyl bedeutet.

30. Verfahren nach Anspruch 29, worin $R^1$ Propyl bedeutet.

31. Verfahren nach Anspruch 24, dadurch gekennzeichnet, dass man rac-6-Acetyl-7-[-5-(4-acetyl-3-hydroxy-2-propylphenoxy)pentyloxy]-3,4-dihydro-2H-1-benzopyran-carbonsäure herstellt.

32. Verfahren nach Anspruch 24, dadurch gekennzeichnet, dass man rac-7-[-3-(4-Acetyl-3-hydroxy-2-propylphenoxy)propoxy]-3,4-dihydro-2H-1-benzopyran-2-carbonsäure herstellt.

33. Verfahren nach Anspruch 24, dadurch gekennzeichnet, dass man rac-6-Acetyl-7-[-3-(4-acetyl-3-hydroxy-propylphenoxy)propoxy]-2-methyl-8-propyl-2H-1-benzopyran-2-propansäure herstellt.

34. Verfahren nach Anspruch 24, dadurch gekennzeichnet, dass man rac-6-[3-(4-Acetyl-3-hydroxy-2-propylphenoxy)propoxy]-3,4-dihydro-2-methyl-2H-1-benzopyran-2-carbonsäure herstellt.

35. Verfahren nach Anspruch 24, dadurch gekennzeichnet, dass man rac-6-7-[3-(4-Acetyl-3-hydroxy-2-propylphenoxy)propoxy]-3,4-dihydro-8-propyl-2H-1-benzopyran-2-carbonsäure herstellt.

36. Verfahren nach Anspruch 24, dadurch gekennzeichnet, dass man rac-6-Acetyl-7-[3-(4-acetyl-3-hydroxy-2-propylphenoxy)propoxy]-3,4-dihydro-2H-benzopyran-2-carbonsäure herstellt.

**Claims** (for the Contracting States BE, CH, DE, FR, GB, IT, LI, LU, NL, SE

1. Compounds of the general formula

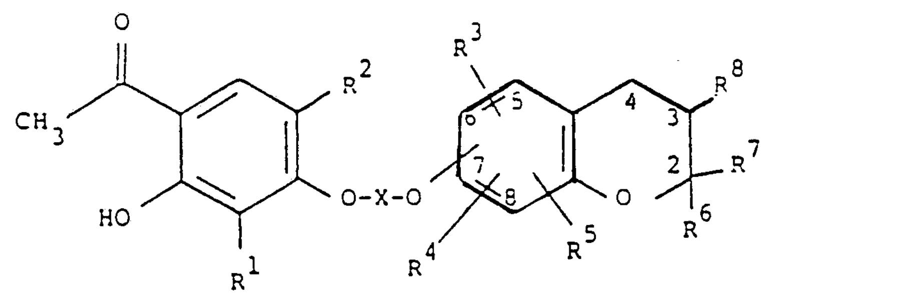

I

wherein $R^1$ signifies hydrogen or lower alkyl, $R^2$ signifies hydrogen or halogen and $R^3$, $R^4$ and $R^5$ each independently signify hydrogen, acyl or lover alkyl, whereby not more than one of $R^3$, $R^4$ and $R^5$ can signify acyl, and either $R^6$ and $R^7$ each signify hydrogen and $R^8$ signifies the group $-COOR^9$, or $R^6$ signifies hydrogen or lower alkyl. $R^7$ signifies the group $-(CH_2)_n-COOR^9$, $R^8$ signifies hydrogen, $R^9$ signifies hydrogen or lover alkyl, X signifies $(C_{3-7})$-alkylene and n signifies a number of 0-4, whereby "acyl" signifies lower alkanoyl or arylcarbonyl, "aryl" signifies phenyl optionally singly or multiply substituted by halogen, trifluoromethyl, lower alkyl, lower alkoxy, nitro, amino, lower alkylamino and di-lower alkylamino and the groups denoted as "lower" have up to 7 carbon atoms.

and, when $R^9$ signifies hydrogen, salts thereof with pharmaceutically acceptable bases.

2. Compounds according to claim 1, wherein $R^6$ signifies hydrogen or lower alkyl, $R^7$ signifies the group $-(CH_2)_n-COOR^9$ and $R^8$ signifies hydrogen and $R^9$ and n have the significance given in claim 1.

3. Compounds of the general formula

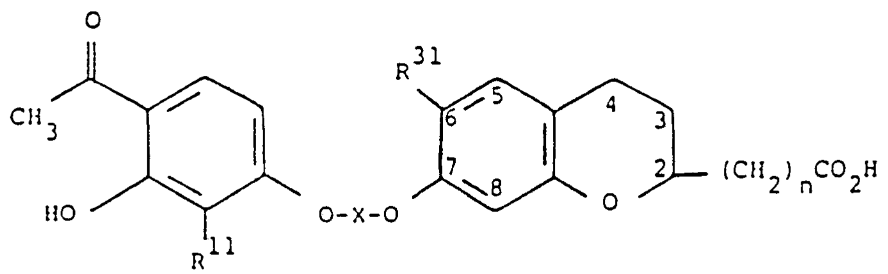

Ib

wherein $R^{11}$ signifies lower alkyl, $R^{31}$ signifies alkyl, X signifies $(C_{3-7})$-alkylene and n signifies a number of 0 to 4.

4. Compounds according to claim 2, wherein $R^1$ signifies lower alkyl, $R^2$ signifies hydrogen, $R^3$ signifies acyl and $R^4$, $R^5$, $R^6$ and $R^9$ each signify hydrogen.

5. Compounds according to claim 4, wherein X signifies alkylene with 3-5 carbon atoms.

6. Compounds according to claim 5, wherein $R^3$ signifies acetyl.

7. Compounds according to claim 6. wherein $R^1$ signifies propyl.

8. rac-6-Acetyl-7-[5-(4-acetyl-3-hydroxy-propylphenoxy)pentyloxy]-3,4-dihydro-2H-1-benzopyran-2-carbocylic acid.

9. rac-7-[3-(4-Acetyl-3-hydroxy-2-propylphenoxy)propoxy]-3,4-dihydro-2H-1-benzopyran-2-carboxylic acid.

10. rac-6-Acetyl-7-[3-(4-acetyl-3-hydroxy-2-propylphenoxy)propoxy]-2-methyl-8-propyl-2H-1-benzopyran-2-propanoic acid.

11. rac-6-[3-(4-Acetyl-3-hydroxy-2-propylphenoxy)propoxy]-3,4-dihydro-2-methyl-2H-1-benzopyran-2-carboxylic acid.

12. rac-7-[3-(4-Acetyl-3-hydroxy-2-propylphenoxy)propoxy]-3,4-dihydro-8-propyl-2H-1-benzopyran-2-carboxylic acid.

13. rac-6-Acetyl-7-[3-(4-acetyl-3-hydroxy-2-propylphenoxy)propoxy]-3,4-dihydro-2H-1-benzopyran-2-carboxylic acid.

14. Compounds of the general formula

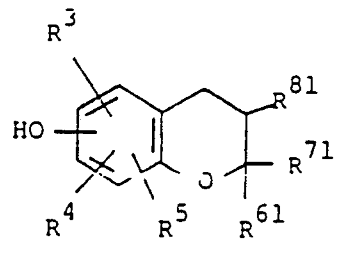

III

wherein $R^3$, $R^4$ and $R^5$ each independently signify hydrogen, acyl or lower alkyl, whereby not more than one of $R^3$, $R^4$ and $R^5$ can signify acyl, and either $R^{61}$ and $R^{71}$ both signify hydrogen and $R^{81}$ signifies the group $-COOR^{91}$, or $R^{61}$ signifies hydrogen or lower alkyl, $R^{71}$ signifies the group $-(CH_2)_n-COOR^{91}$, $R^{81}$ signifies hydrogen, $R^{91}$ signifies lower alkyl and n signifies a number of 0-4, whereby "acyl" signifies lower alkanoyl or arylcarbonyl, "aryl" signifies phenyl optionally singly or multiply substituted by halogen, trifluoromethyl, lower alkyl, lower alkoxy, nitro, amino, lower alkylamino and di-lower alkylamino and the groups denoted as "lower" have up to 7 carbon atoms.

15. Compounds of the general formula

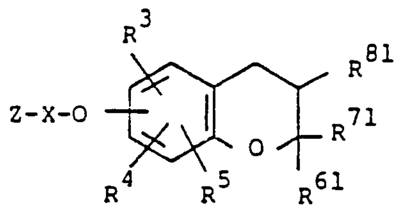

IV

wherein $R^3$, $R^4$ and $R^5$ each independently signify hydrogen, acyl or lower alkyl, whereby not more than one of $R^3$, $R^4$ and $R^5$ can signify acyl, and either $R^{61}$ and $R^{71}$ both signify hydrogen and $R^{81}$ signifies the group $-COOR^{91}$ or $R^{61}$ signifies hydrogen or lower alkyl, $R^{71}$ signifies the group $-(CH_2)_n-COOR^{91}$, $R^{81}$ signifies hydrogen, $R^{91}$ signifies lower alkyl, X signifies $(C_{3-7})$-alkylene, Z signifies a halogen atom and n signifies a number of 0-4, whereby "acyl" signifies lower alkanoyl or arylcarbonyl, "aryl" signifies phenyl optionally singly or multiply substituted by halogen, trifluoromethyl, lower alkyl, lower alkoxy, nitro, amino, lower alkylamino and di-lower alkylamino and the groups denoted as "lower" have up to 7 carbon atoms.

16. Compounds according to any one of claims 1-13 for use as therapeutically active substances.

17. Compounds according to any one of claims 1-13 for use as antiallergic active substances.

18. rac-6-Acetyl-7-[5-(4-acetyl-3-hydroxy-2-propylphenoxy)pentyloxy]-3,4-dihydro-2H-1-benzopyran-2-carboxylic acid for use as a therapeutically active substance.

19. rac-6-Acetyl-7-[5-(4-acetyl-3-hydroxy-2-propylphenoxy)pentyloxy]-3,4-dihydro-2H-1-benzopyran-2-carboxylic acid for use as an antiallergic active substance.

20. A process for the manufacture of compounds according to any one of claims 1-13, characterized by

a) reacting a compound of the general formula

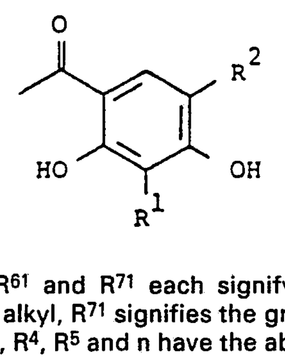

II or

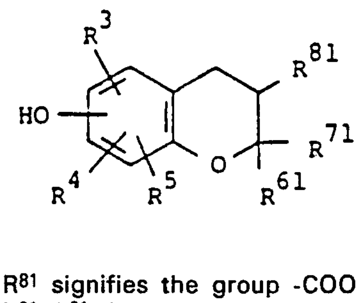

III

wherein either $R^{61}$ and $R^{71}$ each signify hydrogen and $R^{81}$ signifies the group $-COOR^{91}$ or $R^{61}$ signifies hydrogen or lower alkyl, $R^{71}$ signifies the group $-(CH_2)_n-COOR^{91}$, $R^{81}$ signifies hydrogen, and $R^{91}$ signifies lower alkyl and $R^1$, $R^2$, $R^3$, $R^4$, $R^5$ and n have the above significance, whereby "lower alkyl" has up to 7 carbon atoms,

with a compound of the general formula

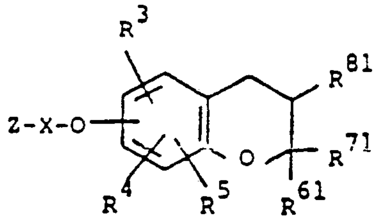

IV and

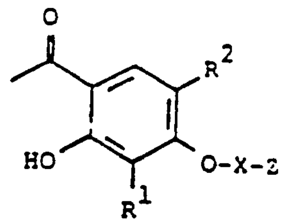

V

wherein $R^1$, $R^2$, $R^3$, $R^4$, $R^5$, $R^{61}$, $R^{71}$, $R^{81}$ and X have the above significance and Z signifies a halogen atom, or

b) hydrolyzing the ester group in a compound of the general formula

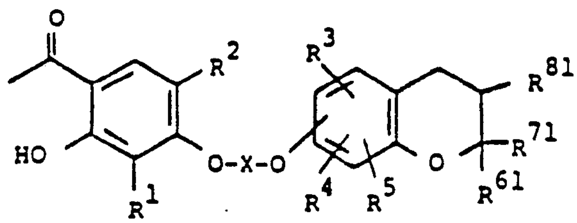

Ic

wherein $R^1$, $R^2$, $R^3$ , $R^4$. $R^5$, $R^{61}$, $R^{71}$, $R^{81}$ and X have the above significance, and, if desired, resolving a racemate obtained into the optically active isomers and/or converting a compound of formula I obtained in which $R^9$ signifies hydrogen with a base into a pharmaceutically acceptable salt.

21. A medicament containing a compound according to any one of claims 1-13 and a therapeutically inert carrier material.

22. An antiallergic medicament containing a compound according to any one of claims 1-13 and a therapeutically inert carrier material.

23. A medicament containing rac-6-acetyl-7-[5-(4-acetyl-3-hydroxy-2-propylphenoxy)pentyloxy]-3,4-dihydro-2H-1-benzopyran-2-carboxylic acid and a therapeutically inert carrier material.

24. An antiallergic medicament containing rac-6-acetyl-7-[5-]4-acetyl-3-hydroxy-2-propylphenoxy)pentyloxy]-3,4-dihydro-2H-1-benzopyran-2-carboxylic acid and a therapeutically inert carrier material.

**Claims** for the Contracting State: AT

1. Compounds of the general formula

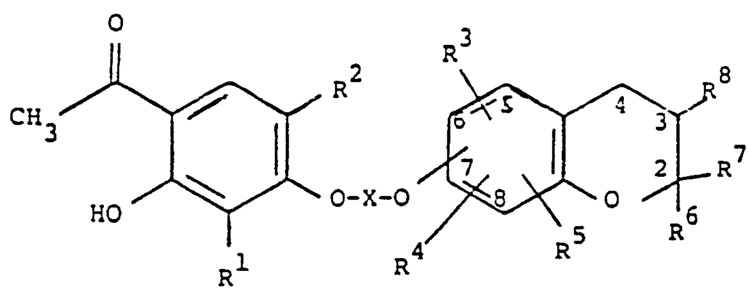

I

wherein $R^1$ signifies hydrogen or lower alkyl, $R^2$ signifies hydrogen or halogen and $R^3$, $R^4$ and $R^5$ each independently signify hydrogen, acyl or lover alkyl, whereby not more than one of $R^3$, $R^4$ and $R^5$ can signify acyl, and either $R^6$ and $R^7$ each signify hydrogen and $R^8$ signifies the group $-COOR^9$, or $R^6$ signifies hydrogen or lower alkyl. $R^7$ signifies the group $-(CH_2)_n-COOR^9$, $R^8$ signifies hydrogen, $R^9$ signifies hydrogen or lover alkyl, X signifies $(C_{3-7})$-alkylene and n signifies a number of 0-4, whereby "acyl" signifies lower alkanoyl or arylcarbonyl, "aryl" signifies phenyl optionally singly or multiply substituted by halogen, trifluoromethyl, lower alkyl, lower alkoxy, nitro, amino, lower alkylamino and di-lower alkylamino and the groups denoted as "lower" have up to 7 carbon atoms.

and, when $R^9$ signifies hydrogen, salts thereof with pharmaceutically acceptable bases.

2. Compounds according to claim 1, wherein $R^6$ signifies hydrogen or lower alkyl, $R^7$ signifies the group $-(CH_2)_n-COOR^9$ and $R^8$ signifies hydrogen and $R^9$ and n have the significance given in claim 1.

3. Compounds of the general formula

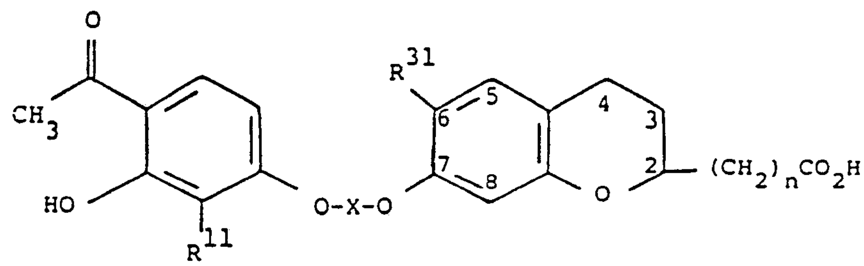

Ib

wherein $R^{11}$ signifies lower alkyl, $R^{31}$ signifies alkyl, X signifies $(C_{3-7})$-alkylene and n signifies a number of 0 to 4.

4. Compounds according to claim 2, wherein $R^1$ signifies lower alkyl, $R^2$ signifies hydrogen, $R^3$ signifies acyl and $R^4$, $R^5$, $R^6$ and $R^9$ each signify hydrogen.

5. Compounds according to claim 4, wherein X signifies alkylene with 3-5 carbon atoms.

6. Compounds according to claim 5, wherein $R^3$ signifies acetyl.

7. Compounds according to claim 6, wherein $R^1$ signifies propyl.

8. rac-6-Acetyl-7-[5-(4-acetyl-3-hydroxy-propyphenoxy)pentyloxy]-3,4-dihydro-2H-1-benzopyran-2-carbocylic acid.

9. rac-7-[3-(4-Acetyl-3-hydroxy-2-propylphenoxy)propoxy]-3,4-dihydro-2H-1-benzopyran-2-carboxylic acid.

10. rac-6-Acetyl-7-[3-(4-acetyl-3-hydroxy-2-propylphenoxy)propoxy]-2-methyl-8-propyl-2H-1-benzopyran-2-propanoic acid.

11. rac-6-[3-(4-Acetyl-3-hydroxy-2-propylphenoxy)propoxy]-3,4-dihydro-2-methyl-2H-1-benzopyran-2-carboxylic acid.

12. rac-7-[3-(4-Acetyl-3-hydroxy-2-propylphenoxy)propoxy]-3,4-dihydro-8-propy]-2H-1-benzopyran-2-carboxylic acid.

13. rac-6-Acetyl-7-[3-(4-acetyl-3-hydroxy-2-propylphenoxy)propoxy]-3,4-dihydro-2H-1-benzopyran-2-carboxylic acid.

14. Compounds of the general formula

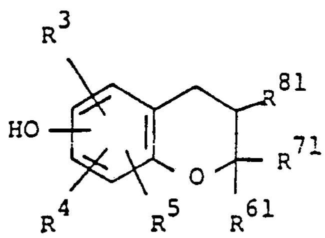

III

wherein $R^3$, $R^4$ and $R^5$ each independently signify hydrogen, acyl or lower alkyl, whereby not more than one of $R^3$, $R^4$ and $R^5$ can signify acyl, and either $R^{61}$ and $R^{71}$ both signify hydrogen and $R^{81}$ signifies the group $-COOR^{91}$, or $R^{61}$ signifies hydrogen or lower alkyl, $R^{71}$ signifies the group $-(CH_2)_n-COOR^{91}$, $R^{81}$ signifies hydrogen, $R^{91}$ signifies lower alkyl and n signifies a number of 0-4, whereby "acyl" signifies lower alkanoyl or arylcarbonyl, "aryl" signifies phenyl optionally singly or multiply substituted by halogen, trifluoromethyl, lower alkyl, lower alkoxy, nitro, amino, lower alkylamino and di-lower alkylamino and the groups denoted as "lower" have up to 7 carbon atoms.

15. Compounds of the general formula

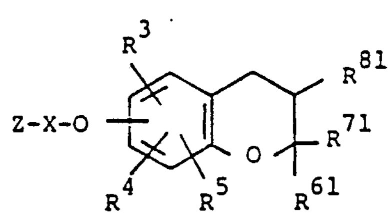

IV

wherein $R^3$, $R^4$ and $R^5$ each independently signify hydrogen, acyl or lower alkyl, whereby not more than one of $R^3$, $R^4$ and $R^5$ can signify acyl, and either $R^{61}$ and $R^{71}$ both signify hydrogen and $R^{81}$ signifies the group $-COOR^{91}$ or $R^{61}$ signifies hydrogen or lower alkyl, $R^{71}$ signifies the group $-(CH_2)_n-COOR^{91}$, $R^{81}$ signifies hydrogen, $R^{91}$ signifies lower alkyl, X signifies $(C_{3-7})$-alkylene, Z signifies a halogen atom and n signifies a

number of 0-4, whereby "acyl" signifies lower alkanoyl or arylcarbonyl, "aryl" signifies phenyl optionally singly or multiply substituted by halogen, trifluoromethyl, lower alkyl, lower alkoxy, nitro, amino, lower alkylamino and di-lower alkylamino and the groups denoted as "lower" have up to 7 carbon atoms.

16. Compounds according to any one of claims 1-13 for use as therapeutically active substances.

17. Compounds according to any one of claims 1-13 for use as antiallergic active substances.

18. rac-6-Acetyl-7-[5-(4-acetyl-3-hydroxy-2-propylphenoxy)pentyloxy]-3,4-dihydro-2H-1-benzopyran-2-carboxylic acid for use as a therapeutically active substance.

19. rac-6-Acetyl-7-[5-(4-acetyl-3-hydroxy-2-propylphenoxy)pentyloxy]-3,4-dihydro-2H-1-benzopyran-2-carboxylic acid for use as an antiallergic active substance.

20. A medicament containing a compound according to any one of claims 1-13 and a therapeutically inert carrier material.

21. An antiallergic medicament containing a compound according to any one of claims 1-13 and a therapeutically inert carrier material.

22. A medicament containing rac-6-acetyl-7-[5-(4-acetyl-3-hydroxy-2-propylphenoxy)pentyloxy]-3,4-dihydro--2H-1-benzopyran-2-carboxylic acid and a therapeutically inert carrier material.

23. An antiallergic medicament containing rac-6-acetyl-7-[5-(4-acetyl-3-hydroxy-2-propylphenoxy)pentyloxy]-3,4-dihydro-2H-1-benzopyran-2-carboxylic acid and a therapeutically inert carrier material.

24. A process for the manufacture of compounds of the general formula

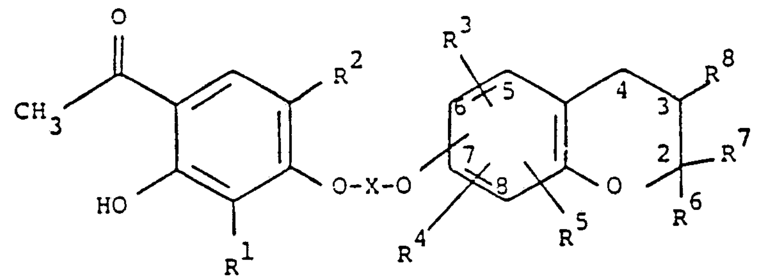

I

wherein $R^1$ signifies hydrogen or lower alkyl, $R^2$ signifies hydrogen or halogen and $R^3$, $R^4$ and $R^5$ each independently signify hydrogen, acyl or lower alkyl, whereby not more than one of $R^3$, $R^4$ and $R^5$ can signify acyl, and either $R^6$ and $R^7$ each signify hydrogen and $R^8$ signifies the group $-COOR^9$, or $R^6$ signifies hydrogen or lower alkyl, $R^7$ signifies the group $-(CH_2)_nCOOR^9$, $R^8$ signifies hydrogen, $R^9$ signifies hydrogen or lower alkyl, X signifies $(C_{3-7})$-alkylene and n signifies a number of 0-4, whereby "acyl" signifies lower alkanoyl or arylcarbonyl, "aryl" signifies phenyl optionally singly or multiply substituted by halogen, trifluoromethyl, lower alkyl, lower alkoxy, nitro, amino, lower alkylamino and di-lower alkylamino and the groups denoted as "lower" have up to 7 carbon atoms, and, when $R^9$ signifies hydrogen, salts thereof with pharmaceutically acceptable bases, characterized by

a) reacting a compound of the general formula

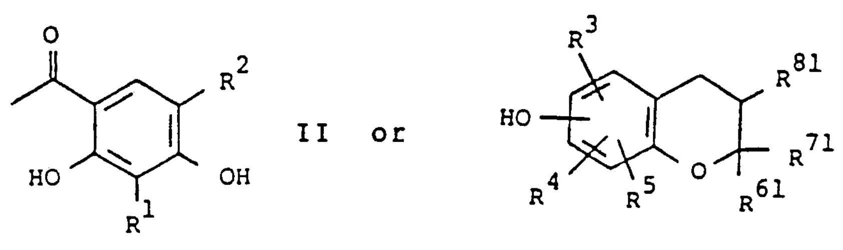

II or III

wherein either $R^{61}$ and $R^{71}$ each signify hydrogen and $R^{81}$ signifies the group $-COOR^{91}$ or $R^{61}$ signifies hydrogen or lower alkyl, $R^{71}$ signifies the group $-(CH_2)_n-COOR^{91}$, $R^{81}$ signifies hydrogen, and $R^{91}$ signifies lower alkyl and $R^1$, $R^2$, $R^3$, $R^4$, $R^5$ and n have the above significance,

with a compound of the general formula

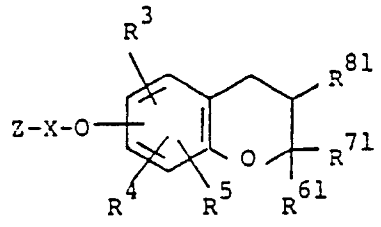 IV and 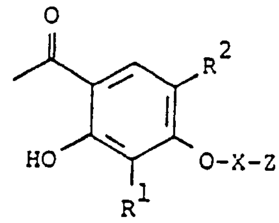 V

wherein $R^1$, $R^2$, $R^3$, $R^4$, $R^5$, $R^{61}$, $R^{71}$, $R^{81}$ and X have the above significance and Z signifies a halogen atom, or
b) hydrolyzing the ester group in a compound of the general formula

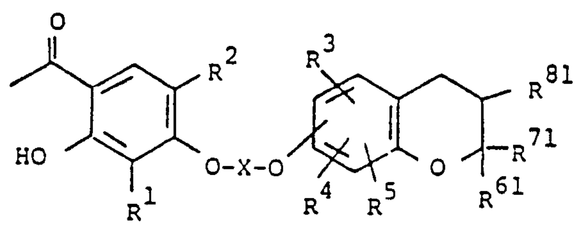 Ic

wherein $R^1$, $R^2$, $R^3$, $R^4$, $R^5$, $R^{61}$, $R^{71}$, $R^{81}$ and X have the above significance,
and, if desired, resolving a racemate obtained into the optically active isomers and/or converting a compound of formula I obtained in which $R^9$ signifies hydrogen with a base into a pharmaceutically acceptable salt.

25. A process according to claim 24, wherin $R^6$ signifies hydrogen or lower alkyl, $R^7$ signifies the group $-(CH_2)_n-COOR^9$ and $R^8$ signifies hydrogen, and $R^9$ and n have the significance given in claim 24.

26. A process according to claim 24, characterized in that compounds of the general formula

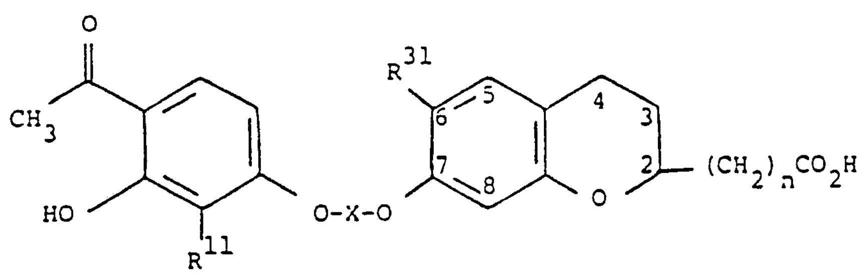 Ib

wherein $R^{11}$ signifies lower alkyl, $R^{31}$ signifies acyl, X signifies $(C_{3-7})$-alkylene and n signifies a number of 0-4, are manufactured.

27. A process according to claim 25, wherein $R^1$ signifies lower alkyl, $R^2$ signifies hydrogen, $R^3$ signifies acyl and $R^4$, $R^5$, $R^6$ and $R^9$ each signify hydrogen.

28. A process according to claim 27, characterized in that X signifies alkylene with 3-5 carbon atoms.

29. A process according to claim 28, wherein $R^3$ signifies acetyl.

30. A process according to claim 29, wherein $R^1$ signifies propyl.

31. A process according to claim 24. wherein rac-6-acetyl-7-[5-(4-acetyl-3-hydroxy-2-propylphenoxy)pentyloxy]-3,4-dihydro-2H-1-benzopyran-2-carboxylic acid is manufactured.

32. A process according to claim 24, wherein rac-7-[3-(4-acetyl-3-hydroxy-2-propylphenoxy)propoxy]-3,4-dihydro-2H-1-benzopyran-2-carboxylic acid is manufactured.

33. A process according to claim 24, wherein rac-6-acetyl-7-[3-(4-acetyl-3-hydroxy-2-propylphenoxy)propoxy]-2-methyl-8-propyl-2H-1-benzopyran-2-propanoic acid is manufactured.

34. A process according to claim 24, wherein rac-6-[3-(4-acetyl-3-hydroxy-2-propylphenoxy)propoxy]-3,4-dihydro-2-methyl-2H-1-benzopyran-2-carboxylic acid is manufactured.

35. A process according to claim 24, wherein rac-7-[3-(4-acetyl-3-hydroxy-2-propylphenoxy)propoxy]-3,4-dihydro-8-propyl-2H-1-benzopyran-2-carboxylic acid is manufactured.

36. A process according to claim 24. wherein rac-6-acetyl-7-[3-(4-acetyl-3-hydroxy-2-propylphenoxy)-propoxy]-3,4-dihydro-2H-1-benzopyran-2-carboxylic acid is manufactured.

**Revendications** pour les Etats contractants: BE, CH, DE, FR, GB, IT, LI, LU, NL, SE

1. Composés de formule générale

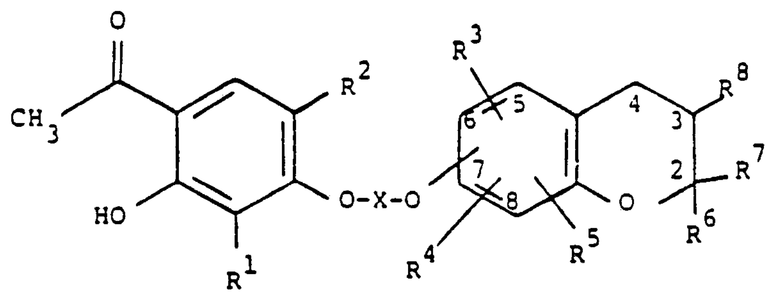

I

dans laquelle $R^1$ représente l'hydrogène ou un groupe alkyle inférieur, $R^2$ représente l'hydrogène ou un halogène et $R^3$, $R^4$ et $R^4$ représentent chacun, indépendamment les uns des autres, l'hydrogène, un groupe acyle ou alkyle inférieur, un seul au maximum des symboles $R^3$, $R^4$ et $R^5$ pouvant représenter un groupe acyle, et, ou bien $R^6$ et $R^7$ représentent tous deux l'hydrogène et $R^8$ le groupe $-COOR^9$, ou bien $R^6$ représente l'hydrogène ou un groupe alkyle inférieur, $R^7$ le groupe $-(CH_2)_n-COOR^9$, $R^8$ l'hydrogène, $R^9$ l'hydrogène ou un groupe alkyle inférieur, X représente un groupe alkylène en C 3-C 7 et n est un nombre allant de 0 à 4,

l'expression "acyle" désignant un groupe alcanoyle, inférieur ou arylcarbonyle, l'expression "aryle" désignant un groupe phényle éventuellement mono- ou poly-substitué par des halogènes, des groupes trifluorométhyle, alkyle inférieur, alcoxy inférieur, nitro, amino, alkylamino inférieur et di-(alkyle inférieur)-amino) et les groupes qualifiés d'inférieurs contenant jusqu'à 7 atomes de carbone,

et, lorsque $R^9$ représente l'hydrogène, leurs sels de bases acceptables pour l'usage pharmaceutique.

2. Composés selon la revendication 1, dans lesquels $R^6$ représente l'hydrogène ou un groupe alkyle inférieur, $R^7$ le groupe $-(CH_2)_n-COOR^9$ et $R^8$ l'hydrogène, et $R^9$ et n ont les significations indiquées dans la revendication 1.

3. Composés de formule générale

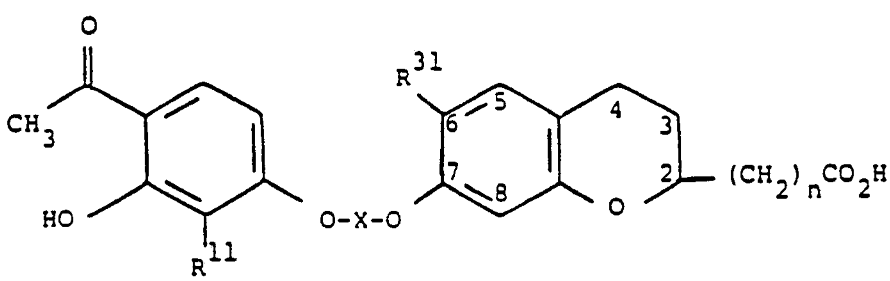

Ib

dans laquelle $R^{11}$ représente un groupe alkyle inférieur, $R^{31}$ un groupe acyle, X un groupe alkylène en C 3-C 7 et n un nombre allant de 0 à 4.

4. Composés selon la revendication 2, dans lesquels $R^1$ représente un groupe alkyle inférieur, $R^2$ l'hydrogène, $R^3$ un groupe acyle et $R^4$, $R^5$, $R^6$ et $R^9$ représentent chacun un atome d'hydrogène.

5. Composés selon la revendication 4, dans lesquels X représente un groupe alkylène en C 3-C 5.

6. Composés selon la revendication 5, dans lesquels $R^3$ représente un groupe acétyle.

7. Composés selon la revendication 6, dans lesquels $R^1$ représente un groupe propyle.

8. L'acide rac-6-acétyl-7-[5-(4-acétyl-3-hydroxy-2-propylphénoxy)-pentyloxy]-3,4-dihydro-2H-1-benzopyranne-2-carboxylique.

9. L'acide rac-7-[3-(4-acétyl-3-hydroxy-2-propylphénoxy)-propoxy]-3,4-dihydro-2H-1-benzopyranne-2-carboxylique.

10. L'acide rac-6-acétyl-7-[3-(4-acétyl-3-hydroxy-2-propylphénoxy)-propoxy]-2-méthyl-8-propyl-2H-1-benzopyranne-2-propanoïque.

11. L'acide rac-6-[3-(4-acétyl-3-hydroxy-2-propylphénoxy)-propoxy]-3,4-dihydro-2-méthyl-2H-1-benzopyranne 2-carboxylique.

12. L'acide rac-7-[3-(4-acétyl-3-hydroxy-2-propylphénoxy)-propoxy]-3,4-dihydro-8-propyl-2H-1-benzopyranne 2-carboxylique.

13. L'acide rac-6-acétyl-7-[3-(4-acétyl-3-hydroxy-2-propylphénoxy)-propoxy]-3,4-dihydro-2H-1-benzopyranne-2-carboxylique.

14. Composés de formule générale

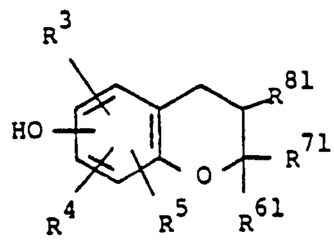

III

dans laquelle $R^3$, $R^4$ et $R^5$ représentent chacun, indépendamment les uns des autres, l'hydrogène, un groupe acyle ou alkyle inférieur, un seul au maximum des symboles $R^3$, $R^4$ et $R^5$ pouvant représenter un groupe acyle, et ou bien $R^{61}$ et $R^{71}$ représentent tous deux l'hydrogène et $R^{81}$ le groupe $-COOR^{91}$, ou bien $R^{61}$ représente l'hydrogène ou un groupe alkyle inférieur, $R^{71}$ le groupe $-(CH_2)_n-COOR^{91}$, représente l'hydrogène, $R^{91}$ un groupe alkyle inférieur et n un nombre allant de 0 à 4,

l'expression "acyle" désignant un groupe alcanoyle inférieur ou arylcarbonyle, l'expression "aryle" désignant un groupe phényle éventuellement mono- ou poly-substitué par des halogènes, des groupes trifluorométhyle, alkyle inférieur, alcoxy inférieur, nitro, amino, alkylamino inférieur et di-(alkyle inférieur)-amino, et les groupes qualifiés d'"inférieurs" contenant jusqu'à 7 atomes de carbone.

15. Composés de formule générale

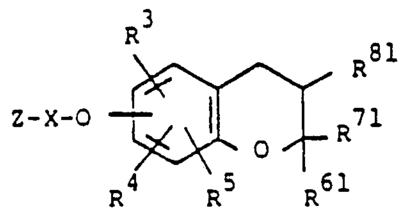

IV

dans laquelle $R^3$, $R^4$ et $R^5$ représentent chacun, indépendamment les uns des autres, l'hydrogène, un groupe acyle ou alkyle inférieur, un seul au maximum des symboles $R^3$, $R^4$ et $R^5$ pouvant représenter un groupe acyle, et ou bien $R^{61}$ et $R^{71}$ représentent tous deux l'hydrogène le groupe $-COOR^{91}$, ou bien $R^{61}$ représente l'hydrogène ou un groupe alkyle inférieur, $R^{71}$ le groupe $-(CH_2)_n-COOR^{91}$, $R^{81}$ représente l'hydrogène, un groupe alkyle inférieur, X un groupe alkylène en C 3-C 7, Z un atome d'halogène et n un nombre allant de 0 à 4,

l'expression "acyle" désignant un groupe alcanoyle inférieur ou arylcarbonyle, l'expression "aryle" désignant un groupe phényle éventuellement mono- ou poly-substitué par des halogènes, des groupes trifluorométhyle, alkyle inférieur, alcoxy inférieur, nitro, amino, alkylamino inférieur et di-(alkyle inférieur)-amino, et les groupes qualifiés d'"inférieurs" contenant jusqu'à 7 atomes de carbone.

16. Composés selon l'une des revendications 1 à 13, pour l'utilisation en tant que substances actives thérapeutiques.

17. Composés selon l'une des revendications 1 à 13, pour l'utilisation en tant que substances actives antiallergiques.

18. L'acide rac-6-acétyl-7-[5-(4-acétyl-3-hydroxy-2-propylphénoxy)-pentyloxy]-3,4-dihydro-2H-1-benzopyranne 2-carboxylique pour l'utilisation en tant que substance active thérapeutique.

19. L'acide rac-6-acétyl-7-[5-(4-acétyl-3-hydroxy-2-propylphénoxy)-pentyloxy]-3,4-dihydro-2H-1-benzopyranne-2-carboxylique pour l'utilisation en tant que substance active anti-allergique.

20. Procédé de préparation des composés selon l'une des revendications 1 à 13, caractérisé en ce que

a) on fait réagir un composé de formule générale

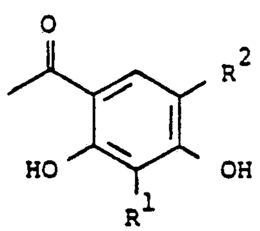

II

ou

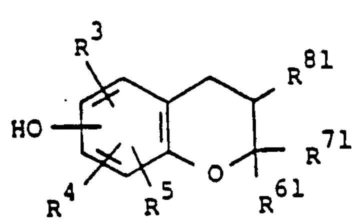

III

dans laquelle ou bien $R^{61}$ et $R^{71}$ représentent chacun l'hydrogène et $R^{81}$ le groupe $-COOR^{91}$, ou bien $R^{61}$, $R^{71}$ le groupe $-(CH_2)_n-COOR^{91}$, $R^{81}$ représente l'hydrogène et $R^{91}$ un groupe alkyle inférieur, et $R^1$, $R^2$, $R^3$, $R^4$, $R^5$ et n ont les significations indiquées ci-dessus, un groupe "alkyle inférieur" contenant jusqu'à 7 atomes de carbone,

avec un composé de formule générale

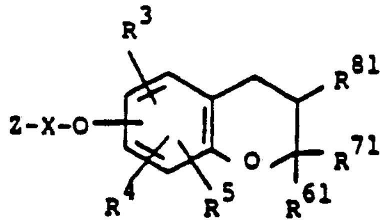 IV et 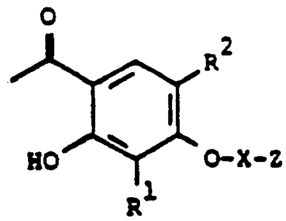 V

dans laquelle $R^1$, $R^2$, $R^3$, $R^4$, $R^5$, $R^{61}$, $R^{71}$ $R^{81}$ et X ont les significations indiquées ci-dessus et Z représente un atome d'halogène, ou bien

b) dans un composé de formule générale

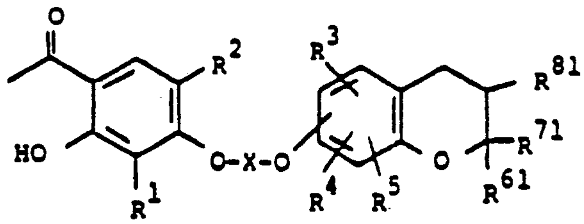 Ic

dans laquelle $R^1$, $R^2$, $R^3$, $R^4$, $R^5$, $R^{61}$, $R^{71}$, $R^{81}$ et X ont les significations indiquées ci-dessus, on hydrolyse le groupe ester et, si on le désire, on résout un racémate obtenu en les isomères optiques et/ou on convertit un composé obtenu répondant à la formule I dans laquelle $R^9$ représente l'hydrogène en un sel acceptable pour l'usage pharmaceutique à l'aide d'une base.

21. Médicament contenant un composé selon l'une des revendications 1 à 13 et un véhicule inerte du point de vue thérapeutique.

22. Agent anti-allergique contenant un composé selon l'une des revendications 1 à 13 et un véhicule inerte du point de vue thérapeutique.

23. Médicament contenant de l'acide rac-6-acétyl-7-[5-(4-acétyl-3-hydroxy-2-propylphénoxy)-pentyloxy]-3,4-dihydro-2H-1-benzopyranne-2-carboxylique et un véhicule inerte du point de vue thérapeutique.

24. Agent anti-allergique contenant de l'acide rac-6-acétyl-7-[5-(4-acétyl-3-hydroxy-2-propylphénoxy)-pentyloxy]-3,4-dihydro-2H-1-benzopyranne-2-carboxylique et un véhicule inerte du point de vue thérapeutique.

**Revendications** pour l'Etat contractant: AT

1. Composés de formule générale

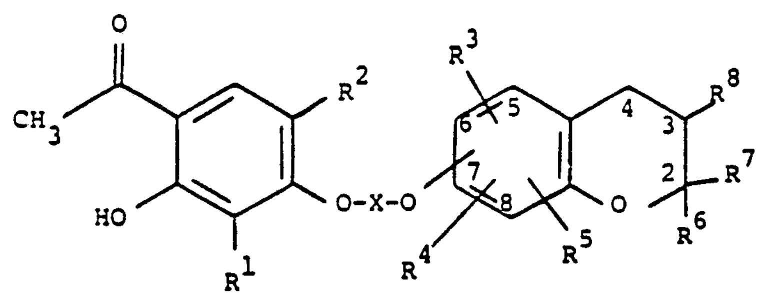 I

dans laquelle $R^1$ représente l'hydrogène ou un groupe alkyle inférieur, $R^2$ représente l'hydrogène ou un halogène et $R^3$, $R^4$ et $R^4$ représentent chacun, indépendamment les uns des autres, l'hydrogène, un groupe acyle ou alkyle inférieur, un seul au maximum des symboles $R^3$, $R^4$ et $R^5$ pouvant représenter un groupe acyle, et, ou bien $R^6$ et $R^7$ représentent tous deux l'hydrogène et $R^8$ le groupe $-COOR^9$, ou bien $R^6$ représente l'hydrogène ou un groupe alkyle inférieur, $R^7$ le groupe $-(CH_2)_n-COOR^9$, $R^8$ l'hydrogène, $R^9$ l'hydrogène ou un groupe alkyle inférieur, X représente un groupe alkylène en C 3-C 7 et n est un nombre allant de 0 à 4, l'expression "acyle" désignant un groupe alcanoyle, inférieur ou arylcarbonyle, l'expression "aryle" désignant un groupe phényle éventuellement mono- ou poly-substitué par des halogènes, des groupes trifluorométhyle, alkyle inférieur, alcoxy inférieur, nitro, amino, alkylamino inférieur et di-(alkyle inférieur)-amino) et les groupes qualifiés d'inférieurs contenant jusqu'à 7 atomes de carbone,

et, lorsque $R^9$ représente l'hydrogène, leurs sels de bases acceptables pour l'usage pharmaceutique.

2. Composés selon la revendication 1, dans lesquels $R^6$ représente l'hydrogène ou un groupe alkyle inférieur,

$R^7$ le groupe $-(CH_2)_n-COOR^9$ et $R^8$ l'hydrogène, et $R^9$ et n ont les significations indiquées dans la revendication 1.

3. Composés de formule générale

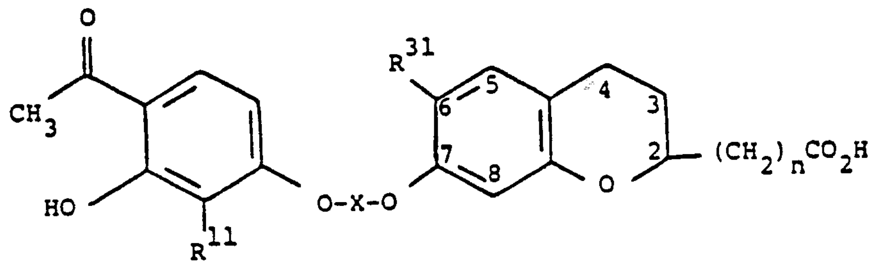

Ib

dans laquelle $R^{11}$ représente un groupe alkyle inférieur, $R^{31}$ un groupe acyle, X un groupe alkylène en C 3-C 7 et n un nombre allant de 0 à 4.

4. Composés selon la revendication 2, dans lesquels $R^1$ représente un groupe alkyle inférieur, $R^2$ l'hydrogène, $R^3$ un groupe acyle et $R^4$, $R^5$, $R^6$ et $R^9$ représentent chacun un atome d'hydrogène.

5. Composés selon la revendication 4, dans lesquels X représente un groupe alkylène en C 3-C 5.

6. Composés selon la revendication 5, dans lesquels $R^3$ représente un groupe acétyle.

7. Composés selon la revendication 6, dans lesquels $R^1$ représente un groupe propyle.

8. L'acide rac-6-acétyl-7-[5-(4-acétyl-3-hydroxy-2-propylphénoxy)-pentyloxy]-3,4-dihydro-2H-1-benzopyranne-2-carboxylique.

9. L'acide rac-7-[3-(4-acétyl-3-hydroxy-2-propylphénoxy)-propoxy]-3,4-dihydro-2H-1-benzopyranne-2-carboxylique.

10. L'acide rac-6-acétyl-7-[3-(4-acétyl-3-hydroxy-2-propylphénoxy)-propoxy]-2-méthyl-8-propyl-2H-1-benzopyranne-2-propanoïque.

11. L'acide rac-6-[3-(4-acétyl-3-hydroxy-2-propylphénoxy)-propoxy]-3,4-dihydro-2-méthyl-2H-1-benzopyranne-2-carboxylique.

12. L'acide rac-7-[3-(4-acétyl-3-hydroxy-2-propylphénoxy)-propoxy]-3,4-dihydro-8-propyl-2H-1-benzopyranne-2-carboxylique.

13. L'acide rac-6-acétyl-7-[3-(4-acétyl-3-hydroxy-2-propylphénoxy)-propoxy]-3,4-dihydro-2H-1-benzopyranne-2-carboxylique.

14. Composés de formule générale

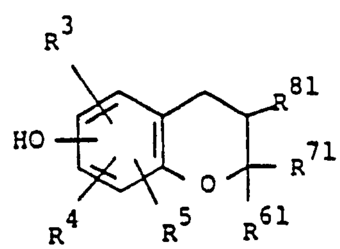

III

dans laquelle $R^3$, $R^4$ et $R^5$ représentent chacun, indépendamment les uns des autres, l'hydrogène, un groupe acyle ou alkyle inférieur, un seul au maximum des symboles $R^3$, $R^4$ et $R^5$ pouvant représenter un groupe acyle, et ou bien $R^{61}$ et $R^{71}$ représentent tous deux l'hydrogène et $R^{81}$ le groupe $-COOR^{91}$, ou bien $R^{61}$ représente l'hydrogène ou un groupe alkyle inférieur, $R^{71}$ le groupe $-(CH_2)_n-COOR^{91}$, représente l'hydrogène, $R^{91}$ un groupe alkyle inférieur et n un nombre allant de 0 à 4,

l'expression "acyle" désignant un groupe alcanoyle inférieur ou arylcarbonyle, l'expression "aryle" désignant un groupe phényle éventuellement mono- ou poly-substitué par des halogènes, des groupes trifluorométhyle, alkyle inférieur, alcoxy inférieur, nitro, amino, alkylamino inférieur et di-(alkyle inférieur)-amino, et les groupes qualifiés d'"inférieurs" contenant jusqu'à 7 atomes de carbone.

15. Composés de formule générale

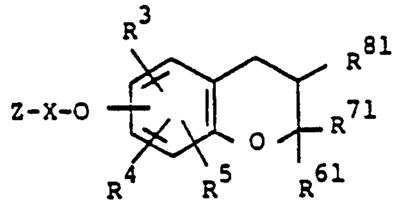

IV

dans laquelle $R^3$, $R^4$ et $R^5$ représentent chacun, indépendamment les uns des autres, l'hydrogène, un groupe acyle ou alkyle inférieur, un seul au maximum des symboles $R^3$, $R^4$ et $R^5$ pouvant représenter un groupe acyle, et ou bien $R^{61}$ et $R^{71}$ représentent tous deux l'hydrogène le groupe $-COOR^{91}$, ou bien $R^{61}$ représente l'hydrogène ou un groupe alkyle inférieur, $R^{71}$ le groupe $-(CH_2)_n-COOR^{91}$, $R^{81}$ représente l'hydrogène, $R^{91}$ un groupe alkyle inférieur, X un groupe alkylène en C 3-C 7, Z un atome d'halogène et n un nombre allant de 0 à 4,

l'expression "acyle" désignant un groupe alcanoyle inférieur ou arylcarbonyle, l'expression "aryle" désignant un groupe phényle éventuellement mono- ou poly-substitué par des halogènes, des groupes trifluorométhyle, alkyle inférieur, alcoxy inférieur, nitro, amino, alkylamino inférieur et di-(alkyle inférieur)-amino, et les groupes qualifiés d'"inférieurs" contenant jusqu'à 7 atomes de carbone.

16. Composés selon l'une des revendications 1 à 13, pour l'utilisation en tant que substances actives thérapeutiques.

17. Composés selon l'une des revendications 1 à 13, pour l'utilisation en tant que substances actives antiallergiques.

18. L'acide rac-6-acétyl-7-[5-(4-acétyl-3-hydroxy-2-propylphénoxy)-pentyloxy]-3,4-dihydro-2H-1-benzopyranne-2-carboxylique pour l'utilisation en tant que substance active thérapeutique.

19. L'acide rac-6-acétyl-7-[5-(4-acétyl-3-hydroxy-2-propylphénoxy)-pentyloxy]-3,4-dihydro-2H-1-benzopyranne-2-carboxylique pour l'utilisation en tant que substance active anti-allergique.

20. Médicament contenant un composé selon l'une des revendications 1 à 13 et un véhicule inerte du point de vue thérapeutique.

21. Agent anti-allergique contenant un composé selon l'une des revendications 1 à 13 et un véhicule inerte du point de vue thérapeutique.

22. Médicament contenant de l'acide rac-6-acétyl-7-[5-(4-acétyl-3-hydroxy-2-propylphénoxy)-pentyloxy]-3,4-dihydro-2H-1-benzopyranne-2-carboxylique et un véhicule inerte du point de vue thérapeutique.

23. Agent anti-allergique contenant de l'acide rac-6-acétyl-7-[5-(4-acétyl-3-hydroxy-2-propylphénoxy)-pentyloxy]-3,4-dihydro-2H-1-benzopyranne-2-carboxylique et un véhicule inerte du point de vue thérapeutique.

24. Procédé de préparation des composés de formule générale

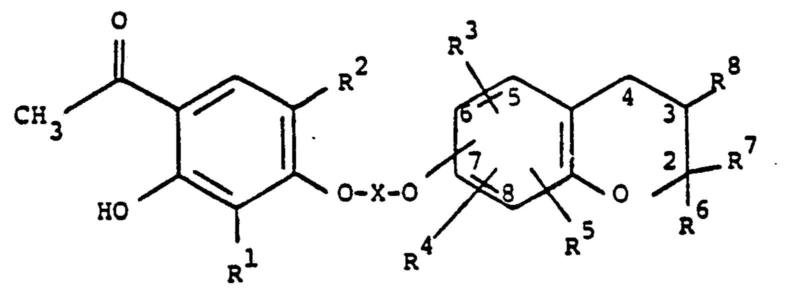

I

dans laquelle $R^1$ représente l'hydrogène ou un groupe alkyle inférieur, $R^2$ représente l'hydrogène ou un halogène et $R^3$, $R^4$ et $R^5$ représentent chacun, indépendamment les uns des autres, l'hydrogène, un groupe acyle ou alkyle inférieur, un seul au maximum des symboles $R^3$, $R^4$ et $R^5$ pouvant représenter un groupe acyle, et ou bien $R^6$ et $R^7$ représentent tous deux l'hydrogène et $R^8$ le groupe $-COOR^9$, ou bien $R^6$ représente l'hydrogène ou un groupe alkyle inférieur, $R^7$ le groupe $-(CH_2)_n-COOR^9$, $R^8$ l'hydrogène, $R^9$ l'hydrogène ou un groupe alkyle inférieur, X représente un groupe alkylène en C 3-C 7 et n est un nombre allant de 0 à 4,

l'expression "acyle" désignant un groupe alcanoyle inférieur ou arylcarbonyle et l'expression "aryle" désignant un groupe phényle éventuellement mono- ou poly-substitué par des halogènes, des groupes trifluorométhyle, alkyle inférieurs, alcoxy inférieurs, nitro, amino, alkylamino inférieurs et di-(alkyie inférieur)-amino, et les groupes qualifiés d'"inférieurs" contenant jusqu'à 7 atomes de carbone,

et, lorsque $R^9$ représente l'hydrogène, de leurs sels de bases acceptables pour l'usage pharmaceutique, caractérisé en ce que

a) on fait réagir un composé de formule générale

20

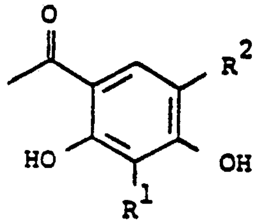

II

ou

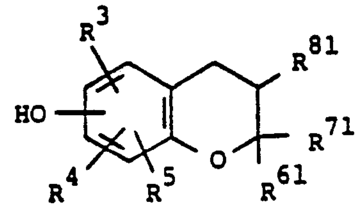

III

dans laquelle ou bien $R^{61}$ et $R^{71}$ représentent chacun l'hydrogène $R^{81}$ le groupe $-COOR^{91}$, ou bien $R^{61}$ représente l'hydrogène ou un groupe alkyle inférieur, $R^{71}$ le groupe $(CH_2)_n-COOR^{91}$, $R^{81}$ représente l'hydrogène et $R^{91}$ un groupe alkyle inférieur, et $R^1$, $R^2$, $R^3$, $R^4$, $R^5$ et n ont les significations indiquées ci-dessus,

avec un composé de formule générale

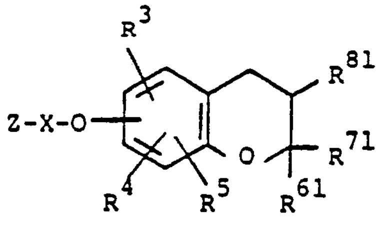 IV et 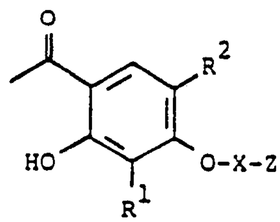 V

dans laquelle $R^1$, $R^2$, $R^3$, $R^4$, $R^5$, $R^{61}$, $R^{71}$, $R^{81}$ et X ont les significations indiquées ci-dessus et Z représente un atome d'halogène, ou bien

b) dans un composé de formule générale

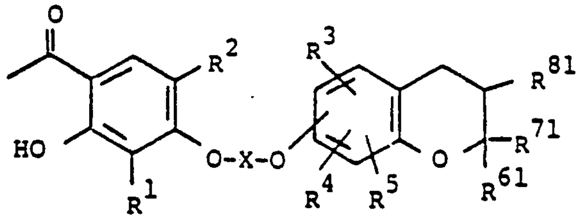 Ic

dans laquelle $R^1$, $R^2$, $R^3$, $R^4$, $R^5$, $R^{61}$, $R^{71}$, $R^{81}$ et X ont les significations indiquées ci-dessus, on hydrolyse le groupe ester et, si on le désire, on résout un racémate obtenu en les isomères optiques et/ou on convertit un composé obtenu, répondant à la formule I dans laquelle $R^9$ représente l'hydrogène, en un sel acceptable pour l'usage pharmaceutique à l'aide d'une base.

25. Procédé selon la revendication 24, dans lequel $R^6$ représente l'hydrogène ou un groupe alkyle inférieur, $R^7$ le groupe $-(CH_2)_n-COOR^9$ et $R^8$ l'hydrogène, et $R^9$ et n ont les significations indiquées dans la revendication 24.

26. Procédé selon la revendication 24, caractérisé en ce que l'on prépare des composés de formule générale

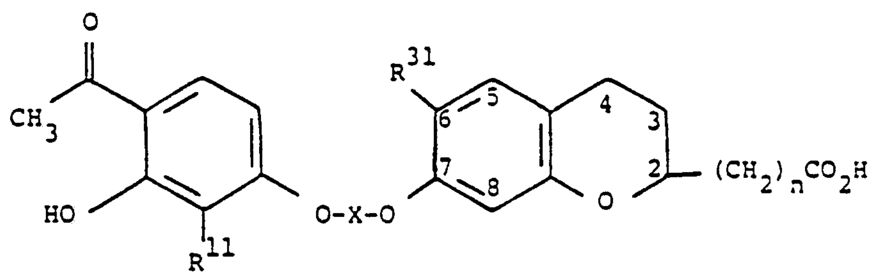 Ib

dans laquell $R^{11}$ représente un groupe alkyle inférieur, $R^{31}$ un groupe acyle, X un groupe alkylène en C 3-C 7 et n un nombre allant de 0 à 4.

27. Procédé selon la revendication 25, dans lequell $R^1$ représente un groupe alkyle inférieur, $R^2$ l'hydrogène, $R^3$ un groupe acyle et $R^4$, $R^5$, $R^6$ et $R^9$ représentent chacun un atome d'hydrogène.

28. Procédé selon la revendication 27, caractérisé en ce que X représente un groupe alkylène en C 3-C 5.

29. Procédé selon la revendication 28, dans lequel $R^3$ représente un groupe acétyle.

30. Procédé selon la revendication 29, dans lequel $R^1$ représente un groupe propyle.

31. Procédé selon la revendication 24, caractérisé en ce que l'on prépare l'acide rac-6-acétyl-7-[5-(4-acétyl-3-hydroxy-2-propylphénoxy)-pentyloxy]-3,4-dihydro-2H-1-benzopyranne-2-carboxylique.

32. Procédé selon la revendication 24, caractérisé en ce que l'on prépare l'acide rac-7-[3-(4-acétyl-3-hydroxy-2-propylphénoxy)-propoxy]-3,4-dihydro-2H-1-benzopyranne-2-carboxylique.

33. Procédé selon la revendication 24, caractérisé en ce que l'on prépare l'acide rac-6-acétyl-7-[3-(4-acétyl-3-hydroxy-2-propylphénoxy)-propoxy]-2-méthyl-8-propyl-2H-1-benzopyranne-2-propanoïque.

34. Procédé selon la revendication 24, caractérisé en ce que l'on prépare l'acide rac-6-[3-(4-acétyl-3-hydroxy-2-propylphénoxy)-propoxy]-3,4-dihydro-2-méthyl-2H-1-benzopyranne-2-carboxylique.

35. Procédé selon la revendication 24, caractérisé en ce que l'on prépare l'acide rac-7-[3-(4-acétyl-3-hydroxy-2-propylphénoxy)-propoxy]-3,4-dihydro-8-propyl-2H-1-benzopyranne-2-carboxylique.

36. Procédé selon la revendication 24, caractérisé en ce que l'on prépare l'acide rac-6-acétyl-7-[3-(4-acétyl-3-hydroxy-2-propylphénoxy)-propoxy]-3,4-dihydro-2H-1-benzopyranne-2-carboxylique.